# EUROPEAN PATENT APPLICATION

(11) **EP 4 461 730 A1**
(43) Date of publication of application: **13.11.2024**
(21) Application number: 23736983.0
(22) Date of filing: 03.01.2023
(51) Int. Cl.: C07D 471/04, A61K 31/63, A61K 31/635, A61P 35/00, A61P 35/02, A61P 1/16

(54) **PROTEIN DEGRADERS DEVELOPED ON BASIS OF BCL-2 FAMILY PROTEIN LIGAND COMPOUNDS AND USE THEREOF**

(30) Priority: 04.01.2022 CN 202210005499
(71) Applicant: Shanghaitech University, Shanghai 201210 (CN); Fudan University, Shanghai 200433 (CN)
(72) Inventor: YANG, Xiaobao, Shanghai 201306 (CN); JIANG, Biao, Shanghai 201210 (CN); TAN, Wenfu, Shanghai 201203 (CN)
(74) Representative: J A Kemp LLP
(86) International application number: PCT/CN2023/070102
(87) International publication number: WO 2023/131118

(57) **Abstract**

The present disclosure provides protein degraders developed based on BCL-2 family protein lig and compounds, including compounds of Formula (I) or their salts, enantiomers, stereoisomers, solvates, prodrugs, or polymorphs, and their applications for treating diseases.

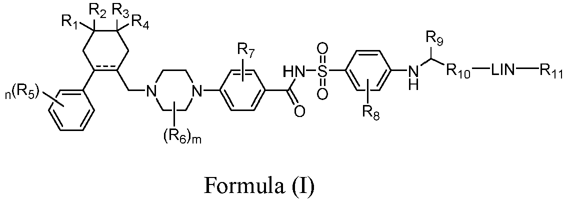

## Description

### Technical Field

The present disclosure relates to protein degraders developed based on BCL-2 family protein ligand compounds comprising the compound of Formula (I) or salts, enantiomers, stereoisomers, solvates, isotopically enriched analogs, prodrugs, or polymorphs thereof and their applications for treating diseases.

### Background

Apoptosis or programmed cell death is a process in which cells undergo a series of programmed events leading to their death. During early development, apoptosis can eliminate unwanted cells, while in adulthood, it is mainly responsible for eliminating damaged and irreparable cells in the body, thereby ensuring proper development of the body. Apoptosis plays an important role in various physiological processes, such as participating in normal cell renewal, the development and improvement of the immune system, hormone-dependent atrophy, embryonic development, and chemically induced cell death (Elmore, S., Toxicol Pathol, 2007. 35(4): p. 495-516.). However, abnormal apoptosis (excessive or insufficient) can lead to the development of various diseases, including neurodegenerative diseases, cardiovascular diseases, autoimmune diseases, and tumors. Studies have shown that tumors can reduce their sensitivity to anti-tumor drugs by inhibiting apoptosis pathways during their development and progression (Scott, W.L. et al., Carcinogenesis, 2000.21(3):p. 485-495.). Therefore, activating apoptosis signals in tumor tissues may be an effective anti-tumor strategy.

The BCL-2 (B cell lymphoma-2) protein family is a key regulator of apoptosis, which can both induce and inhibit apoptosis. This kind of proteins are mainly located on the mitochondria and plays a role as an "apoptosis switch" by regulating mitochondrial membrane permeability. BCL-2 has four highly conserved homologous (BH) domains: BH1, BH2, BH3 and BH4. Depending on the composition of the homologous domains, it can be divided into three subtypes, wherein one subtype has anti-apoptotic function and two subtypes have pro-apoptotic function (Daniel, N.N., Clin Cancer Res, 2007. 13(24): p. 7254-7263.). The BH1 and BH2 homologous domains of BCL-2 can form dimers with pro-apoptotic proteins, and the BH3 domain is crucial in the interaction between pro-apoptotic and anti-apoptotic proteins, and is therefore included in all subtypes. The BH4 domain is mainly included in the anti-apoptotic BCL-2 subtype. Anti-apoptotic proteins include BCL-2, BCL-XL, BCL-W, MCL1, A1 and BCL-B, which consist of 3-4 BH domains; while pro-apoptotic proteins can be divided into two types, one type of pro-apoptotic protein includes BAX, BAK and BOK, which is composed of BH1, BH2 and BH3, and another type of pro-apoptotic protein includes BID, BIM, BAD, PUMA, NOXA, BMF, HRK and BIK, which is composed of BH3 only (Youle, R.J., et al., Nat Rev Mol Cell Biol, 2008. 9(1): p. 47-59.). Under normal physiological conditions, the levels of BCL-2 family proteins are kept in a homeostasis, and cell signaling can regulate the expression of pro-apoptotic or anti-apoptotic proteins, thus resulting the survival or death of cells. When balance between the expression of anti-apoptotic proteins and pro-apoptotic proteins favors cell death, the pro-apoptotic proteins permeabilize the outer mitochondrial membrane through oligomerization on the outer mitochondrial membrane, releasing cytochrome C, triggering the activation of caspase enzyme cascades, and thus cleaving downstream substrates to cause cell death (Opferman, J.T. and A. Kothari, Cell Death Differ, 2018. 25(1): p. 37-45.)

After more than 20 years of development, numerous small molecule BCL-2 inhibitors have been discovered, and the development of small molecule inhibitors targeting the BCL-2 protein family has become a hot topic in the field of oncology. The anti-apoptotic BCL-2 protein exerts its anti-apoptotic function by binding to the BH3 domain of proapoptotic protein through its hydrophobic pocket (BH3 binding pocket). The BCL-2 family protein antagonists, also known as "BH3 domain mimetics", mainly prevent the binding of the anti-apoptotic BCL-2 family proteins to the pro-apoptotic proteins BAX and BAK by inserting into the shared BH3 domain of the anti-apoptotic BCL-2 family proteins, thereby inhibiting the function of the anti-apoptotic BCL-2 protein and initiating apoptosis of tumor cells.

To date, ABT-199 (Venetoclax) is the only anti-tumor drug targeting BCL-2 family proteins approved by the FDA to be used in the treatment of certain hematological malignancies, such as chronic lymphocytic leukemia and acute myeloid leukemia (Deeks, E.D., Drugs, 2016. 76(9): p. 979-87.; Carter, P.J. and G.A. Lazar, Nat Rev Drug Discov, 2018. 17(3): p. 197-223.). However, due to the fact that the growth of most solid tumors is independent of BCL-2 protein, ABT-199 has no significant therapeutic effect on solid tumors, while BCL-XL protein is significantly overexpressed in various solid tumors and hematologic malignancies (Leverson, J.M., et al, Sci Transl Med, 2015. 7(279): p. 279ra40.); on the other hand, studies have shown that BCL-XL expression in tumor tissues is positively correlated with tumor resistance to various therapies (Vogler, M., Adv Med, 2014: p. 943648.), thus indicating that BCL-XL is a potentially promising anti-tumor molecular target.

BCL-XL (B-cell lymphoma-extra large) is one of the key members of the anti-apoptotic proteins of BCL-2 family, located on the mitochondria, and is a key regulator of apoptosis. It is also invovled in many cellular pathophysiological processes of a variety of cells, such as mitochondrial ATP synthesis, protein acetylation, and cell mitosis (Michels, J., et al., International Journal of Cell Biology, 2013. 2013: p. 1-10.)

Studies have shown that senescense of vascular cells is the primary key pathogenesis of retinopathy, such as diabetic macular edema (DME) and wet age-related macular degeneration (wAMD) (Oubaha M., et al., Sci Transl Med. 2016 Oct 26, 8(362):362ra144.). Targeting the anti-apoptotic protein BCL-XL can induce apoptosis of senescent vascular endothelial cells, thereby achieving the goal of treating DME and wAMD (Crespo-Garcia S., et al., Cell Metabolism. 2021 Apr 6, 33(4):818-832.e7.). The small molecule inhibitor UBX1325 targeting BCL-XL is currently in the clinical phase II for treatment of DME, wAMD (clinicaltrials.gov).

Studies have also shown that BCL-XL is overexpressed in many tumors, such as liver cancer, non-Hodgkin's lymphoma, chondrosarcoma, and colorectal cancer (Li, M., et al., Pharmacological Research, 2019. 151(4): p. 104547.). In chondrosarcoma, BCL-XL plays the most critical role compared to other BCL2 family members, and inhibiting BCL-XL can enhance the sensitivity of chondrosarcoma cells to conventional chemotherapy (Jong, Y.D., et al., Oncogenesis, 2018. 7(9)). In addition, the invasiveness of colorectal cancer cells is closely related to BCL-XL, and only BCL-XL is significantly upregulated among the BCL2 protein family in colorectal cancer (Liu, W.D., et al., Official journal of Balkan Union of Oncology, 2014. 19(4): p. 925.; Scherr, A.L., et al., Cell Death & Disease, 2016. 7(8): p. e2342.). Small molecule inhibitors targeting BCL-XL, such as ABT-263, have significant antitumor effects. However, due to the essential role of BCL-XL for plateletes survival by antagonizing the pro-apoptotic effect of Bax, inhibition of BCL-XL may cause apoptosis of platelets and consequent thromocytopenia. Hence, the clinical development of ABT-263 failed due to the thromocytopenia resulting from inhibition of BCL-XL by ABT-263 (Tse, C., et al., Cancer Research, 2008. 68(9): p. 3421.). Despite being one of the most important validated anticancer targets, there is no safe and effective treatment for BCL-XL.

PROTAC (Proteolysis Targeting Chimeria) is a bifunctional heterozygous small molecule where a target protein ligand connects to an E3 ubiquitin ligase ligand through a suitable link chain, promoting the formation of a ternary complex between the target protein and the E3 ligase, thereby bringing the target protein closer to the ubiquitination system and achieving ubiquitination of the target protein which is subsequently recognized by a proteasome 26S subunit, ultimately leading to degradation of the target protein (Sun, X., et al., Sig Transduct Target Ther, 2019. 4(1): p. 33.). The process of protein degradation caused by PROTAC molecules is similar to a catalytic reaction, thus enabling the degradation at extremely low doses and more potent and less toxic than conventional small-molecule inhibitors (Lu, J., et al., Chem Biol, 2015. 22(6): p.755-763.). Moreover, since PROTAC relies on E3 ubiquitin ligases to mediate protein degradation, even if the target protein itself is widely expressed in the body, it still has cell or tissue selectivity by utilizing the differential expression of E3 ubiquitin ligase between tumor cells and normal tissues. For example, DT2216, which has been investigated in clinical trials and used ABT-263 as a ligand for the target protein BCL-XL and VHL, which is low expressed in platelets, as an E3 ubiquitin ligase ligand, was obseved to significantly improve anti-tumor activity and reduce platelet toxicity (Khan, S., et al., Nature medicine, 2019. 25(4): p. 1938-1947.).

Abnormally activated Hedgehog (Hh) signaling pathway is closely related to the occurrence and development of various tumors in the human body, such as basal cell carcinoma and medulloblastoma (Nat Genet, 2016. 48(4): p. 398-406; Cancer Cell, 2014. 25(3): p. 393-405.). The activation of Hh signaling activity is a cascade reaction, which invovles several key molecules including the PTCH receptor with a 12-fold transmembrane domain, the SMO (Smoothened) receptor with 7-fold transmembrane structures, SUFU proteins, and the GLI protein family of nuclear transcription factors. In the absence of exogenous HH ligands, PTCH inhibits SMO activity, leading to the HH pathway kept in an inactive state. The binding of the exogenous HH ligand to PTCH disregulate its inhibition of the SMO, thus causing the accumulation of SMO into central cilia in the signaling pathway. The accumulation of SMO into central cilia further causes the transcriptional complex of SUFU-GLI to also translocate to the cilia, consequently relieving the inhibitory effect of SUFU on GLI. Furthermore, the phosphorylation of GLI controlled by kinases, such as GSK 3 and CK1, ultimately enables transcriptionally active GLI into the nucleus to initiate downstream target gene transcription, and activation of the Hh signaling pathway (Nat Rev Mol Cell Biol, 2013. 14(7): p. 416-29; Genes Dev, 2010. 24(7): p. 670-82.). Currently, all FDA approved drugs for the treatment of basal cell carcinoma are developed by targeting SMO (Cell, 2016.164 (5): p.831.). It is conceivable that these current SMO inhibitors have no significant inhibitory effect on the tumors with abnormal Hh activation caused by the genetic changes of the key signal molecules downstream of SMO, such as SMO mutation, SUFU loss-of-function mutation, and GLI amplification. Recently, studies have shown that SUFU protein contains a conserved BH3 sequence which can bind to anti-apoptotic proteins such as BCL-XL, BCL-2, and MCL-1 in the cell, thereby releasing the inhibitory effect of SUFU on GLI protein, ultimately leading to the activation of the Hh pathway. Interrupting the binding of anti-apoptotic proteins such as BCL-XL to SUFU can effectively inhibit the activation of Hh signaling pathway (Nat Cell Biol, 2017.19 (10): p.1226-1236.). Based on this, targeting BCL-2 family proteins is also a strategy to develop inhibitors targeting the Hh signaling pathway.

Therefore, there is an urgent need for a series of novel PROTAC inhibitors for the treatment and/or prevention of diseases or disorders mediated by BCL-2 family proteins, or diseases or disorders dependent on the Hedgehog signaling pathway, or diseases associated therewith.

### Summary of Invention

In view of the above, an objective of the present disclosure is to provide novel protein degraders developed based on BCL-2 family protein ligand compounds as well as their applications and usage methods. The novel protein degraders developed based on BCL-2 family protein ligand compounds or their salts (including pharmaceutically acceptable salts), stereoisomers (including enantiomers), solvates, prodrugs or polymorphs of the present disclosure can bind to target proteins, recruit target proteins to E3 ubiquitin ligases for ubiquitination labeling and degradation, thereby exhibiting desired pharmacological activities.

In some embodiments, the novel protein degraders developed based on BCL-2 family protein ligand compounds of the present disclosure may be the compound of Formula (I) 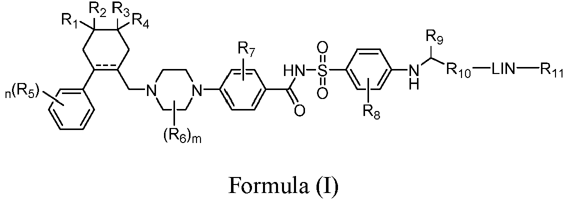 or salts, stereoisomers (including enantiomers), solvates, isotopically enriched analogs, prodrugs, or polymorphs thereof,
wherein dotted line indicates the optional presence of a double bond;
R₁, R₂, R₃, R₄ are the same or different and each independently represents hydrogen, C₁₋₄ alkyl, halogen, or halogenated C₁₋₄ alkyl;
(R₅)ₙ indicates that the benzene ring is substituted with n R₅, with each R₅ being the same or different and each independently representing halogen, C₁₋₆ alkyl, or halogenated C₁₋₆ alkyl; n represents an integer of 1, 2, 3, 4 or 5;
(R₆)ₘ indicates that piperazine ring is substituted with m R₆, with each R₆ being the same or different and each independently representing halogen, C₁₋₆ alkyl, or halogenated C₁₋₆ alkyl; m represents an integer of 0, 1, 2, 3, 4, 5, 6, 7 or 8;
R₇ represents hydrogen or 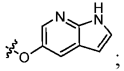
R₈ represents hydrogen or -SO₂CF₃ or -NO₂;
R₉ represents hydrogen or C₁₋₄ alkyl;
R₁₀ represents the following group: 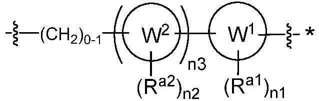
wherein ring W¹ represents 4- to 6-membered nitrogen-containing heterocyclylene; and (R^{a1})ₙ₁ indicates that ring W¹ is substituted with n1 R^{a1}, with each R^{a1} being the same or different and each independently representing C₁₋₆ alkyl, halogenated C₁₋₆ alkyl, C₁₋₆ alkoxy, cyano, halogen, or oxo; and n1 represents an integer of 0-8;
ring W² represents 4- to 6-membered nitrogen-containing heterocyclylene, or C₃₋₆ cycloalkylene; n3 represents an integer of 0 or 1; and (R^{a2})ₙ₂ indicates that ring W² is substituted with n2 R^{a2}, with each R^{a2} being the same or different and each independently representing C₁₋₆ alkyl, halogenated C₁₋₆ alkyl, C₁₋₆ alkoxy, cyano, halogen, or oxo; and n2 represent an integer of 0-8; and
symbol * indicates the point of attachment to LIN;
LIN represents:
   optionally substituted linear or branched C₁₋₃₀ alkylene; or
   optionally substituted linear or branched C₂₋₃₀ alkylene, wherein one or more groups R_{b} and/or one or more groups R_{c} and/or any combination of one or more groups R_{b} and R_{c} are inserted into the backbone carbon chain of the linear or branched C₂₋₃₀ alkylene group, wherein carbon-carbon bond between one or more pairs of adjacent carbon atoms in the backbone carbon chain is interrupted by the group R_{b}, R_{c}, or a combination of R_{b} and R_{c}; wherein each R_{b} is selected from the group consisting of O, N(R_{d}), C(O), C(O)O, S(O), S(O)₂, S(O)₂NH, NHS(O)₂, OC(O), C(O)N(R_{d}), N(R_{d})C(O), or N(R_{d})C(O)N(R_{d}), where each R_{b} independently represents H or C₁₋₆ alkyl, and in case that two or more groups R_{b} are inserted into the backbone carbon chain of the linear or branched C₂₋₃₀ alkylene group, the two or more groups R_{b} are not directly connected to each other; and wherein each R_{c} is selected from the group consisting of cycloalkylene, arylene, heterocyclylene, heteroarylene, alkynylene, alkenylene, or any combination thereof, wherein the cycloalkylene, arylene, heterocyclylene, and heteroarylene are each independently optionally substituted with a substituent selected from the group consisting of C₁₋₆ alkyl, halogenated C₁₋₆ alkyl, halogen, C₁₋₆ alkoxy, or any combination thereof; and
R₁₁ represents the following structure of Formula (II): 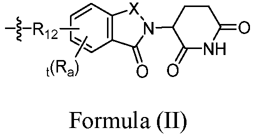
   wherein R₁₂ represents N(Rₑ), O, S, C₂₋₆ alkynylene, or C₂₋₆ alkenylene, where Rₑ represents H or C₁₋₆ alkyl, or R₁₂ represents a bond;
   (Rₐ)ₜ indicates that the benzene ring is substituted with t Rₐ, with each Rₐ being the same or different and each independently representing bromine, and t represents an integer of 0, 1, 2 or 3; and
   X represents C(O) or CH₂;
wherein in case that t represents an integer of 0:
when R₇ represents 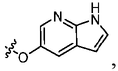 n1 represents an integer of 1-8, and R^{a1} are the same or different and each independently represents C₁₋₆ alkyl, halogenated C₁₋₆ alkyl, C₁₋₆ alkoxy, or halogen; or
when R₇ represents 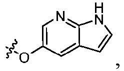 R₈ represents -SO₂CF₃.

In another aspect, the present disclosure provides a pharmaceutical composition comprising the compound of Formula (I) or salts, stereoisomers (including enantiomers), solvates, isotopically enriched analogs, prodrugs, or polymorphs thereof, and at least one pharmaceutically acceptable carrier.

In another aspect, the novel protein degraders developed based on BCL-2 family protein ligand compounds of the present disclosure may be the following compounds or salts, stereoisomers (including enantiomers), solvates, prodrugs, or polymorphs thereof:
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-oxoisoindolin-5-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1,3-dioxoisoindolin-5-yl)methyl)piperazin-1-yl)-1-(phenylthio)butan-2-yl)amino)-3-((trifluoromethyl)sulfonyl)phenyl)sulfonyl)benzamide;
2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((1r,4r)-4-(4-((2-(2,6-dioxopiperidin-3-yl)-1-oxoisoindolin-5-yl)methyl)piperazin-1-yl)cyclohexyl)methyl)amino)-3-nitrophenyl)sulfonyl)benzamide;
2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((1s,4s)-4-(4-((2-(2,6-dioxopiperidin-3-yl)-1-oxoisoindolin-5-yl)methyl)piperazin-1-yl)cyclohexyl)methyl)amino)-3-nitrophenyl)sulfonyl)benzamide; or
2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((1r,4r)-4-(4-((2-(2,6-dioxopiperidin-3-yl)-1,3-dioxoisoindolin-5-yl)methyl)piperazin-1-yl)cyclohexyl)methyl)amino)-3-nitrophenyl)sulfonyl)benzamide.

In another aspect, the present disclosure provides a pharmaceutical composition comprising the above protein degraders compounds or salts, stereoisomers (including enantiomers), solvates, prodrugs, or polymorphs thereof, and at least one pharmaceutically acceptable carrier.

In a further aspect, the present disclosure further provides a medicine kit or reagent kit comprising: the protein degraders compounds or a pharmaceutically acceptable salt of the present disclosure, or the pharmaceutical composition comprising the same.

In a further aspect, the present disclosure provides the protein degraders compounds or salts, stereoisomers (including enantiomers), solvates, prodrugs, or polymorphs thereof of the present disclosure, or the pharmaceutical composition comprising the same as active ingredient for use as a medicament.

In a further aspect, the present disclosure provides the protein degraders compounds or salts, stereoisomers (including enantiomers), solvates, prodrugs, or polymorphs thereof of the present disclosure, or the pharmaceutical composition comprising the same as active ingredient for use as a medicament.

In a further aspect, the present disclosure provides the protein degraders compounds or salts, stereoisomers (including enantiomers), solvates, prodrugs, or polymorphs thereof of the present disclosure, or the pharmaceutical composition comprising the same as active ingredient for use in the prevention and/or treatment of a disease or disorder selected from the group consisting of neurodegenerative diseases; angiocardiopathy; autoimmune disease; myelofibrosis; renal fibrosis; hepatic fibrosis; cirrhosis; tumors; multiple-organ dysfunction syndrome (MODS); multiple-organ failure caused by cachexia and tangnsepsis shock; organ (including kidney, heart, lung) or tissue transplant rejection; diabetes mellitus; transplant rejection; retinopathy and acute liver failure.

In a further aspect, the present disclosure provides use of the protein degraders compounds or salts, stereoisomers (including enantiomers), solvates, prodrugs, or polymorphs thereof of the present disclosure for the manufacture of a medicament for the prevention and/or treatment of a disease or disorder selected from the group consisting of neurodegenerative diseases; angiocardiopathy; autoimmune disease; myelofibrosis; renal fibrosis; hepatic fibrosis; cirrhosis; tumors; multiple-organ dysfunction syndrome (MODS); multiple-organ failure caused by cachexia and tangnsepsis shock; organ (including kidney, heart, lung) or tissue transplant rejection; diabetes mellitus; transplant rejection; retinopathy and acute liver failure.

In a further aspect, the present disclosure provides a method for treating or preventing a disease or disorder in a subject, comprising administering to the subject a therapeutically effective amount of the protein degraders compounds or salts, enantiomers, stereoisomers, solvates, prodrugs, or polymorphs thereof of the present disclosure or the pharmaceutical composition comprising the same, or the compounds or salts, stereoisomers (including enantiomers), solvates, prodrugs, or polymorphs thereof of the present disclosure or the pharmaceutical composition comprising the same as active ingredient, wherein the disease or disorder selected from the group consisting of neurodegenerative diseases; angiocardiopathy; autoimmune disease; myelofibrosis; renal fibrosis; hepatic fibrosis; cirrhosis; tumors; multiple-organ dysfunction syndrome (MODS); multiple-organ failure caused by cachexia and tangnsepsis shock; organ (including kidney, heart, lung) or tissue transplant rejection; diabetes mellitus; transplant rejection; retinopathy and acute liver failure.

### Brief Description of the Drawings

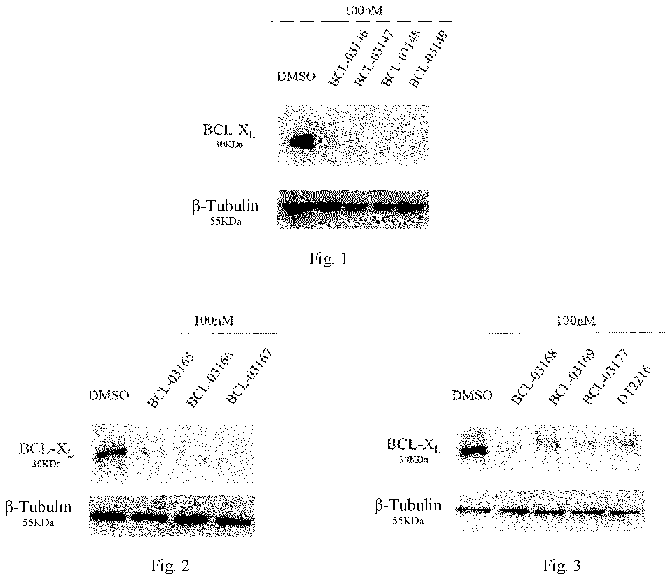

Figures 1-3 are schematic diagrams of the results of protein immunoblotting experiments, which show that compounds BCL-03146, BCL-03147, BCL-03148, BCL-03149, etc. (100 nM) of the present invention significantly reduce the expression of BCL-XL protein in Molt-4 cells after treatment of 8 h at 100 nM concentration, and have a significant advantage in protein degradation of BCL-XL compared with DT2216 (CAS No.: 2365172-42-3; BCL-XL degrader reported in the literature).

### Detailed Description of the Invention

The following detailed description is provided as exemplary specific embodiments to assist those skilled in the art in understanding and practicing the present disclosure. It should be appreciated, however, that such description is not intended to limit the scope of the present disclosure, and that various modifications and changes may be made to the specific embodiments described in the present disclosure without departing from the spirit and scope of the present disclosure. Such changes and modifications are to be understood as being included within the scope of the present invention as defined by the appended claims.

### I. Compounds

### Compounds of Formula (I)

The present disclosure provides the compound of Formula (I) or salts (including pharmaceutically acceptable salts), stereoisomers (including enantiomers), solvates, isotopically enriched analogs, prodrugs, or polymorphs thereof: 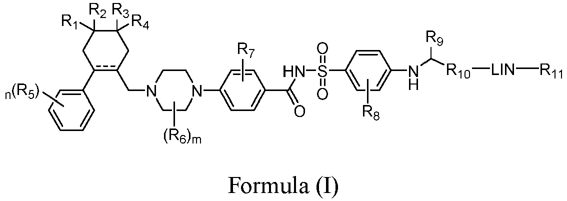 wherein R₁, R₂, R₃, R₄, (R₅)ₙ, (R₆)ₘ, R₇, R₈, R₉, R₁₀, LIN, and R₁₁ are as defined in the compound of Formula (I) above.

In some embodiments, the dotted line in the compound of Formula (I) indicates the optional presence of a double bond.

In some embodiments, the dotted line in the compound of Formula (I) indicates the presence of a double bond. In this case, the group containing the dotted line in the compound of Formula (I) is shown below:

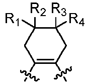

In some embodiments, the dotted line in the compound of Formula (I) indicates the absence of a double bond. In this case, the group containing the dotted line in the compound of Formula (I) is shown below:

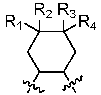

In some embodiments, R₁, R₂, R₃, and R₄ of the compound of Formula (I) are the same or different and each independently represents hydrogen, C₁₋₄ alkyl (e.g., methyl, ethyl, propyl, isopropyl, butyl, and tert-butyl), halogen (e.g., fluorine, chlorine, bromine, or iodine), or halogenated C₁₋₄ alkyl (e.g., halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, R₁ and R₂ of the compound of Formula (I) represents hydrogen, and R₃ and R₄ represents methyl.

In some embodiments, R₁ and R₂ of the compound of Formula (I) represents methyl, and R₃ and R₄ represents hydrogen.

In some embodiments, (R₅)ₙ of the compound of Formula (I) indicates that the benzene ring is substituted with n R₅, with each R₅ being the same or different and each independently representing halogen (e.g., fluorine, chlorine, bromine, or iodine), C₁₋₆ alkyl (e.g., C₁₋₄ alkyl or C₁₋₃ alkyl, such as methyl, ethyl, propyl, isopropyl, butyl, sec-butyl or tert-butyl), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and n represents an integer of 1, 2, 3, 4 or 5. In some embodiments, n represents an integer of 1, and R₅ represents halogen (e.g., fluorine, chlorine, bromine, or iodine).

In some embodiments, (R₆)ₘ of the compound of Formula (I) indicates that piperazine ring is substituted with m R₆, with each R₆ being the same or different and each independently representing halogen (e.g., fluorine, chlorine, bromine, or iodine), C₁₋₆ alkyl (e.g., C₁₋₄ alkyl or C₁₋₃ alkyl, such as methyl, ethyl, propyl, isopropyl, butyl, sec-butyl or tert-butyl), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and m represents an integer of 0, 1, 2, 3, 4, 5, 6, 7 or 8.

In some embodiments, R₇ represents hydrogen or

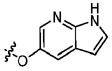

In some embodiments, R₇ represents hydrogen.

In some embodiments, R₇ represents

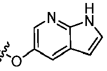

In some embodiments, R₈ represents hydrogen or -SO₂CF₃ or -NO₂.

In some embodiments, R₈ represents hydrogen.

In some embodiments, R₈ represents -SO₂CF₃.

In some embodiments, R₈ represents -NO₂.

In some embodiments, R₉ represents hydrogen or C₁₋₄ alkyl (e.g., C₁₋₃ alkyl, such as methyl, ethyl, propyl, isopropyl, butyl, sec-butyl or tert-butyl).

In some embodiments, R₉ represents hydrogen.

In some embodiments, R₉ represents C₁₋₄ alkyl (e.g., C₁₋₃ alkyl, such as methyl, ethyl, propyl, isopropyl, butyl, sec-butyl or tert-butyl).

In some embodiments, R₁₀ represents the following group: 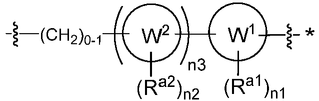
wherein ring W¹ represents 4- to 6-membered nitrogen-containing heterocyclylene; and (R^{a1})ₙ₁ indicates that ring W¹ is substituted with n1 R^{a1}, with each R^{a1} being the same or different and each independently representing C₁₋₆ alkyl (e.g., C₁₋₄ alkyl or C₁₋₃ alkyl, such as methyl, ethyl, propyl, isopropyl, butyl, sec-butyl or tert-butyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy or C₁₋₃ alkoxy, such as methoxy, ethoxy, propoxy, isopropoxy, butoxy, sec-butoxy or tert-butoxy), cyano, halogen (e.g., fluorine, chlorine, bromine, or iodine), or oxo; and n1 represents an integer of 0-8;
ring W² represents 4- to 6-membered nitrogen-containing heterocyclylene, or C₃₋₆ cycloalkylene; n3 represents an integer of 0 or 1; and (R^{a2})ₙ₂ indicates that ring W² is substituted with n2 R^{a2}, with each R^{a2} being the same or different and each independently representing C₁₋₆ alkyl (e.g., C₁₋₄ alkyl or C₁₋₃ alkyl, such as methyl, ethyl, propyl, isopropyl, butyl, sec-butyl or tert-butyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy or C₁₋₃ alkoxy, such as methoxy, ethoxy, propoxy, isopropoxy, butoxy, sec-butoxy or tert-butoxy), cyano, halogen (e.g., fluorine, chlorine, bromine, or iodine), or oxo; and n2 represents an integer of 0-8; and
symbol * indicates the point of attachment to LIN.

In some embodiments, ring W¹ represents 4- to 6-membered nitrogen-containing heterocyclylene, and the 4- to 6-membered nitrogen-containing heterocyclylene includes but is not limited to e.g., piperidinylene, piperazinylene, morpholinylene, azetidinylene, pyrrolidinylene, imidazolidylene, pyrazolidylene, oxazolidinylene, thiazolidinylene, or thiomorpholinylene.

In some embodiments, (R^{a1})ₙ₁ indicates that ring W¹ is substituted with n1 R^{a1}, with each R^{a1} being the same or different and each independently representing C₁₋₆ alkyl (e.g., C₁₋₄ alkyl or C₁₋₃ alkyl, such as methyl, ethyl, propyl, isopropyl, butyl, sec-butyl or tert-butyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy or C₁₋₃ alkoxy, such as methoxy, ethoxy, propoxy, isopropoxy, butoxy, sec-butoxy or tert-butoxy), cyano, halogen (e.g., fluorine, chlorine, bromine, or iodine), or oxo, and n1 represents an integer of 0-8 (e.g., an integer of 0, 1, 2, 3, or 4).

In some embodiments, ring W² represents 4- to 6-membered nitrogen-containing heterocyclylene, and the 4- to 6-membered nitrogen-containing heterocyclylene includes but is not limited to e.g., piperidinylene, piperazinylene, morpholinylene, azetidinylene, pyrrolidinylene, imidazolidylene, pyrazolidylene, oxazolidinylene, thiazolidinylene, or thiomorpholinylene.

In some embodiments, ring W² represents C₃₋₆ cycloalkylene, and the 4- to 6-membered cycloalkylene includes but is not limited to e.g., cyclopropyl, cyclobutyl, cyclopentyl, or cyclohexyl.

In some embodiments, (R^{a2})ₙ₂ indicates that ring W² is substituted with n2 R^{a2}, with each R^{a2} being the same or different and each independently representing C₁₋₆ alkyl (e.g., C₁₋₄ alkyl or C₁₋₃ alkyl, such as methyl, ethyl, propyl, isopropyl, butyl, sec-butyl or tert-butyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy or C₁₋₃ alkoxy, such as methoxy, ethoxy, propoxy, isopropoxy, butoxy, sec-butoxy or tert-butoxy), cyano, halogen (e.g., fluorine, chlorine, bromine, or iodine), or oxo; and n2 represents an integer of 0-8 (e.g., an integer of 0, 1, 2, 3, or 4).

In some embodiments, n3 represents an integer of 0 or 1.

In some embodiments, n3 represents an integer of 0.

In some embodiments, n3 represents an integer of 1.

In some embodiments, R₁₀ represents the following groups: 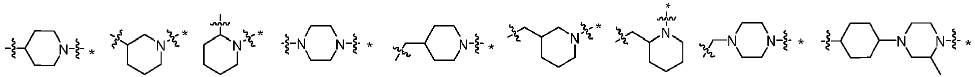 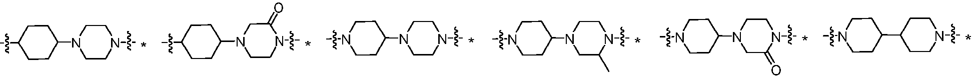 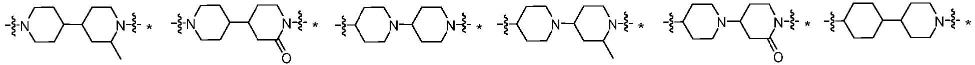 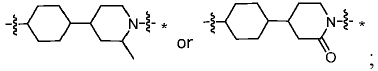 wherein symbol * indicates the point of attachment to LIN.

In some embodiments, R₁₁ represents the following structure of Formula (II-1), Formula (II-2), Formula (II-3), Formula (II-4), Formula (II-5), Formula (II-6), Formula (II-7), Formula (II-8), Formula (II-9), Formula (II-10), Formula II-11), or Formula (II-12): 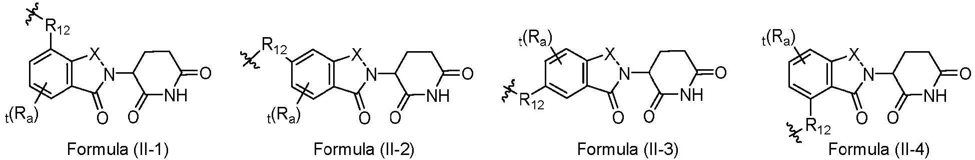 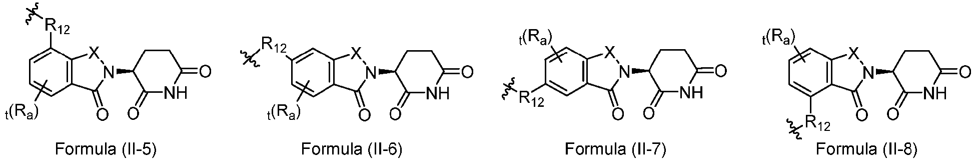 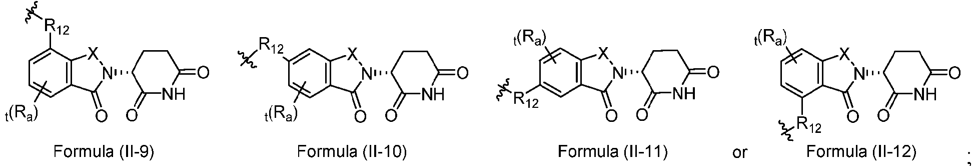
wherein R₁₂ represents N(Rₑ), O, S, C₂₋₆ alkynylene (e.g., ethynylene), or C₂₋₆ alkenylene (e.g., vinylene), wherein Rₑ represents H or C₁₋₆ alkyl (e.g., C₁₋₄ alkyl or C₁₋₃ alkyl, such as methyl, ethyl, propyl, isopropyl, butyl, sec-butyl or tert-butyl), or R₁₂ represents a bond;
X represents C(O) or CH₂; and
(Rₐ)ₜ indicates that the benzene ring is substituted with t Rₐ, with each Rₐ being the same or different and each independently representing bromine, and t represents an integer of 0, 1, 2 or 3.

In some embodiments, X represents C(O).

In some embodiments, X represents CH₂.

In some embodiments, R₁₂ represents a bond.

In some embodiments, t represents an integer of 0.

In some embodiments, t represents an integer of 1, 2 or 3.

In some embodiments, R₁₁ represents the structure of the following formula:

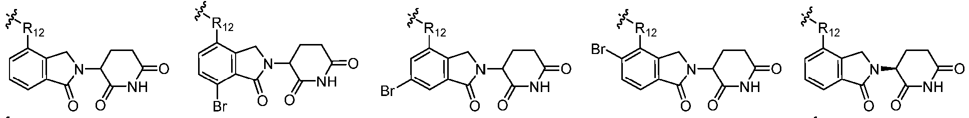

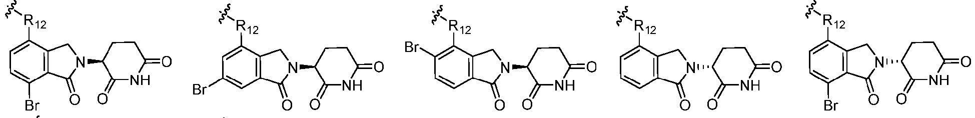

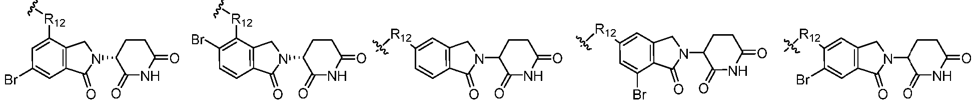

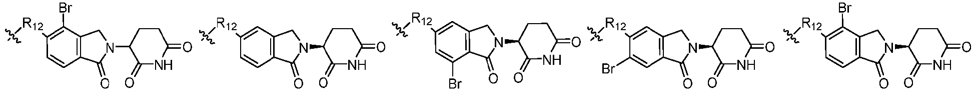

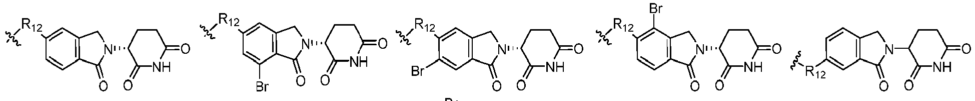

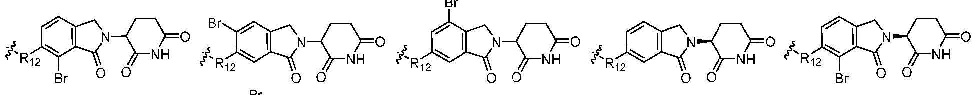

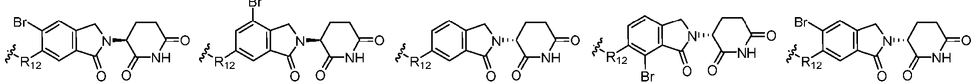

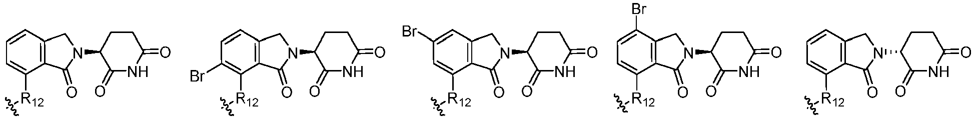

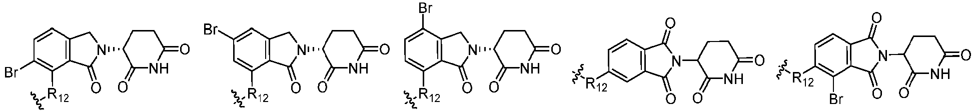

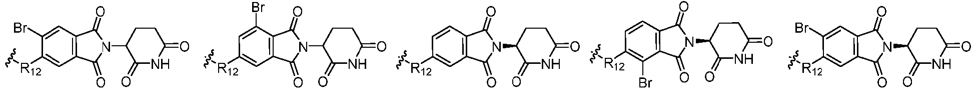

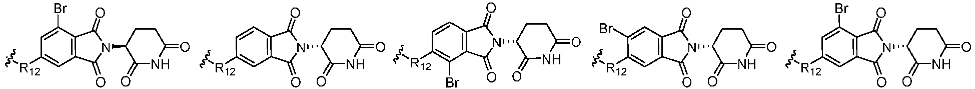

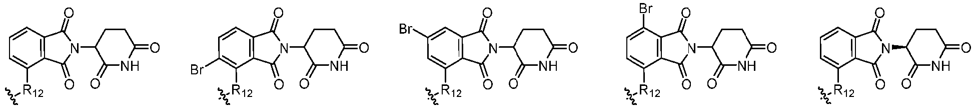

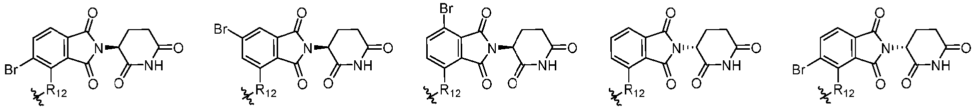

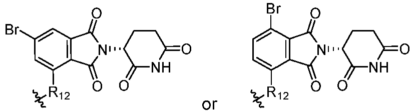

Herein, in case that t represents an integer of 0, when R₇ represents 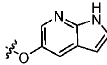 , n1 represents an integer of 1-8 (e.g., an integer of 1, 2, 3, 4, 5, 6, 7 or 8), and R^{a1} are the same or different and each independently represents C₁₋₆ alkyl (e.g., C₁₋₄ alkyl or C₁₋₃ alkyl, such as methyl, ethyl, propyl, isopropyl, butyl, sec-butyl or tert-butyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy or C₁₋₃ alkoxy, such as methoxy, ethoxy, propoxy, isopropoxy, butoxy, sec-butoxy or tert-butoxy), or halogen (e.g., fluorine, chlorine, bromine, or iodine).

Herein, in case that t represents an integer of 0, when R₇ represents 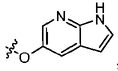 , R₈ represents -SO₂CF₃.

In some embodiments, the LIN represents the structure of the following formula:

#-C₁₋₃₀ alkylene -;

#-(C(R^{a3})(R^{a4}))ₙ₄-(R_{b}-(C(R^{a5})(R^{a6}))ₙ₅)ₘ₁-;

#-(C(R^{a3})(R^{a4}))ₙ₄-(R_{b}-(C(R^{a5})(R^{a6}))ₙ₅)ₘ₁-(R_{b}-(C(R^{a7})(R^{a8}))ₙ₆)ₘ₂-;

#-(C(R^{a3})(R^{a4}))ₙ₄-(R_{b}-(C(R^{a5})(R^{a6}))ₙ₅)ₘ₁-(R_{b}-(C(R^{a7})(R^{a8}))ₙ₆)ₘ₂-(R_{b}-(C(R^{a9})(R^{a10}))ₙ₇)ₘ₃-;

#-(C(R^{a3})(R^{a4}))ₙ₄-(R_{c}-(C(R^{a5})(R^{a6}))ₙ₅)ₘ₁-;

#-(C(R^{a3})(R^{a4}))ₙ₄-(R_{c}-(C(R^{a5})(R^{a6}))ₙ₅)ₘ₁-(R_{c}-(C(R^{a7})(R^{a8}))ₙ₆)ₘ₂-;

#-(C(R^{a3})(R^{a4}))ₙ₄-(R_{c}-(C(R^{a5})(R^{a6}))ₙ₅)ₘ₁-(R_{c}-(C(R^{a7})(R^{a8}))ₙ₆)ₘ₂-(R_{c}-(C(R^{a9})(R^{a10}))ₙ₇)ₘ₃-;

#-(C(R^{a3})(R^{a4}))n₄-(R_{b}-R_{c}-(C(R^{a5})(R^{a6}))ₙ₅)ₘ₁-;

#-(C(R^{a3})(R^{a4}))ₙ₄-(R_{b}-(C(R^{a5})(R^{a6}))ₙ₅)ₘ₁-(R_{c}-(C(R^{a7})(R^{a8}))ₙ₆)ₘ₂-;

#-(C(R^{a3})(R^{a4}))ₙ₄-(R_{c}-R_{b}-(C(R^{a5})(R^{a6}))ₙ₅)ₘ₁-; or

#-(C(R^{a3})(R^{a4}))ₙ₄-(R_{c}-(C(R^{a5})(R^{a6}))ₙ₅)ₘ₁-(R_{b}-(C(R^{a7})(R^{a8}))ₙ₆)ₘ₂-;

wherein each group R_{b} is selected from the group consisting of O, N(Ra), C(O), C(O)O, OC(O), S(O), S(O)₂, S(O)₂NH, NHS(O)₂, C(O)N(R_{d}), N(R_{d})C(O), or N(R_{d})C(O)N(R_{d}), wherein each R_{d} independently represents H or C₁₋₆ alkyl (e.g., C₁₋₄ alkyl, such as methyl, ethyl, propyl, isopropyl, butyl, sec-butyl or tert-butyl); each group R_{c} is selected from the group consisting of cycloalkylene (e.g., C₃₋₂₀ cycloalkylene), arylene (e.g., C₃₋₂₀ arylene), heterocyclylene (e.g., 4- to 20-membered heterocyclylene), heteroarylene (e.g., 4- to 20-membered heteroarylene), alkynylene (e.g., C₂₋₆ alkynylene), alkenylene (e.g., C₂₋₆ alkenylene) or any combination thereof, wherein the cycloalkylene, the arylene, the heterocyclylene and the heteroarylene are each independently optionally substituted with a substituent selected from the group consisting of C₁₋₆ alkyl (e.g., C₁₋₄ alkyl or C₁₋₃ alkyl, such as methyl, ethyl, propyl, isopropyl, butyl, sec-butyl or tert-butyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), halogen (e.g., fluorine, chlorine, bromine, or iodine), C₁₋₆ alkoxy (e.g., C₁₋₄ alkoxy or C₁₋₃ alkoxy, such as methoxy, ethoxy, propoxy, isopropoxy, butoxy, sec-butoxy or tert-butoxy), or any combination thereof;
a hydrogen atom of one or more CH₂ of the C₁₋₃₀ alkylene is optionally replaced by a substituent selected from the group consisting of: C₁₋₆ alkyl (e.g., C₁₋₄ alkyl or C₁₋₃ alkyl, such as methyl, ethyl, propyl, isopropyl, butyl, sec-butyl or tert-butyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), halogen (e.g., fluorine, chlorine, bromine, or iodine), C₁₋₆ alkoxy (e.g., C₁₋₄ alkoxy or C₁₋₃ alkoxy, such as methoxy, ethoxy, propoxy, isopropoxy, butoxy, sec-butoxy or tert-butoxy), or any combination thereof;
R^{a3}, R^{a4}, R^{a5}, R^{a6}, R^{a7}, R^{a8}, R^{a9}, and R^{a10} are the same or different and each independently represents H, C₁₋₆ alkyl (e.g., C₁₋₄ alkyl or C₁₋₃ alkyl, such as methyl, ethyl, propyl, isopropyl, butyl, sec-butyl or tert-butyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), halogen (e.g., fluorine, chlorine, bromine, or iodine), or C₁₋₆ alkoxy (e.g., C₁₋₄ alkoxy or C₁₋₃ alkoxy, such as methoxy, ethoxy, propoxy, isopropoxy, butoxy, sec-butoxy or tert-butoxy);
n4, n5, n6, n7, m1, m2, and m3 are each independently selected from the group consisting of an integer of 1, 2, 3, 4, 5, 6, 7, 8, 9, 10, 11, 12, 13, 14, or 15; and
symbol # indicates the point of attachment to R₁₀.

In some embodiments, the LIN represents the structure of the following formula:

#-(C(R^{a3})(R^{a4}))ₙ₄-(O(C(R^{a5})(R^{a6}))ₙ₅)ₘ₁-;

#-(C(R^{a3})(R^{a4}))ₙ₄-(O-(C(R^{a5})(R^{a6}))ₙ₅)ₘ₁-(O-(C(R^{a7})(R^{a8}))ₙ₆)ₘ₂-;

#-(C(R^{a3})(R^{a4})ₙ₄-(O-(C(R^{a5})(R^{a6}))ₙ₅)ₘ₁-(O-(C(R^{a7})(R^{a8}))ₙ₆)ₘ₂-(O(C(R^{a9})(R^{a10}))ₙ₇)ₘ₃-;

#-(C(R^{a3})(R^{a4}))ₙ₄-(N(R_{d})-(C(R^{a5})(R^{a6}))ₙ₅)ₘ₁-;

#-(C(R^{a3})(R^{a4}))ₙ₄-(N(R_{d})-(C(R^{a5})(R^{a6}))ₙ₅))ₘ₁-(N(R_{d})-(C(R^{a7})(R^{a8}))ₙ₆)ₘ₂-;

#-(C(R^{a3})(R^{a4}))ₙ₄-(N(R_{d})-(C(R^{a5})(R^{a6}))ₙ₅)ₘ₁-(N(R_{d})-(C(R^{a7})(R^{a7})(R^{a8}))ₙ₆)ₘ₂-(N(R_{d})-(C(R^{a9})(R^{a10}))ₙ₇)ₘ₃-;

#-(C(R^{a3})(R^{a4}))ₙ₄-(C(O)N(R_{d})-(C(R^{a5})(R^{a6}))ₙ₅)ₘ₁-;

#-(C(R^{a3})(R^{a4}))ₙ₄-(C(O)N(R_{d})-(C(R^{a5})(R^{a6}))ₙ₅)ₘ₁-(C(O)N(R_{d})-(C(R^{a7})(R^{a8}))ₙ₆)ₘ₂-;

#-(C(R^{a3})(R^{a4}))ₙ₄-(C(O)N(R_{d})-(C(R^{a5})(R^{a6}))ₙ₅)ₘ₁-(C(O)N(R_{d})-(C(R^{a7})(R^{a8}))ₙ₆)ₘ₂-(C(O)N(R_{d})- (C(R^{a9})(R^{a10}))ₙ₇)ₘ₃-;

#-(C(R^{a3})(R^{a4}))ₙ₄-(C(O)N(R_{d})-(C(R^{a5})(R^{a6}))ₙ₅)ₘ₁-(O-(C(R^{a7})(R^{a8}))ₙ₆)ₘ₂-;

#-(C(R^{a3})(R^{a4}))ₙ₄-C(O)N(R_{d})-(C(R^{a5})(R^{a6}))ₙ₅-(O-(C(R^{a7})(R^{a8}))ₙ₆)ₘ₁-;

#-(C(R^{a3})(R^{a4}))ₙ₄-(N(R_{d})C(O)-(C(R^{a5})(R^{a6}))ₙ₅)ₘ₁-;

#-(C(R^{a3})(R^{a4}))ₙ₄-(N(R_{d})C(O)-(C(R^{a5})(R^{a6}))ₙ₅)ₘ₁-(O-(C(R^{a7})(R^{a8}))ₙ₆)ₘ₂-;

#-(C(R^{a3})(R^{a4}))ₙ₄-(N(R_{d})C(O)-(C(R^{a5})(R^{a6}))ₙ₅)ₘ₁-(O-(C(R^{a7})(R^{a8}))ₙ₆)ₘ₂-(O-(C(R^{a9})(R^{a10}))ₙ₇)ₘ₃-;

#-(C(R^{a3})(R^{a4}))ₙ₄-N(R_{d})C(O)-(C(R^{a5})(R^{a6}))ₙ₅-(O-(C(R^{a7})(R^{a8}))ₙ₆)ₘ₁-;

#-(C(R^{a3})(R^{a4}))ₙ₄-N(R_{d})C(O)N(R_{d})-(C(R^{a5})(R^{a6}))ₙ₅-;

#-(C(R^{a3})(R^{a4}))ₙ₄-C(O)-(C(R^{a5})(R^{a6}))ₙ₅-;

#-(C(R^{a3})(R^{a4}))ₙ₄-(arylene-(C(R^{a5})(R^{a6}))ₙ₅)ₘ₁-;

#-(C(R^{a3})(R^{a4}))ₙ₄-(arylene-(C(R^{a5})(R^{a6}))ₙ₅)ₘ₁-arylene-(C(R^{a7})(R^{a8}))ₙ₆-;

#-(C(R^{a3})(R^{a4}))ₙ₄-(heterocyclylene-(C(R^{a5})(R^{a6}))ₙ₅)ₘ₁-;

#-(C(R^{a3})(R^{a4}))ₙ₄-(heterocyclylene-(C(R^{a5})(R^{a6}))ₙ₅)ₘ₁-(heterocyclylene-(C(R^{a7})(R^{a8}))ₙ₆)ₘ₂-;

#-(C(R^{a3})(R^{a4}))ₙ₄-(heteroarylene-(C(R^{a5})(R^{a6}))ₙ₅)ₘ₁-;

#-(C(R^{a3})(R^{a4}))ₙ₄-(heteroarylene-(C(R^{a5})(R^{a6}))ₙ₅)ₘ₁-(heteroarylene-(C(R^{a7})(R^{a8}))ₙ₆)ₘ₂-;

#-(C(R^{a3})(R^{a4}))ₙ₄-(cycloalkylene-(C(R^{a5})(R^{a6}))ₙ₅)ₘ₁-; or

#-(C(R^{a3})(R^{a4}))ₙ₄-(cycloalkylene-(C(R^{a5})(R^{a6}))ₙ₅)ₘ₁-(cycloalkylene-(C(R^{a7})(R^{a8}))ₙ₆)ₘ₂-;

wherein each R_{d} independently represents H or C₁₋₆ alkyl (e.g., C₁₋₄ alkyl or C₁₋₃ alkyl, such as methyl, ethyl, propyl, isopropyl, butyl, sec-butyl or tert-butyl);
the cycloalkylene (e.g., C₃₋₂₀ cycloalkylene), the arylene (e.g., C₃₋₂₀ arylene), the heterocyclylene (e.g., 4- to 20-membered heterocyclylene) and the heteroarylene (e.g., 4- to 20-membered heteroarylene) are each independently optionally substituted with a substituent selected from the group consisting of C₁₋₆ alkyl (e.g., C₁₋₄ alkyl or C₁₋₃ alkyl, such as methyl, ethyl, propyl, isopropyl, butyl, sec-butyl or tert-butyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), halogen (e.g., fluorine, chlorine, bromine, or iodine), C₁₋₆ alkoxy (e.g., C₁₋₄ alkoxy or C₁₋₃ alkoxy, such as methoxy, ethoxy, propoxy, isopropoxy, butoxy, sec-butoxy or tert-butoxy), or any combination thereof;
R^{a3}, R^{a4}, R^{a5}, R^{a6}, R^{a7}, R^{a8}, R^{a9}, and R^{a10} are the same or different and each independently represents H, C₁₋₆ alkyl (e.g., C₁₋₄ alkyl or C₁₋₃ alkyl, such as methyl, ethyl, propyl, isopropyl, butyl, sec-butyl or tert-butyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), halogen (e.g., fluorine, chlorine, bromine, or iodine), or C₁₋₆ alkoxy (e.g., C₁₋₄ alkoxy or C₁₋₃ alkoxy, such as methoxy, ethoxy, propoxy, isopropoxy, butoxy, sec-butoxy or tert-butoxy);
n4, n5, n6, n7, m1, m2, and m3 are each independently selected from the group consisting of an integer of 1, 2, 3, 4, 5, 6, 7, 8, 9, 10, 11, 12, 13, 14, or 15; and
symbol # indicates the point of attachment to R₁₀.

In some embodiments, the LIN represents the following groups:

#-CH₂-, #-(CH₂)₂-, #-(CH₂)₃-, #-(CH₂)₄-, #-(CH₂)₅-, #-(CH₂)₆-, #-(CH₂)₇-, #-(CH₂)₈-, #-(CH₂)₉-, #-(CH₂)₁₀-, #-(CH₂)₁₁-, #-(CH₂)₁₂-, #-(CH₂)₁₃-, #-(CH₂)₁₄-, #-(CH₂)₁₅-, #-(CH₂)₁₆-, #-(CH₂)₁₇-, #- (CH₂)₁₈-, #-(CH₂)₁₉-, #-(CH₂)₂₀-, #-(CH₂)₂₁-, #-(CH₂)₂₂-, #-(CH₂)₂₅-, or #-(CH₂)₃₀-;#-CH₂-O-CH₂-, #- CH₂-O-(CH₂)₂-, #-(CH₂)₁-O-(CH₂)₃-, #-(CH₂)₁-O-(CH₂)₄-, #-(CH₂)₁-O-(CH₂)₅-, #-(CH₂)₁-O-(CH₂)₆-, #-(CH₂)₁-O-(CH₂)₇-, #-(CH₂)₁-O-(CH₂)₈-, #-(CH₂)₁-O-(CH₂)₉-, #-(CH₂)₁-O-(CH₂)₁₀-, #-(CH₂)₂-O- (CH₂)₁-, #-(CH₂)₂-O-(CH₂)₂-, #-(CH₂)₂-O-(CH₂)₃-, #-(CH₂)₂-O-(CH₂)₄-, #-(CH₂)₂-O-(CH₂)₅-, #- (CH₂)₂-O-(CH₂)₆-, #-(CH₂)₂-O-(CH₂)₇-, #-(CH₂)₂-O-(CH₂)₈-, #-(CH₂)₂-O-(CH₂)₉-, #-(CH₂)₂-O- (CH₂)₁₀-, #-(CH₂)₂-O-(CH₂)₁₁-, #-(CH₂)₂-O-(CH₂)₁₂-, #-(CH₂)₃-O-(CH₂)₁-, #-(CH₂)₃-O-(CH₂)₂-, #- (CH₂)₃-O-(CH₂)₃-, #-(CH₂)₃-O-(CH₂)₄-, #-(CH₂)₃-O-(CH₂)₅-, #-(CH₂)₃-O-(CH₂)₆-, #-(CH₂)₃-O- (CH₂)₇-, #-(CH₂)₄-O-(CH₂)₁-, #-(CH₂)₄-O-(CH₂)₂-, #-(CH₂)₄-O-(CH₂)₃-, #-(CH₂)₄-O-(CH₂)₄-, #- (CH₂)₄-O-(CH₂)₅-, #-(CH₂)₄-O-(CH₂)₆-, #-(CH₂)₅-O-(CH₂)₁-, #-(CH₂)₅-O-(CH₂)₂-, #-(CH₂)₅-O- (CH₂)₃-, #-(CH₂)₅-O-(CH₂)₄-, #-(CH₂)₅-O-(CH₂)₅-, #-(CH₂)₆-O-(CH₂)₁-, #-(CH₂)₆-O-(CH₂)₂-, #- (CH₂)₆-O-(CH₂)₃-, #-(CH₂)₆-O-(CH₂)₄-, #-(CH₂)₇-O-(CH₂)₁-, #-(CH₂)₇-O-(CH₂)₂-, #-(CH₂)₇-O- (CH₂)₃-, #-(CH₂)₈-O-(CH₂)₁-, #-(CH₂)₈-O-(CH₂)₂-, #-CH(CH₃)-O-(CH₂)₁-, #-CH(CH₃)-O-(CH₂)₂-, #- CH(CH₃)-O-(CH₂)₃-, #-CH(CH₃)-O-(CH₂)₄-, #-CH(CH₃)-O-(CH₂)₅-, #-CH(CH₃)-O-(CH₂)₆-, #- CH(CH₃)-O-(CH₂)₇-, #-CH(CH₃)-O-(CH₂)₈-, #-CH(CH₃)-O-(CH₂)₉-, #-CH(CH₃)-O-(CH₂)₁₀-, #-CH₂- (O(CH₂)₂)₂-, #-CH₂-(O(CH₂)₂)₃-, #-CH₂-(O(CH₂)₂)₄-, #-CH₂-(O(CH₂)₂)₅-, #-CH₂-(O(CH₂)₂)₆-, #-CH₂- (O(CH₂)₂)₇-, #-CH₂-(O(CH₂)₂)₈-, #-CH₂-(O(CH₂)₂)₉-, #-CH₂-(O(CH₂)₂)₁₀-, #-CH₂-(O(CH₂)₂)₁-OCH₂-, #-CH₂-(O(CH₂)₂)₂-OCH₂-, #-CH₂-(O(CH₂)₂)₃-OCH₂-, #-CH₂-(O(CH₂)₂)₄-OCH₂-, #-CH₂-(O(CH₂)₂)₅- OCH₂-, #-CH₂-(O(CH₂)₂)₆-OCH₂-, #-CH₂-(O(CH₂)₂)₇-OCH₂-, #-CH₂-(O(CH₂)₂)₈-OCH₂-, #-CH₂- (O(CH₂)₂)₉-OCH₂-, #-CH₂-(O(CH₂)₂)₁₀-OCH₂-, #-(CH₂)₂-(O(CH₂)₂)₂-, #-(CH₂)₂-(O(CH₂)₂)₃-, #- (CH₂)₂-(O(CH₂)₂)₄-, #-(CH₂)₂-(O(CH₂)₂)₅-, #-(CH₂)₂-(O(CH₂)₂)₆-, #-(CH₂)₂-(O(CH₂)₂)₇-, #-(CH₂)₂- (O(CH₂)₂)₈-, #-(CH₂)₂-(O(CH₂)₂)₉-, #-(CH₂)₂-(O(CH₂)₂)₁₀-, #-(CH₂)₃-(O(CH₂)₂)₂-, #-(CH₂)₃- (O(CH₂)₂)₃-, #-(CH₂)₃-(O(CH₂)₂)₄-, #-(CH₂)₃-(O(CH₂)₂)₅-, #-(CH₂)₃-(O(CH₂)₂)₆-, #-(CH₂)₃- (O(CH₂)₂)₇-, #-(CH₂)₃-(O(CH₂)₂)₈-, #-(CH₂)₃-(O(CH₂)₂)₉-, #-(CH₂)₃-(O(CH₂)₂)₁₀-, #-(CH₂)₄- (O(CH₂)₂)₂-, #-(CH₂)₄-(O(CH₂)₂)₃-, #-(CH₂)₄-(O(CH₂)₂)₄-, #-(CH₂)₄-(O(CH₂)₂)₅-, #-(CH₂)₄- (O(CH₂)₂)₆-, #-(CH₂)₄-(O(CH₂)₂)₇-, #-(CH₂)₄-(O(CH₂)₂)₈-, #-(CH₂)₄-(O(CH₂)₂)₉-, #-(CH₂)₄- (O(CH₂)₂)₁₀-, #-CH₂-(O(CH₂)₃)₂-, #-CH₂-(O(CH₂)₃)₃-, #-CH₂-(O(CH₂)₃)₄-, #-CH₂-(O(CH₂)₃)₅-, #- CH₂-(O(CH₂)₃)₆-, #-CH₂-(O(CH₂)₃)₇-, #-CH₂-(O(CH₂)₃)₈-, #-CH₂-(O(CH₂)₃)₉-, #-CH₂-(O(CH₂)₃)₁₀-, #-(CH₂)₂-(O(CH₂)₃)₂-, #-(CH₂)₂-(O(CH₂)₃)₃-, #-(CH₂)₂-(O(CH₂)₃)₄-, #-(CH₂)₂-(O(CH₂)₃)₅-, #-(CH₂)₂- (O(CH₂)₃)₆-, #-(CH₂)₂-(O(CH₂)₃)₇-, #-(CH₂)₂-(O(CH₂)₃)₈-, #-(CH₂)₂-(O(CH₂)₃)₉-, #-(CH₂)₂- (O(CH₂)₃)₁₀-, #-(CH₂)₃-(O(CH₂)₃)₂-, #-(CH₂)₃-(O(CH₂)₃)₃-, #-(CH₂)₃-(O(CH₂)₃)₄-, #-(CH₂)₃- (O(CH₂)₃)₅-, #-(CH₂)₃-(O(CH₂)₃)₆-, #-(CH₂)₃-(O(CH₂)₃)₇-, #-(CH₂)₃-(O(CH₂)₃)₈-, #-(CH₂)₃- (O(CH₂)₃)₉-, #-(CH₂)₃-(O(CH₂)₃)₁₀-, #-CH₂-O-(CH₂)₂-O-(CH₂)₃-, #-CH₂-(O(CH₂)₂)₂-(O(CH₂)₃)₂-, #- CH₂-(O(CH₂)₂)₃-(O(CH₂)₃)₃-, #-CH₂-(O(CH₂)₂)₄-(O(CH₂)₃)₄-, #-CH₂-(O(CH₂)₂)₅-(O(CH₂)₃)₅-, #-CH₂- (O(CH₂)₂)₆-(O(CH₂)₃)₆-, #-(CH₂)₂-O-(CH₂)₂-O-(CH₂)₃-, #-(CH₂)₂-(O(CH₂)₂)₂-(O(CH₂)₃)₂-, #-(CH₂)₂- (O(CH₂)₂)₃-(O(CH₂)₃)₃-, #-(CH₂)₂-(O(CH₂)₂)₄-(O(CH₂)₃)₄-, #-(CH₂)₂-(O(CH₂)₂)₅-(O(CH₂)₃)₅-, #- (CH₂)₂-(O(CH₂)₂)₆-(O(CH₂)₃)₆-, #-(CH₂)₃-O-(CH₂)₂-O-(CH₂)₃-, #-(CH₂)₃-(O(CH₂)₂)₂-(O(CH₂)₃)₂-, #- (CH₂)₃-(O(CH₂)₂)₃-(O(CH₂)₃)₃-, #-(CH₂)₃-(O(CH₂)₂)₄-(O(CH₂)₃)₄-, #-(CH₂)₃-(O(CH₂)₂)₅-(O(CH₂)₃)₅-, #-(CH₂)₃-(O(CH₂)₂)₆-(O(CH₂)₃)₆-, #-CH₂-O-(CH₂)₃-O-(CH₂)₂-, #-CH₂-(O(CH₂)₃)₂-(O(CH₂)₂)₂-, #- CH₂-(O(CH₂)₃)₃-(O(CH₂)₂)₃-, #-CH₂-(O(CH₂)₃)₄-(O(CH₂)₂)₄-, #-CH₂-(O(CH₂)₃)₅-(O(CH₂)₂)₅-, #-CH₂- (O(CH₂)₃)₆-(O(CH₂)₂)₆-, #-(CH₂)₂-O-(CH₂)₃-O-(CH₂)₂-, #-(CH₂)₂-(O(CH₂)₃)₂-(O(CH₂)₂)₂-, #-(CH₂)₂- (O(CH₂)₃)₃-(O(CH₂)₂)₃-, #-(CH₂)₂-(O(CH₂)₃)₄-(O(CH₂)₂)₄-, #-(CH₂)₂-(O(CH₂)₃)₅-(O(CH₂)₂)₅-, #- (CH₂)₂-(O(CH₂)₃)₆-(O(CH₂)₂)₆-, #-(CH₂)₃-O-(CH₂)₃-O-(CH₂)₂-, #-(CH₂)₃-(O(CH₂)₃)₂-(O(CH₂)₂)₂-, #- (CH₂)₃-(O(CH₂)₃)₃-(O(CH₂)₂)₃-, #-(CH₂)₃-(O(CH₂)₃)₄-(O(CH₂)₂)₄-, #-(CH₂)₃-(O(CH₂)₃)₅-(O(CH₂)₂)₅-, #-(CH₂)₃-(O(CH₂)₃)₆-(O(CH₂)₂)₆-, #-CH₂-O-(CH₂)₂-O-CH₂-, #-(CH₂)₂-O-(CH₂)₂-O-CH₂-, #-(CH₂)₂- (O(CH₂)₂)₂-O-(CH₂)₃-, #-(CH₂)₂-(O(CH₂)₂)₃-O-(CH₂)₃-, #-(CH₂)₂-(O(CH₂)₂)₄-O-(CH₂)₃-, #-(CH₂)₅- (O(CH₂)₂)₂-O-(CH₂)₅-, #-(CH₂)₅-(O(CH₂)₂)₂-O-(CH₂)₆-, #-(CH₂)₁-N(R_{g})-(CH₂)₁-, #-(CH₂)₁-N(R_{g})- (CH₂)₂-, #-(CH₂)₁-N(R_{g})-(CH₂)₃-, #-(CH₂)₁-N(R_{g})-(CH₂)₄-, #-(CH₂)₁-N(R_{g})-(CH₂)₅-, #-(CH₂)₁-N(R_{g})- (CH₂)₆-, #-(CH₂)₁-N(R_{g})-(CH₂)₇-, #-(CH₂)₁-N(R_{g})-(CH₂)₈-, #-(CH₂)₁-N(R_{g})-(CH₂)₉-, #-(CH₂)₁-N(R_{g})- (CH₂)₁₀-, #-(CH₂)₂-N(R_{g})-(CH₂)₁-, #-(CH₂)₂-N(R_{g})-(CH₂)₂-, #-(CH₂)₂-N(R_{g})-(CH₂)₃-, #-(CH₂)₂-N(R_{g})- (CH₂)₄-, #-(CH₂)₂-N(R_{g})-(CH₂)₅-, #-(CH₂)₂-N(R_{g})-(CH₂)₆-, #-(CH₂)₂-N(R_{g})-(CH₂)₇-, #-(CH₂)₂-N(R_{g})- (CH₂)₈-, #-(CH₂)₂-N(R_{g})-(CH₂)₉-, #-(CH₂)₂-N(R_{g})-(CH₂)₁₀-, #-(CH₂)₂-N(R_{g})-(CH₂)₁₁-, #-(CH₂)₂- N(R_{g})-(CH₂)₁₂-, #-(CH₂)₃-N(R_{g})-(CH₂)₁-, #-(CH₂)₃-N(R_{g})-(CH₂)₂-, #-(CH₂)₃-N(R_{g})-(CH₂)₃-, #-(CH₂)₄- N(R_{g})-(CH₂)₁-, #-(CH₂)₄-N(R_{g})-(CH₂)₂-, #-(CH₂)₄-N(R_{g})-(CH₂)₃-, #-(CH₂)₄-N(R_{g})-(CH₂)₄-, #-(CH₂)₅- N(R_{g})-(CH₂)₁-, #-(CH₂)₅-N(R_{g})-(CH₂)₂-, #-(CH₂)₅-N(R_{g})-(CH₂)₃-, #-(CH₂)₅-N(R_{g})-(CH₂)₄-, #-(CH₂)₅- N(R_{g})-(CH₂)₅-, #-(CH₂)₆-N(R_{g})-(CH₂)₁-, #-(CH₂)₆-N(R_{g})-(CH₂)₂-, #-(CH₂)₆-N(R_{g})-(CH₂)₃-, #-(CH₂)₇- N(R_{g})-(CH₂)₁-, #-(CH₂)₇-N(R_{g})-(CH₂)₂-, #-(CH₂)₇-N(R_{g})-(CH₂)₃-, #-(CH₂)₈-N(R_{g})-(CH₂)₁-, #-(CH₂)₈- N(R_{g})-(CH₂)₂-, #-(CH₂)₈-N(R_{g})-(CH₂)₃-, #-CH(CH₃)-N(R_{g})-(CH₂)₁-, #-CH(CH₃)-N(R_{g})-(CH₂)₂-, #- CH(CH₃)-N(R_{g})-(CH₂)₃-, #-CH(CH₃)-N(R_{g})-(CH₂)₄-, #-CH(CH₃)-N(R_{g})-(CH₂)₅-, #-CH(CH₃)-N(R_{g})- (CH₂)₆-, #-CH(CH₃)-N(R_{g})-(CH₂)₇-, #-CH(CH₃)-N(R_{g})-(CH₂)₈-, #-CH(CH₃)-N(R_{g})-(CH₂)₉-, #- CH(CH₃)-N(R_{g})-(CH₂)₁₀-, #-CH₂C(O)NHCH₂-, #-(CH₂)₂C(O)NH(CH₂)₂-, #-(CH₂)₂C(O)NH(CH₂)₃-, #-(CH₂)₂C(O)NH(CH₂)₄-, #-(CH₂)₂C(O)NH(CH₂)₅-, #-(CH₂)₃C(O)NH(CH₂)₃-, #- (CH₂)₃C(O)NH(CH₂)₄-, #-(CH₂)₄C(O)NH(CH₂)₄-, #-(CH₂)₅C(O)NH(CH₂)₅-, #- (CH₂)₆C(O)NH(CH₂)₇-, #-(CH₂)₆C(O)NH(CH₂)₆-, #-(CH₂)₇C(O)NH(CH₂)₇-, #-(CH₂)₈C(O)NH(CH₂)₈, U-(CH₂)₉C(O)NH(CH₂)₉-, #-(CH₂)₁₀C(O)NH(CH₂)₁₀-, #-(CH₂)₂C(O)NH(CH₂)₂-O-(CH₂)₂-, #- CH₂NHC(O)CH₂-, #-(CH₂)₂NHC(O)(CH₂)₂-, #-(CH₂)₂NHC(O)(CH₂)₃-, #-(CH₂)₂NHC(O)(CH₂)₄-, #- (CH₂)₂NHC(O)(CH₂)₅-, #-(CH₂)₃NHC(O)(CH₂)₃-, #-(CH₂)₃NHC(O)(CH₂)₄-, #- (CH₂)₄NHC(O)(CH₂)₄-, #-(CH₂)₅NHC(O)(CH₂)₅-, #-(CH₂)₆NHC(O)(CH₂)₇-, #- (CH₂)₆NHC(O)(CH₂)₆-, #-(CH₂)₇NHC(O)(CH₂)₇-, #-(CH₂)₈NHC(O)(CH₂)₈, #-(CH₂)₉NHC(O)(CH₂)₉-, #-(CH₂)₁₀NHC(O)(CH₂)₁₀-, #-(CH₂)₄NHC(O)(CH₂)₈-, #-(CH₂)₂NHC(O)(CH₂)₂-O-(CH₂)₂-, #- (CH₂)₄NHC(O)CH₂-, #-CH₂-piperidinylene-CH₂-, #-CH₂-piperidinylene-(CH₂)₂-, #-CH₂- piperidinylene-(CH₂)₃-, #-CH₂-piperidinylene-(CH₂)₄-, #-CH₂-piperidinylene-(CH₂)₅-, #-CH₂- ₂)₆-, #-CH₂-piperidinylene-(CH₂)₇-, #-CH₂-piperidinylene-(CH₂)₈-, #-(CH₂)₂- piperidinylene-(CH₂)₁-, #-(CH₂)₂-piperidinylene-(CH₂)₂-, #-(CH₂)₂-piperidinylene-(CH₂)₃-, #-(CH₂)₂- piperidinylene-(CH₂)₄-, #-(CH₂)₂-piperidinylene-(CH₂)₅-, #-(CH₂)₂-piperidinylene-(CH₂)₆-, #-(CH₂)₂- piperidinylene-(CH₂)₇-, #-(CH₂)₂-piperidinylene-(CH₂)₈-, #-(CH₂)₃-piperidinylene-CH₂-, #-(CH₂)₃- piperidinylene-(CH₂)₂-, #-(CH₂)₃-piperidinylene-(CH₂)₃-, #-(CH₂)₃-piperidinylene-(CH₂)₄-, #-(CH₂)₃- piperidinylene-(CH₂)₅-, #-(CH₂)₃-piperidinylene-(CH₂)₆-, #-(CH₂)₃-piperidinylene-(CH₂)₇-, #-(CH₂)₃- piperidinylene-(CH₂)₈-, #-(CH₂)₄-piperidinylene-CH₂-, #-(CH₂)₄-piperidinylene-(CH₂)₂-, #-(CH₂)₄- piperidinylene-(CH₂)₃-, #-(CH₂)₄-piperidinylene-(CH₂)₄-, #-(CH₂)₄-piperidinylene-(CH₂)₅-, #-(CH₂)₄- piperidinylene-(CH₂)₆-, #-(CH₂)₄-piperidinylene-(CH₂)₇-, #-(CH₂)₄-piperidinylene-(CH₂)₈-, #-(CH₂)₅- piperidinylene-(CH₂)₁-, #-(CH₂)₅-piperidinylene-(CH₂)₂-, #-(CH₂)₅-piperidinylene-(CH₂)₃-, #-(CH₂)₅- piperidinylene-(CH₂)₄-, #-(CH₂)₅-piperidinylene-(CH₂)₅-, #-(CH₂)₅-piperidinylene-(CH₂)₆-, #-(CH₂)₅- piperidinylene-(CH₂)₇-, #-(CH₂)₅-piperidinylene-(CH₂)₈-, #-(CH₂)₆-piperidinylene-(CH₂)₁-, #-(CH₂)₆- piperidinylene-(CH₂)₂-, #-(CH₂)₆-piperidinylene-(CH₂)₃-, #-(CH₂)₆-piperidinylene-(CH₂)₄-, #-(CH₂)₆- piperidinylene-(CH₂)₅-, #-(CH₂)₆-piperidinylene-(CH₂)₆-, #-(CH₂)₆-piperidinylene-(CH₂)₇-, #-(CH₂)₆- piperidinylene-(CH₂)₈-, #-(CH₂)₇-piperidinylene-(CH₂)₁-, #-(CH₂)₇-piperidinylene-(CH₂)₂-, #-(CH₂)₇- piperidinylene-(CH₂)₃-, #-(CH₂)₇-piperidinylene-(CH₂)₄-, #-(CH₂)₇-piperidinylene-(CH₂)₈-, #-(CH₂)₈- piperidinylene-CH₂-, #-(CH₂)₈-piperidinylene-(CH₂)₂-, #-(CH₂)₈-piperidinylene-(CH₂)₃-, #-(CH₂)₈- piperidinylene-(CH₂)₄-, #-(CH₂)₈-piperidinylene-(CH₂)₅-, #-(CH₂)₈-piperidinylene-(CH₂)₆-, #-(CH₂)₈- piperidinylene-(CH₂)₇-, #-(CH₂)₈-piperidinylene-(CH₂)₈-, #-CH₂-N(R_{g})-CH₂-piperidinylene-CH₂-, #- CH₂-N(R_{g})-CH₂-piperidinylene-(CH₂)₂-, #-CH₂-N(R_{g})-CH₂-piperidinylene-(CH₂)₃-, #-CH₂-N(R_{g})- CH₂-piperidinylene-(CH₂)₄-, #-CH₂-N(R_{g})-CH₂-piperidinylene-(CH₂)₅-, #-CH₂-N(R_{g})-CH₂- piperidinylene-(CH₂)₆, #-CH₂-N(R_{g})-CH₂-piperidinylene-(CH₂)₇-, #-CH₂-N(R_{g})-CH₂-piperidinylene- (CH₂)₈, #-CH₂-N(R_{g})-(CH₂)₂-piperidinylene-CH₂-, #-CH₂-N(R_{g})-(CH₂)₂-piperidinylene-(CH₂)₂-, #- CH₂-N(R_{g})-(CH₂)₂-piperidinylene-(CH₂)₃-, #-CH₂-N(R_{g})-(CH₂)₂-piperidinylene-(CH₂)₄-, #-CH₂- N(R_{g})-(CH₂)₂-piperidinylene-(CH₂)₅-, #-CH₂-N(R_{g})-(CH₂)₂-piperidinylene-(CH₂)₆-, #-CH₂-N(R_{g})- (CH₂)₂-piperidinylene-(CH₂)₇-, #-CH₂-N(R_{g})-(CH₂)₂-piperidinylene-(CH₂)₈-, #-(CH₂)₂-N(R_{g})-CH₂- piperidinylene-CH₂-, #-(CH₂)₂-N(R_{g})-CH₂-piperidinylene-(CH₂)₂-, #-(CH₂)₂-N(R_{g})-CH₂- piperidinylene-(CH₂)₃-, #-(CH₂)₂-N(R_{g})-CH₂-piperidinylene-(CH₂)₄-, #-(CH₂)₂-N(R_{g})-CH₂- piperidinylene-(CH₂)₅-, #-(CH₂)₂-N(R_{g})-CH₂-piperidinylene-(CH₂)₆-, #-(CH₂)₂-N(R_{g})-CH₂- piperidinylene-(CH₂)₇-, #-(CH₂)₂-N(R_{g})-CH₂-piperidinylene-(CH₂)₈-, #-(CH₂)₃-N(R_{g})-CH₂- piperidinylene-CH₂-, #-(CH₂)₃-N(R_{g})-CH₂-piperidinylene-(CH₂)₂-, #-(CH₂)₃-N(R_{g})-CH₂- piperidinylene-(CH₂)₃-, #-(CH₂)₃-N(R_{g})-CH₂-piperidinylene-(CH₂)₈-, #-(CH₂)₄-N(R_{g})-CH₂- piperidinylene-CH₂-, #-(CH₂)₄-N(R_{g})-CH₂-piperidinylene-(CH₂)₂-, #-(CH₂)₄-N(R_{g})-CH₂- piperidinylene-(CH₂)₃-, #-(CH₂)₄-N(R_{g})-CH₂-piperidinylene-(CH₂)₈-, #-(CH₂)₅-N(R_{g})-CH₂- piperidinylene-(CH₂)₃-, #-(CH₂)₅-N(R_{g})-CH₂-piperidinylene-(CH₂)₈-, #-(CH₂)₆-N(R_{g})-CH₂- piperidinylene-(CH₂)₃-, #-(CH₂)₆-N(R_{g})-CH₂-piperidinylene-(CH₂)₈-, #-(CH₂)₇-N(R_{g})-CH₂- piperidinylene-(CH₂)₃-, #-(CH₂)₇-N(R_{g})-CH₂-piperidinylene-(CH₂)₈-, #-(CH₂)₈-N(R_{g})-CH₂- piperidinylene-CH₂-, #-(CH₂)₈-N(R_{g})-CH₂-piperidinylene-(CH₂)₂-, #-(CH₂)₈-N(R_{g})-CH₂- piperidinylene-(CH₂)₃-, #-(CH₂)₈-N(R_{g})-CH₂-piperidinylene-(CH₂)₄-, #-(CH₂)₈-N(R_{g})-CH₂- piperidinylene-(CH₂)₅-, #-(CH₂)₈-N(R_{g})-CH₂-piperidinylene-(CH₂)₆-, #-(CH₂)₈-N(R_{g})-CH₂- piperidinylene-(CH₂)₇-, #-(CH₂)₈-N(R_{g})-CH₂-piperidinylene-(CH₂)₈-, #-CH₂-piperazinylene-CH₂-, #- CH₂-piperazinylene-(CH₂)₂-, #-CH₂-piperazinylene-(CH₂)₃-, #-CH₂-piperazinylene-(CH₂)₄-, #-CH₂- piperazinylene-(CH₂)₅-, #-CH₂-piperazinylene-(CH₂)₆-, #-CH₂-piperazinylene-(CH₂)₇-, #-CH₂- piperazinylene-(CH₂)₈-, #-(CH₂)₂-piperazinylene-(CH₂)₁-, #-(CH₂)₂-piperazinylene-(CH₂)₂-, #-(CH₂)₂- piperazinylene-(CH₂)₃-, #-(CH₂)₂-piperazinylene-(CH₂)₄-, #-(CH₂)₂-piperazinylene-(CH₂)₅-, #-(CH₂)₂- piperazinylene-(CH₂)₆-, #-(CH₂)₂-piperazinylene-(CH₂)₇-, #-(CH₂)₂-piperazinylene-(CH₂)₈-, #-(CH₂)₃- piperazinylene-CH₂-, #-(CH₂)₃-piperazinylene-(CH₂)₂-, #-(CH₂)₃-piperazinylene-(CH₂)₃-, #-(CH₂)₃- piperazinylene-(CH₂)₄-, #-(CH₂)₃-piperazinylene-(CH₂)₅-, #-(CH₂)₃-piperazinylene-(CH₂)₆-, #-(CH₂)₃- piperazinylene-(CH₂)₇-, #-(CH₂)₃-piperazinylene-(CH₂)₈-, #-(CH₂)₄-piperazinylene-CH₂-, #-(CH₂)₄- piperazinylene-(CH₂)₂-, #-(CH₂)₄-piperazinylene-(CH₂)₃-, #-(CH₂)₄-piperazinylene-(CH₂)₄-, #-(CH₂)₄- piperazinylene-(CH₂)₅-, #-(CH₂)₄-piperazinylene-(CH₂)₆-, #-(CH₂)₄-piperazinylene-(CH₂)₇-, #-(CH₂)₄- piperazinylene-(CH₂)₈-, #-(CH₂)₅-piperazinylene-(CH₂)₁-, #-(CH₂)₅-piperazinylene-(CH₂)₂-, #-(CH₂)₅- piperazinylene-(CH₂)₃-, #-(CH₂)₅-piperazinylene-(CH₂)₄-, #-(CH₂)₅-piperazinylene-(CH₂)₅-, #-(CH₂)₅- piperazinylene-(CH₂)₆-, #-(CH₂)₅-piperazinylene-(CH₂)₇-, #-(CH₂)₅-piperazinylene-(CH₂)₈-, #-(CH₂)₆- piperazinylene-(CH₂)₁-, #-(CH₂)₆-piperazinylene-(CH₂)₂-, #-(CH₂)₆-piperazinylene-(CH₂)₃-, #-(CH₂)₆- piperazinylene-(CH₂)₄-, #-(CH₂)₆-piperazinylene-(CH₂)₅-, #-(CH₂)₆-piperazinylene-(CH₂)₆-, #-(CH₂)₆- piperazinylene-(CH₂)₇-, #-(CH₂)₆-piperazinylene-(CH₂)₈-, #-(CH₂)₇-piperazinylene-(CH₂)₁-, #-(CH₂)₇- piperazinylene-(CH₂)₂-, #-(CH₂)₇-piperazinylene-(CH₂)₃-, #-(CH₂)₇-piperazinylene-(CH₂)₄-, #-(CH₂)₇- piperazinylene-(CH₂)₈-, #-(CH₂)₈-piperazinylene-CH₂-, #-(CH₂)₈-piperazinylene-(CH₂)₂-, #-(CH₂)₈- piperazinylene-(CH₂)₃-, #-(CH₂)₈-piperazinylene-(CH₂)₄-, #-(CH₂)₈-piperazinylene-(CH₂)₅-, #-(CH₂)₈- piperazinylene-(CH₂)₆-, #-(CH₂)₈-piperazinylene-(CH₂)₇-, or #-(CH₂)₈-piperazinylene-(CH₂)₈-;

wherein the piperidinylene and the piperazinylene are each independently optionally substituted with a substituent selected from the group consisting of C₁₋₆ alkyl (e.g., C₁₋₄ alkyl or C₁₋₃ alkyl, such as methyl, ethyl, propyl, isopropyl, butyl, sec-butyl or tert-butyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), halogen (e.g., fluorine, chlorine, bromine, or iodine), C₁₋₆ alkoxy (e.g., C₁₋₄ alkoxy or C₁₋₃ alkoxy, such as methoxy, ethoxy, propoxy, isopropoxy, butoxy, sec-butoxy or tert-butoxy), or any combination thereof;
each R_{d} independently represents H or C₁₋₆ alkyl (e.g., C₁₋₄ alkyl or C₁₋₃ alkyl, such as methyl, ethyl, propyl, isopropyl, butyl, sec-butyl or tert-butyl);
symbol # indicates the point of attachment to R₁₀; and
n4, n5, n6, n7, m1, m2, and m3 are each independently selected from the group consisting of an integer of 1, 2, 3, 4, 5, 6, 7, 8, 9, 10, 11, 12, 13, 14, or 15.

In some embodiments, the compound of Formula (I) is also of the structure of Formula (I-1): 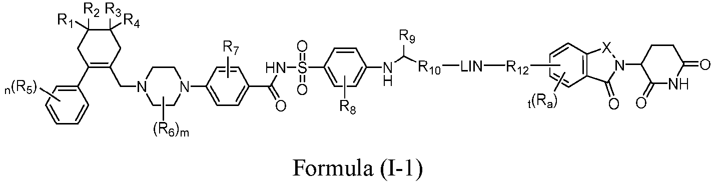 wherein R₁, R₂, R₃, R₄, (R₅)ₙ, (R₆)ₘ, R₇, R₈, R₉, R₁₀, LIN, R₁₂, (Rₐ)ₜ and X are as defined in the compound of Formula (I) above and each embodiments thereof.

In some embodiments, the compound of Formula (I) is also of the structure of Formula (I-2) or Formula (I-3): 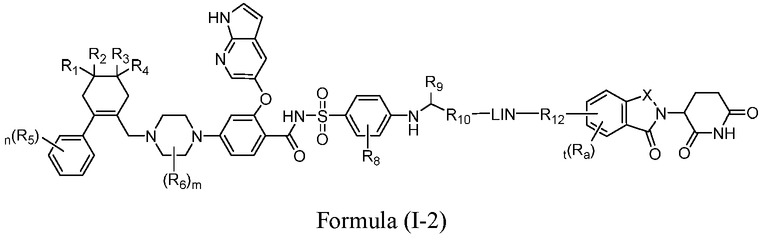 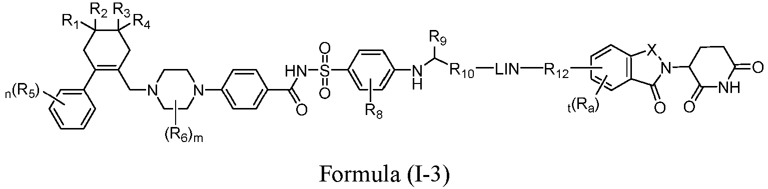 wherein R₁, R₂, R₃, R₄, (R₅)ₙ, (R₆)ₘ, R₈, R₉, R₁₀, LIN, R₁₂, (Rₐ)ₜ and X are as defined in the compound of Formula (I) above and each embodiments thereof.

In some embodiments, the compound of Formula (I) is also of the structure of Formula (I-2-1): 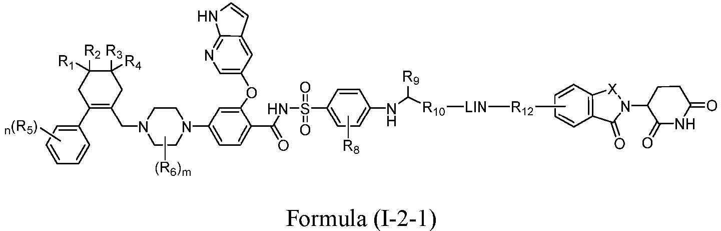 wherein R₁₀ represents the following group: 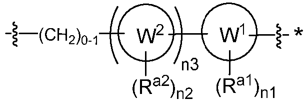
wherein ring W¹ represents 4- to 6-membered nitrogen-containing heterocyclylene; (R^{a1})ₙ₁ indicates that ring W¹ is substituted with n1R^{a1}, with each R^{a1} being the same or different and each independently representing C₁₋₆ alkyl (e.g., C₁₋₄ alkyl or C₁₋₃ alkyl, such as methyl, ethyl, propyl, isopropyl, butyl, sec-butyl or tert-butyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy or C₁₋₃ alkoxy, such as methoxy, ethoxy, propoxy, isopropoxy, butoxy, sec-butoxy or tert-butoxy), cyano, or halogen (e.g., fluorine, chlorine, bromine, or iodine), and n1 represents an integer of 1-8;
ring W² represents 4- to 6-membered nitrogen-containing heterocyclylene, or C₃₋₆ cycloalkylene; n3 represents an integer of 0 or 1; (R^{a2})ₙ₂ indicates that ring W² is substituted with n2 R^{a2}, with each R^{a2} being the same or different and each independently representing C₁₋₆ alkyl (e.g., C₁₋₄ alkyl or C₁₋₃ alkyl, such as methyl, ethyl, propyl, isopropyl, butyl, sec-butyl or tert-butyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy or C₁₋₃ alkoxy, such as methoxy, ethoxy, propoxy, isopropoxy, butoxy, sec-butoxy or tert-butoxy), cyano, halogen (e.g., fluorine, chlorine, bromine, or iodine), or oxo; n2 represents an integer of 0-8; and
symbol * indicates the point of attachment to LIN; and
R₁, R₂, R₃, R₄, (R₅)ₙ, (R₆)ₘ, Rs, R₉, R₁₀, LIN, R₁₂ and X are as defined in the compound of Formula (I) above and each embodiments thereof.

In some embodiments, ring W¹ of the compound of Formula (I-2-1) represents 4- to 6-membered nitrogen-containing heterocyclylene, and the 4- to 6-membered nitrogen-containing heterocyclylene includes but is not limited to e.g., piperidinylene, piperazinylene, morpholinylene, azetidinylene, pyrrolidinylene, imidazolidylene, pyrazolidylene, oxazolidinylene, thiazolidinylene, or thiomorpholinylene.

In some embodiments, (R^{a1})ₙ₁ of the compound of Formula (I-2-1) indicates that ring W¹ is substituted with n1 R^{a1}, with each R^{a1} being the same or different and each independently representing C₁₋₆ alkyl (e.g., C₁₋₄ alkyl or C₁₋₃ alkyl, such as methyl, ethyl, propyl, isopropyl, butyl, sec-butyl or tert-butyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy or C₁₋₃ alkoxy, such as methoxy, ethoxy, propoxy, isopropoxy, butoxy, sec-butoxy or tert-butoxy), cyano, halogen (e.g., fluorine, chlorine, bromine, or iodine), or oxo, n1 represents an integer of 1-8 (e.g., an integer of 1, 2, 3, or 4).

In some embodiments, ring W² of the compound of Formula (I-2-1) represents 4- to 6-membered nitrogen-containing heterocyclylene, and the 4- to 6-membered nitrogen-containing heterocyclylene includes but is not limited to e.g., piperidinylene, piperazinylene, morpholinylene, azetidinylene, pyrrolidinylene, imidazolidylene, pyrazolidylene, oxazolidinylene, thiazolidinylene, or thiomorpholinylene.

In some embodiments, ring W² of the compound of Formula (I-2-1) represents C₃₋₆ cycloalkylene, and the 4- to 6-membered cycloalkylene includes but is not limited to e.g., cyclopropyl, cyclobutyl, cyclopentyl, or cyclohexyl.

In some embodiments, (R^{a2})ₙ₂ of the compound of Formula (I-2-1) indicates that ring W² is substituted with n2 R^{a2}, with each R^{a2} being the same or different and each independently representing C₁₋₆ alkyl (e.g., C₁₋₄ alkyl or C₁₋₃ alkyl, such as methyl, ethyl, propyl, isopropyl, butyl, sec-butyl or tert-butyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy or C₁₋₃ alkoxy, such as methoxy, ethoxy, propoxy, isopropoxy, butoxy, sec-butoxy or tert-butoxy), cyano, halogen (e.g., fluorine, chlorine, bromine, or iodine), or oxo, n2 represents an integer of 0-8 (e.g., an integer of 0, 1, 2, 3, or 4).

In some embodiments, n3 of the compound of Formula (I-2-1) represents an integer of 0 or 1.

In some embodiments, n3 of the compound of Formula (I-2-1) represents an integer of 0.

In some embodiments, n3 of the compound of Formula (I-2-1) represents an integer of 1.

In some embodiments, R₁₀ of the compound of Formula (I-2-1) represents the following groups: 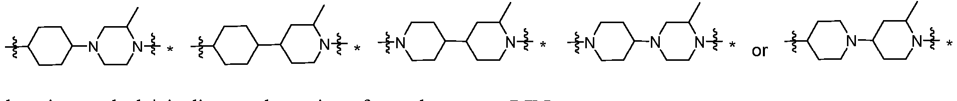 wherein symbol * indicates the point of attachment to LIN.

In some embodiments, the compound of Formula (I) is also of the structure of Formula (I-2-1): 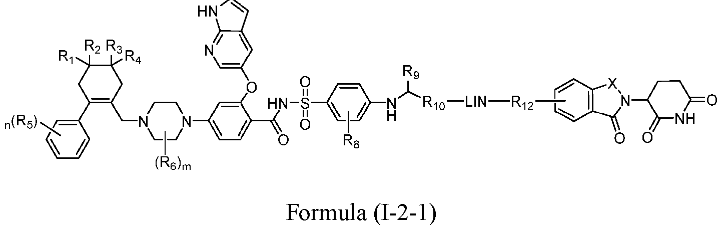 wherein R₈ represents -SO₂CF₃; and
R₁, R₂, R₃, R₄, (R₅)ₙ, (R₆)ₘ, R₉, R₁₀, LIN, R₁₂ and X are as defined in the compound of Formula (I) above and each embodiments thereof.

In some embodiments, the compound of Formula (I) is also of the structure of Formula (I-2-2), Formula (I-2-3), Formula (I-2-4), Formula (I-2-5), Formula (I-3-1), Formula (I-3-2), Formula (I-3-3), or Formula (I-3-4): 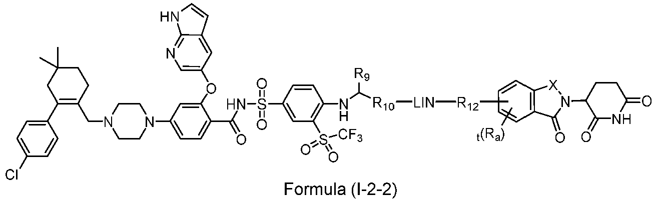 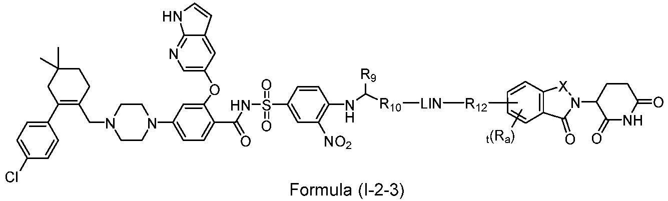 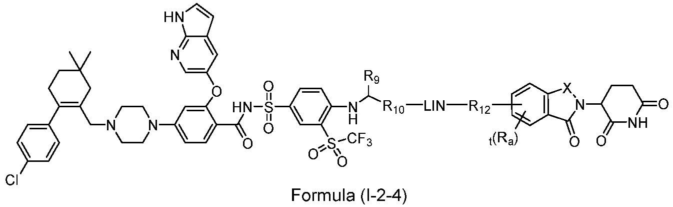 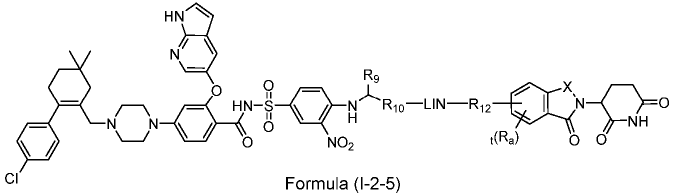 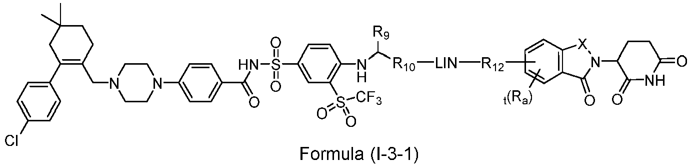 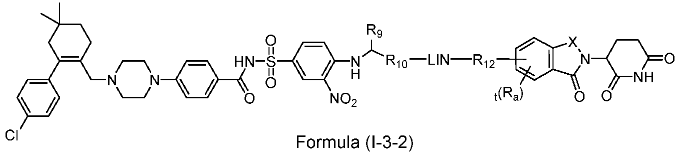 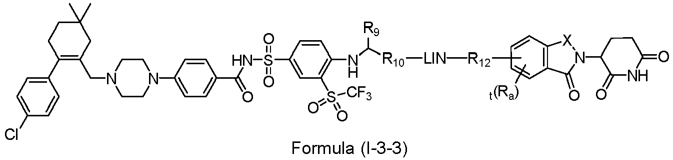 or 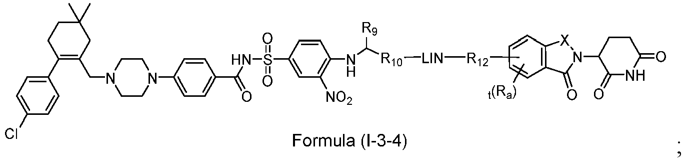 wherein R₉, R₁₀, LIN, R₁₂, (Rₐ)ₜ and X are as defined in the compound of Formula (I) above and each embodiments thereof.

In some embodiments, the compounds of Formula (I) and their salts (including pharmaceutically acceptable salts, such as hydrochloride, etc.), prodrugs, solvates, isotopically enriched analogs, polymorphs, stereoisomers (including enantiomers and diastereomers), or mixture of stereoisomers thereof in Table 1 below are provided.

**Table 1 Compounds of the present disclosure**

| Com poun dNo. | Structure of compound | Compound's name | Chinese translation of compound's name |
|---|---|---|---|
| BCL-0316 5 | 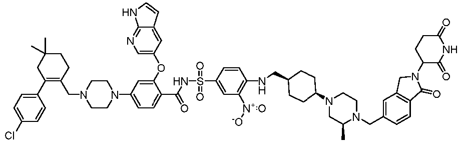 | 2-((1H-pyrrolo[2,3-b]pyridin-5 -yl)oxy)-4-(4-((4'-chloro-5,5-dimethyl-3,4,5,6-tetrahydro-[1,1'-biphenyl]-2-yl)methyl)piperazin-1-yl)-N-((4-((((cis)-4-((3S)-4-((2-(2,6-dioxopiperidin-3-yl)-1-oxoisoindolin-5-yl)methyl)-3-methylpiperazin-1-yl)cyclohexyl)methyl)a mino)-3-nitrophenyl)sulfonyl)be nzamide | 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((cis)-4-((3S)-4-((2-(2,6-dioxopiperidin-3-yl)-1-oxoisoindolin-5-yl)methyl)-3-methylpiperazin-1-yl)cyclohexyl)methyl) amino)-3-nitrophenyl)sulfonyl) benzamide |
| BCL-0316 6 | 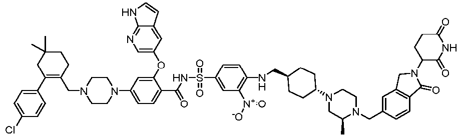 | 2-((1H-pyrrolo[2,3-b]pyridin-5 -yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-((3S)-4-((2-(2,6-dioxopiperidin-3-yl)-1-oxoisoindolin-5 - yl)methyl)-3-methylpiperazin-1-yl)cyclohexyl)methyl)a mino)-3-nitrophenyl)sulfonyl)be nzamide | 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-((3S)-4-((2-(2,6-dioxopiperidin-3 -yl)-1-oxoisoindolin-5- yl)methyl)-3-methylpiperazin-1-yl)cyclohexyl)methyl) amino)-3-nitrophenyl)sulfonyl) benzamide |
| BCL-0316 7 | 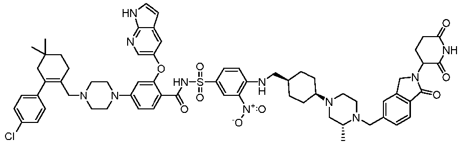 | 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((cis)-4-((3R)-4-((2-(2,6-dioxopiperidin-3-yl)-1-oxoisoindolin-5 - yl)methyl)-3-methylpiperazin-1-yl)cyclohexyl)methyl)a mino)-3-nitrophenyl)sulfonyl)be nzamide | 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((cis)-4-((3R)-4-((2-(2,6-dioxopiperidin-3-yl)-1-oxoisoindolin-5 - yl)methyl)-3-methylpiperazin-1-yl)cyclohexyl)methyl) amino)-3-nitrophenyl)sulfonyl) benzamide |
| BCL-0316 8 | 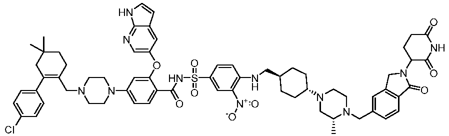 | 2-((1H-pyrrolo[2,3-b]pyridin-5 -yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-((3R)-4-((2-(2,6-dioxopiperidin-3-yl)-1-oxoisoindolin-5- yl)methyl)-3-methylpiperazin-1-yl)cyclohexyl)methyl)a mino)-3-nitrophenyl)sulfonyl)be nzamide | 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-((3R)-4-((2-(2,6-dioxopiperidin-3-yl)-1-oxoisoindolin-5-yl)methyl)-3-methylpiperazin-1-yl)cyclohexyl)methyl) amino)-3-nitrophenyl)sulfonyl) benzamide |
| BCL-0316 9 | 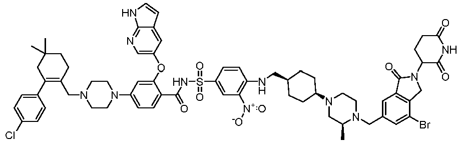 | 2-((1H-pyrrolo[2,3-b]pyridin-5-yl)oxy)-N-((4-((((cis)-4-((3S)-4-((7-bromo-2-(2,6-dioxopiperidin-3 -yl)-3- oxoisoindolin-5 - yl)methyl)-3-methylpiperazin-1-yl)cyclohexyl)methyl)a mino)-3-nitrophenyl)sulfonyl)-4-(4-((4'-chloro-5,5-dimethyl-3,4,5,6-tetrahydro-[1,1'-biphenyl]-2-yl)methyl)piperazin-1-yl)benzamide | 2-((1H-pyrrolo[2,3-b]pyridin-5-yl)oxy)-N-((4-((((cis)-4-((3S)-4-((7-bromo-2-(2,6-dioxopiperidin-3 -yl)-3-oxoisoindolin-5-yl)methyl)-3-methylpiperazin-1-yl)cyclohexyl)methyl) amino)-3-nitrophenyl)sulfonyl)-4-(4-((4'-chloro-5,5-dimethyl-3,4,5,6-tetrahydro-[1,1'-biphenyl]-2-yl)methyl)piperazin-1-yl)benzamide |
| BCL-0317 0 | 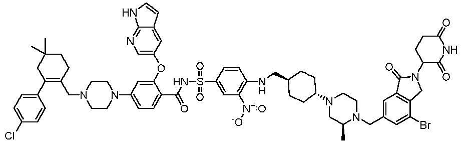 | 2-((1H-pyrrolo[2,3-b]pyridin-5 -yl)oxy)-N-((4-((((trans)-4-((3S)-4-((7-bromo-2-(2,6-dioxopiperidin-3 -yl)-3- oxoisoindolin-5-yl)methyl)-3-methylpiperazin-1-yl)cyclohexyl)methyl)a mino)-3-nitrophenyl)sulfonyl)-4-(4-((4'-chloro-5,5-dimethyl-3,4,5,6-tetrahydro-[1,1'-biphenyl]-2-yl)methyl)piperazin-1-yl)benzamide | 2-((1H-pyrrolo[2,3-b]pyridin-5-yl)oxy)-N-((4-((((trans)-4-((3 S)-4-((7-bromo-2-(2,6-dioxopiperidin-3 -yl)-3 -oxoisoindolin-5-yl)methyl)-3-methylpiperazin-1-yl)cyclohexyl)methyl) amino)-3-nitrophenyl)sulfonyl)-4-(4-((4'-chloro-5,5-dimethyl-3,4,5,6-tetrahydro-[1,1'-biphenyl]-2-yl)methyl)piperazin-1-yl)benzamide |
| BCL-0317 1 | 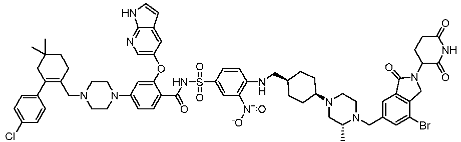 | 2-((1H-pyrrolo[2,3-b]pyridin-5 -yl)oxy)-N-((4-((((cis)-4-((3R)-4-((7-bromo-2-(2,6-dioxopiperidin-3 -yl)-3-oxoisoindolin-5-yl)methyl)-3-methylpiperazin-1-yl)cyclohexyl)methyl)a mino)-3-nitrophenyl)sulfonyl)-4-(4-((4'-chloro-5,5-dimethyl-3,4,5,6-tetrahydro-[1,1'-biphenyl]-2-yl)methyl)piperazin-1-yl)benzamide | 2-((1H-pyrrolo[2,3-b]pyridin-5-yl)oxy)-N-((4-((((cis)-4-((3R)-4-((7-bromo-2-(2,6-dioxopiperidin-3-yl)-3-oxoisoindolin-5-yl)methyl)-3-methylpiperazin-1-yl)cyclohexyl)methyl) amino)-3-nitrophenyl)sulfonyl)-4-(4-((4'-chloro-5,5-dimethyl-3,4,5,6-tetrahydro-[1,1'-biphenyl]-2-yl)methyl)piperazin-1-yl)benzamide |
| BCL-0317 2 | 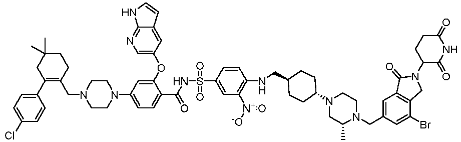 | 2-((1H-pyrrolo[2,3-b]pyridin-5 -yl)oxy)-N-((4-((((trans)-4-((3R)-4-((7-bromo-2-(2,6-dioxopiperidin-3 -yl)-3-oxoisoindolin-5-yl)methyl)-3-methylpiperazin-1-yl)cyclohexyl)methyl)a mino)-3-nitrophenyl)sulfonyl)-4-(4-((4'-chloro-5,5-dimethyl-3,4,5,6-tetrahydro-[1,1'-biphenyl]-2-yl)methyl)piperazin-1-yl)benzamide | 2-((1H-pyrrolo[2,3-b]pyridin-5-yl)oxy)-N-((4-((((trans)-4-((3R)-4-((7-bromo-2-(2,6-dioxopiperidin-3-yl)-3-oxoisoindolin-5-yl)methyl)-3-methylpiperazin-1-yl)cyclohexyl)methyl) amino)-3-nitrophenyl)sulfonyl)-4-(4-((4'-chloro-5,5-dimethyl-3,4,5,6-tetrahydro-[1,1'-biphenyl]-2-yl)methyl)piperazin-1-yl)benzamide |
| BCL-0319 0 | 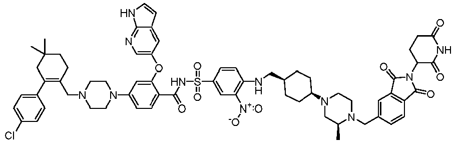 | 2-((1H-pyrrolo[2,3-b]pyridin-5 -yl)oxy)-4-(4-((4'-chloro-5,5-dimethyl-3,4,5,6-tetrahydro-[1,1'-biphenyl]-2-yl)methyl)piperazin-1-yl)-N-((4-((((cis)-4-((3S)-4-((2-(2,6-dioxopiperidin-3 -yl)-1,3-dioxoisoindolin-5-yl)methyl)-3-methylpiperazin-1-yl)cyclohexyl)methyl)a mino)-3-nitrophenyl)sulfonyl)be nzamide | 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((cis)-4-((3S)-4-((2-(2,6-dioxopiperidin-3 -yl)-1,3-dioxoisoindolin-5-yl)methyl)-3-methylpiperazin-1-yl)cyclohexyl)methyl) amino)-3-nitrophenyl)sulfonyl) benzamide |
| BCL-0314 6 | 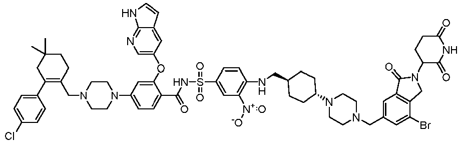 | 2-((1H-pyrrolo[2,3-b]pyridin-5 -yl)oxy)-N-((4-((((trans)-4-(4-((7-bromo-2-(2,6-dioxopiperidin-3 -yl)-3-oxoisoindolin-5-yl)methyl)piperazin-1-yl)cyclohexyl)methyl)a mino)-3-nitrophenyl)sulfonyl)-4-(4-((4'-chloro-5,5-dimethyl-3,4,5,6-tetrahydro-[1,1'-biphenyl]-2-yl)methyl)piperazin-1-yl)benzamide | 2-((1H-pyrrolo[2,3-b]pyridin-5-yl)oxy)-N-((4-((((trans)-4-(4-((7-bromo-2-(2,6-dioxopiperidin-3 -yl)-3-oxoisoindolin-5-yl)methyl)piperazin-1-yl)cyclohexyl)methyl) amino)-3-nitrophenyl)sulfonyl)-4-(4-((4'-chloro-5,5-dimethyl-3,4,5,6-tetrahydro-[1,1'-biphenyl]-2-yl)methyl)piperazin-1-yl)benzamide |
| BCL-0314 9 | 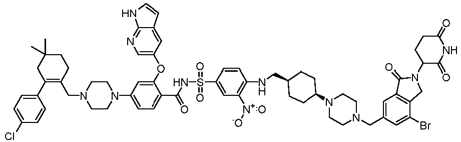 | 2-((1H-pyrrolo[2,3-b]pyridin-5 -yl)oxy)-N-((4-((((cis)-4-(4-((7-bromo-2-(2,6-dioxopiperidin-3-yl)-3-oxoisoindolin-5-yl)methyl)piperazin-1-yl)cyclohexyl)methyl)a mino)-3-nitrophenyl)sulfonyl)-4-(4-((4'-chloro-5,5-dimethyl-3,4,5,6-tetrahydro-[1,1'-biphenyl]-2-yl)methyl)piperazin-1-yl)benzamide | 2-((1H-pyrrolo[2,3-b]pyridin-5-yl)oxy)-N-((4-((((cis)-4-(4-((7-bromo-2-(2,6-dioxopiperidin-3 -yl)-3-oxoisoindolin-5-yl)methyl)piperazin-1-yl)cyclohexyl)methyl) amino)-3-nitrophenyl)sulfonyl)-4-(4-((4'-chloro-5,5-dimethyl-3,4,5,6-tetrahydro-[1,1'-biphenyl]-2-yl)methyl)piperazin-1-yl)benzamide |
| BCL-0317 9 | 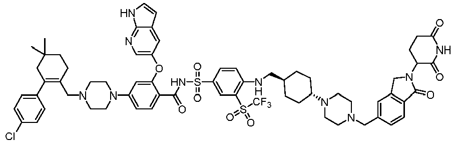 | 2-((1H-pyrrolo[2,3-b]pyridin-5 -yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-(4-((2-(2,6-dioxopiperidin-3-yl)-1-oxoisoindolin-5-yl)methyl)piperazin-1-yl)cyclohexyl)methyl)a mino)-3-((trifluoromethyl)sulfo nyl)phenyl)sulfonyl)be nzamide | 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-(4-((2-(2,6-dioxopiperidin-3-yl)-1-oxoisoindolin-5-yl)methyl)piperazin-1-yl)cyclohexyl)methyl) amino)-3-((trifluoromethyl)sulf onyl)phenyl)sulfonyl) benzamide |
| BCL-0318 5 | 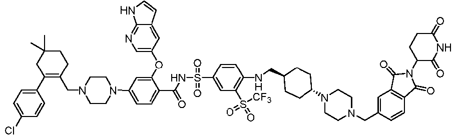 | 2-((1H-pyrrolo[2,3-b]pyridin-5 -yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-(4-((2-(2,6-dioxopiperidin-3 -yl)-1,3-dioxoisoindolin-5-yl)methyl)piperazin-1-yl)cyclohexyl)methyl)a mino)-3-((trifluoromethyl)sulfo nyl)phenyl)sulfonyl)be nzamide | 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-(4-((2-(2,6-dioxopiperidin-3 -yl)-1,3 -dioxoisoindolin-5-yl)methyl)piperazin-1-yl)cyclohexyl)methyl) amino)-3-((trifluoromethyl)sulf onyl)phenyl)sulfonyl) benzamide |
| BCL-0318 0 | 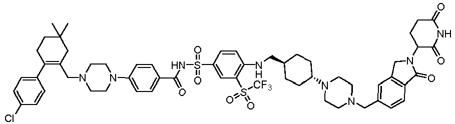 | 4-(4-((4'-chloro-4,4-dimethyl-3,4,5,6-tetrahydro-[1,1'-biphenyl]-2-yl)methyl)piperazin-1-yl)-N-((4-((((trans)-4-(4-((2-(2,6-dioxopiperidin-3-yl)-1-oxoisoindolin-5-yl)methyl)piperazin-1-yl)cyclohexyl)methyl)a mino)-3-((trifluoromethyl)sulfo nyl)phenyl)sulfonyl)be nzamide | 4-(4-((4'-chloro-4,4-dimethyl-3,4,5,6-tetrahydro-[1,1'-biphenyl]-2-yl)methyl)piperazin-1-yl)-N-((4-((((trans)-4-(4-((2-(2,6-dioxopiperidin-3 -yl)-1-oxoisoindolin-5-yl)methyl)piperazin-1-yl)cyclohexyl)methyl) amino)-3-((trifluoromethyl)sulf onyl)phenyl)sulfonyl) benzamide |
| BCL-0318 1 | 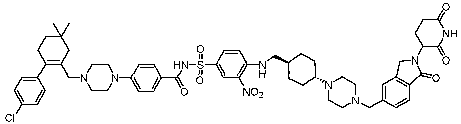 | 4-(4-((4'-chloro-4,4-dimethyl-3,4,5,6-tetrahydro-[1,1'-biphenyl]-2-yl)methyl)piperazin-1-yl)-N-((4-((((trans)-4-(4-((2-(2,6-dioxopiperidin-3-yl)-1-oxoisoindolin-5-yl)methyl)piperazin-1-yl)cyclohexyl)methyl)a mino)-3-nitrophenyl)sulfonyl)be nzamide | 4-(4-((4'-chloro-4,4-dimethyl-3,4,5,6-tetrahydro-[1,1'-biphenyl]-2-yl)methyl)piperazin-1-yl)-N-((4-((((trans)-4-(4-((2-(2,6-dioxopiperidin-3 -yl)-1-oxoisoindolin-5-yl)methyl)piperazin-1-yl)cyclohexyl)methyl) amino)-3-nitrophenyl)sulfonyl) benzamide |
| BCL-0318 6 | 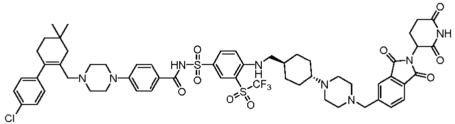 | 4-(4-((4'-chloro-4,4-dimethyl-3,4,5,6-tetrahydro-[1,1'-biphenyl]-2-yl)methyl)piperazin-1-yl)-N-((4-((((trans)-4-(4-((2-(2,6-dioxopiperidin-3-yl)-1,3-dioxoisoindolin-5-yl)methyl)piperazin-1-yl)cyclohexyl)methyl)a mino)-3-((trifluoromethyl)sulfo nyl)phenyl)sulfonyl)be nzamide | 4-(4-((4'-chloro-4,4-dimethyl-3,4,5,6-tetrahydro-[1,1'-biphenyl]-2-yl)methyl)piperazin-1-yl)-N-((4-((((trans)-4-(4-((2-(2,6-dioxopiperidin-3-yl)-1,3-dioxoisoindolin-5-yl)methyl)piperazin-1-yl)cyclohexyl)methyl) amino)-3-((trifluoromethyl)sulf onyl)phenyl)sulfonyl) benzamide |
| BCL-0318 7 | 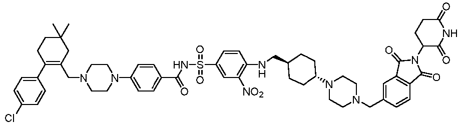 | 4-(4-((4'-chloro-4,4-dimethyl-3,4,5,6-tetrahydro-[1,1'-biphenyl]-2-yl)methyl)piperazin-1-yl)-N-((4-((((trans)-4-(4-((2-(2,6-dioxopiperidin-3-yl)-1,3-dioxoisoindolin-5-yl)methyl)piperazin-1-yl)cyclohexyl)methyl)a mino)-3-nitrophenyl)sulfonyl)be nzamide | 4-(4-((4'-chloro-4,4-dimethyl-3,4,5,6-tetrahydro-[1,1'-biphenyl]-2-yl)methyl)piperazin-1-yl)-N-((4-((((trans)-4-(4-((2-(2,6-dioxopiperidin-3-yl)-1,3-dioxoisoindolin-5-yl)methyl)piperazin-1-yl)cyclohexyl)methyl) amino)-3-nitrophenyl)sulfonyl) benzamide |
| BCL-0318 2 | 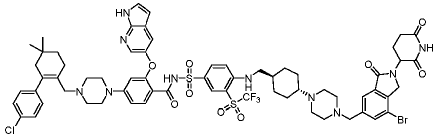 | 2-((1H-pyrrolo[2,3-b]pyridin-5-yl)oxy)-N-((4-((((trans)-4-(4-((7-bromo-2-(2,6-dioxopiperidin-3-yl)-3-oxoisoindolin-5-yl)methyl)piperazin-1-yl)cyclohexyl)methyl)a mino)-3-((trifluoromethyl)sulfo nyl)phenyl)sulfonyl)-4-(4-((4'-chloro-5,5-dimethyl-3,4,5,6-tetrahydro-[1,1'-biphenyl]-2-yl)methyl)piperazin-1-yl)benzamide | 2-((1H-pyrrolo[2,3-b]pyridin-5-yl)oxy)-N-((4-((((trans)-4-(4-((7-bromo-2-(2,6-dioxopiperidin-3-yl)-3-oxoisoindolin-5-yl)methyl)piperazin-1-yl)cyclohexyl)methyl) amino)-3-((trifluoromethyl)sulf onyl)phenyl)sulfonyl) -4-(4-((4'-chloro-5,5-dimethyl-3,4,5,6-tetrahydro-[1,1'-biphenyl]-2-yl)methyl)piperazin-1-yl)benzamide |
| BCL-0318 3 | 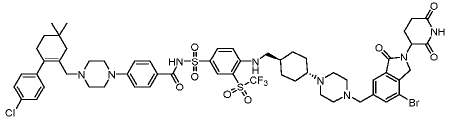 | ((trifluoromethyl)sulfo nyl)phenyl)sulfonyl)-4-(4-((4'-chloro-4,4-dimethyl-3,4,5,6-tetrahydro-[1,1'-biphenyl]-2-yl)methyl)piperazin-1-yl)benzamide | N-((4-((((trans)-4-(4-((7-bromo-2-(2,6-dioxopiperidin-3-yl)-3-oxoisoindolin-5-yl)methyl)piperazin-1-yl)cyclohexyl)methyl)amino)-3-((trifluoromethyl)sulf onyl)phenyl)sulfonyl) -4-(4-((4'-chloro-4,4-dimethyl-3,4,5,6-tetrahydro-[1,1'-biphenyl]-2-yl)methyl)piperazin-1-yl)benzamide |
| BCL-0318 4 | 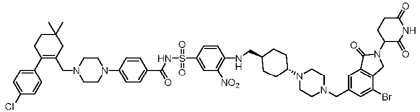 | N-((4-((((trans)-4-(4-((7-bromo-2-(2,6-dioxopiperidin-3-yl)-3-oxoisoindolin-5-yl)methyl)piperazin-1-yl)cyclohexyl)methyl)a mino)-3-nitrophenyl)sulfonyl)-4-(4-((4'-chloro-4,4-dimethyl-3,4,5,6-tetrahydro-[1,1'-biphenyl]-2-yl)methyl)piperazin-1-yl)benzamide | N-((4-((((trans)-4-(4-((7-bromo-2-(2,6-dioxopiperidin-3-yl)-3-oxoisoindolin-5-yl)methyl)piperazin-1-yl)cyclohexyl)methyl) amino)-3-nitrophenyl)sulfonyl)-4-(4-((4'-chloro-4,4-dimethyl-3,4,5,6-tetrahydro-[1,1'-biphenyl]-2-yl)methyl)piperazin-1-yl)benzamide |

In some embodiments, hydrochlorides, sulfates, citrates, maleates, sulfonates, methanesulfonates, citrates, lactates, tartrates, fumarates, phosphates, dihydrophosphates, pyrophosphates, metaphosphates, oxalates, malonates, benzoates, mandelates, succinates, trifluoroacetates, hydroxyacetates, or p-toluenesulfonates of the compound of Formula (I) of the present disclosure are provided.

In another aspect, the novel protein degraders developed based on BCL-2 family protein ligand compounds and their salts (including pharmaceutically acceptable salts, such as hydrochloride, etc.), stereoisomers (including enantiomers and diastereomers), solvates, isotopically enriched analogs, prodrugs, or polymorphs thereof in Table 2 below are provided.

**Table 2 Compounds of the present disclosure**

| Com poun d No. | Structure of compound | Compound's name | Chinese translation of compound's name |
|---|---|---|---|
| BCL-0317 7 | 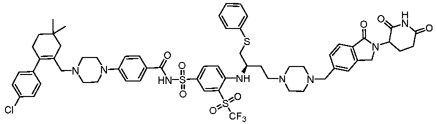 | 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-oxoisoindolin-5-yl)methyl)piperazin-1-yl)-1-(phenylthio)butan-2-yl)amino)-3-((trifluoromethyl)sulfonyl )phenyl)sulfonyl)benzami de | 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-oxoisoindolin-5-yl)methyl)piperazin-1-yl)-1-(phenylthio)butan-2-yl)amino)-3-((trifluoromethyl)sulf onyl)phenyl)sulfonyl)benzamide |
| BCL-0318 9 | 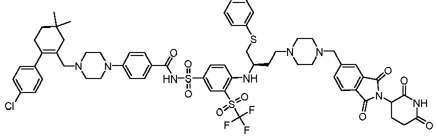 | 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1,3-dioxoisoindolin-5-yl)methyl)piperazin-1-yl)-1-(phenylthio)butan-2-yl)amino)-3-((trifluoromethyl)sulfonyl )phenyl)sulfonyl)benzami de | 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1,3-dioxoisoindolin-5-yl)methyl)piperazin-1-yl)-1-(phenylthio)butan-2-yl)amino)-3-((trifluoromethyl)sulf onyl)phenyl)sulfonyl) benzamide |
| BCL-0314 7 | 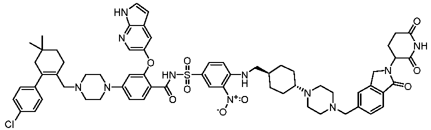 | 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-(4-((2-(2,6-dioxopiperidin-3-yl)-1-oxoisoindolin-5-yl)methyl)piperazin-1-yl)cyclohexyl)methyl)ami no)-3-nitrophenyl)sulfonyl)benz amide | 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-(4-((2-(2,6-dioxopiperidin-3-yl)-1-oxoisoindolin-5-yl)methyl)piperazin-1-yl)cyclohexyl)methyl) amino)-3-nitrophenyl)sulfonyl)b enzamide |
| BCL-0314 8 | 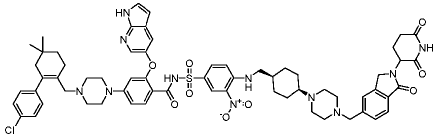 | 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((cis)-4-(4-((2-(2,6-dioxopiperidin-3-yl)-1-oxoisoindolin-5-yl)methyl)piperazin-1-yl)cyclohexyl)methyl)ami no)-3-nitrophenyl)sulfonyl)benz amide | 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((cis)-4-(4-((2-(2,6-dioxopiperidin-3-yl)-1-oxoisoindolin-5-yl)methyl)piperazin-1-yl)cyclohexyl)methyl) amino)-3-nitrophenyl)sulfonyl)b enzamide |
| BCL-0318 8 | 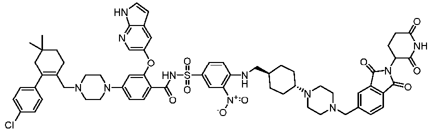 | 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-(4-((2-(2,6-dioxopiperidin-3-yl)-1,3-dioxoisoindolin-5-yl)methyl)piperazin-1-yl)cyclohexyl)methyl)ami no)-3-nitrophenyl)sulfonyl)benz amide | 1-yl)-N-((4-((((trans)-4-(4-((2-(2,6-dioxopiperidin-3-yl)-1,3-dioxoisoindolin-5-yl)methyl)piperazin-1-yl)cyclohexyl)methyl) amino)-3-nitrophenyl)sulfonyl)b enzamide |
| BCL-0317 8 | 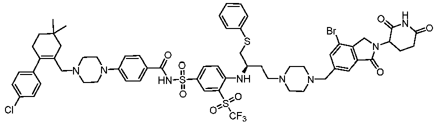 | N-((4-(((2R)-4-(4-((7-bromo-2-(2,6-dioxopiperidin-3-yl)-3-oxoisoindolin-5-yl)methyl)piperazin-1-yl)-1-(phenylthio)butan-2-yl)amino)-3-((trifluoromethyl)sulfonyl )phenyl)sulfonyl)-4-(4-((4'-chloro-4,4-dimethyl-3,4,5,6-tetrahydro-[1,1'-biphenyl]-2-yl)methyl)piperazin-1-yl)benzamide | N-((4-(((2R)-4-(4-((7-bromo-2-(2,6-dioxopiperidin-3-yl)-3-oxoisoindolin-5-yl)methyl)piperazin-1-yl)-1-(phenylthio)butan-2-yl)amino)-3-((trifluoromethyl)sulf onyl)phenyl)sulfonyl) -4-(4-((4'-chloro-4,4-dimethyl-3,4,5,6-tetrahydro-[1,1'-biphenyl]-2-yl)methyl)piperazin-1-yl)benzamide |

In some embodiments, hydrochlorides, sulfates, citrates, maleates, sulfonates, methanesulfonates, citrates, lactates, tartrates, fumarates, phosphates, dihydrophosphates, pyrophosphates, metaphosphates, oxalates, malonates, benzoates, mandelates, succinates, trifluoroacetates, hydroxyacetates, or p-toluenesulfonates of the compounds of the present disclosure in table 2 are provided.

### II. Other Forms of Compounds (including salts, enantiomers, stereoisomers, solvates, prodrugs, or polymorphs of compounds)

The compounds of the present disclosure have the structures of any one of Formula (I), Formula (I-1), Formula (I-2), Formula (I-3), Formula (I-2-1), Formula (I-2-2), Formula (I-2-3), Formula (I-2-4), Formula (I-2-5), Formula (I-3-1), Formula (I-3-2), Formula (I-3-3), or Formula (I-3-4), or any one of the structures in tables 1 or 2. Unless otherwise specified, all references to the compounds of the present disclosure also include compounds of any one of Formula (I), Formula (I-1), Formula (I-2), Formula (I-3), Formula (I-2-1), Formula (I-2-2), Formula (I-2-3), Formula (I-2-4), Formula (I-2-5), Formula (I-3-1), Formula (I-3-2), Formula (I-3-3), or Formula (I-3-4) and specific compounds within the scope of these general formulae, e.g., compounds in tables 1 or 2.

It should be recognized that compounds of the present disclosure (including compounds of any one of Formula (I), Formula (I-1), Formula (I-2), Formula (I-3), Formula (I-2-1), Formula (I-2-2), Formula (I-2-3), Formula (I-2-4), Formula (I-2-5), Formula (I-3-1), Formula (I-3-2), Formula (I-3-3), or Formula (I-3-4), or compounds in tables 1 or 2) may have a stereo-configuration and thus can exist in more than one stereoisomeric form. The present disclosure also relates to optically enriched compounds having a stereo-configuration, e.g., greater than about 90% enantiomeric/diastereomeric excess ("ee"), such as about 95% ee or 97% ee, or greater than 99% ee, and mixtures thereof, including racemic mixtures. As used herein, "optically enriched" means that a mixture of enantiomers consists of a significantly greater proportion of one enantiomer, and can be described by enantiomeric excess (ee%). Purification of isomers and separation of mixtures of isomers can be accomplished by standard techniques known in the art (e.g., column chromatography, preparative TLC, preparative HPLC, asymmetric synthesis (e.g., by using chiral intermediates) and/or or chiral resolution, etc.).

In some embodiments, polymorph forms or salts of the compounds of the present disclosure are also provided. Salts of the compounds of the present disclosure can be pharmaceutically acceptable salts including, but not limited to, hydrochlorides, sulfates, citrates, maleates, methanesulfonates, citrates, lactates, tartrates, fumarates, phosphates, dihydrophosphates, pyrophosphates, metaphosphates, oxalates, malonates, benzoates, mandelates, succinates, trifluoroacetates, hydroxyacetates, or p-toluenesulfonates, etc. The compounds of the present disclosure can exist as non-solvated or solvated forms in pharmaceutically acceptable solvents such as water, ethanol, and the like. In some embodiments, compounds of the present disclosure can be prepared as prodrugs or precursor drugs. Prodrugs can be converted into parent drugs in the body to play their role.

### III. Compositions/Formulations

In some embodiments, the present disclosure provides a pharmaceutical composition comprising as an active ingredient the compound of the present disclosure or a pharmaceutically acceptable salt, solvate, polymorph, prodrug, stereoisomer (including enantiomer), or mixture of stereoisomers thereof, and at least one pharmaceutically acceptable carrier.

In some embodiments, pharmaceutically acceptable carriers include, but are not limited to, fillers, stabilizers, dispersants, suspending agents, diluents, excipients, thickeners, colorants, solvents, or encapsulating materials. Each carrier must be "acceptable" in the sense of being compatible with the other ingredients of the formulation (including the compounds useful in the present disclosure) and not injurious to the patient. Some examples of materials that can be used as pharmaceutically acceptable carriers include: sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; polyethylene oxide, polyvinylpyrrolidone, polyacrylamide, poloxamer; and other common non-toxic compatible substances used in pharmaceutical formulations.

The pharmaceutical composition of the present disclosure can further comprise at least one second therapeutic agent, e.g., an anticancer agent. The second therapeutic agent may be used in combination with the compounds of the present disclosure (including compounds of any one of Formula (I), Formula (I-1), Formula (I-2), Formula (I-3), Formula (I-2-1), Formula (I-2-2), Formula (I-2-3), Formula (I-2-4), Formula (I-2-5), Formula (I-3-1), Formula (I-3-2), Formula (I-3-3), or Formula (I-3-4), or compounds in tables 1 or 2) to treat the diseases or disorders as disclosed herein. The second therapeutic agent includes, but is not limited to, chemotherapeutic agents, immunotherapeutic agents, gene therapy agents, and the like.

The pharmaceutical composition of the present disclosure comprising, as an active ingredient, the compounds of the present disclosure (including compounds of any one of Formula (I), Formula (I-1), Formula (I-2), Formula (I-3), Formula (I-2-1), Formula (I-2-2), Formula (I-2-3), Formula (I-2-4), Formula (I-2-5), Formula (I-3-1), Formula (I-3-2), Formula (I-3-3), or Formula (I-3-4), or compounds in tables 1 or 2) or a pharmaceutically acceptable salt thereof can be formulated into any suitable formulations such as sprays, patches, tablets (such as conventional tablets, dispersible tablets, orally disintegrating tablets), capsules (such as soft capsules, hard capsules, enteric-coated capsules), dragees, troches, powders, granules, powder injections, suppositories, or liquid formulations (such as suspensions (e.g., aqueous or oily suspensions), solutions, emulsions, or syrups), or conventional injection dosage forms such as injectable solutions (e.g., sterile injectable solutions formulated according to methods known in the art using water, Ringer's solution, or isotonic sodium chloride solution or the like as a vehicle or solvent) or lyophilized injectable formulation and the like, depending upon a suitable route of administration (including, but not limited to, nasal administration, inhalation administration, topical administration, oral administration, oral mucosal administration, rectal administration, intrapleural administration, intraperitoneal administration, vaginal administration, intramuscular administration, subcutaneous administration, transdermal administration, epidural administration, intrathecal administration, and intravenous administration). Those skilled in the art can also formulate the compounds of the present disclosure (including compounds of any one of Formula (I), Formula (I-1), Formula (I-2), Formula (I-3), Formula (I-2-1), Formula (I-2-2), Formula (I-2-3), Formula (I-2-4), Formula (I-2-5), Formula (I-3-1), Formula (I-3-2), Formula (I-3-3), or Formula (I-3-4), or compounds in tables 1 or 2) into conventional, dispersible, chewable, orally disintegrating or rapidly dissolving formulations, or sustained-release capsules or controlled-release capsules as needed.

The compounds of the present disclosure (including compounds of any one of Formula (I), Formula (I-1), Formula (I-2), Formula (I-3), Formula (I-2-1), Formula (I-2-2), Formula (I-2-3), Formula (I-2-4), Formula (I-2-5), Formula (I-3-1), Formula (I-3-2), Formula (I-3-3), or Formula (I-3-4), or compounds in tables 1 or 2), as an active ingredient, is contained in the pharmaceutically acceptable carrier or diluent in an amount sufficient to deliver to a subject a therapeutically effective amount for the indication to be treated, without causing serious toxic effects in the subject treated. A dosage of the active compound for all diseases or disorders mentioned herein ranges, for example, from about 5ng/kg to 500mg/kg, from about 10ng/kg to 300mg/kg per day, such as from 0.1 to 100 mg/kg, or from 0.5 to about 25mg per kilogram body weight of the subject per day.

The compounds of the present disclosure (including compounds of any one of Formula (I), Formula (I-1), Formula (I-2), Formula (I-3), Formula (I-2-1), Formula (I-2-2), Formula (I-2-3), Formula (I-2-4), Formula (I-2-5), Formula (I-3-1), Formula (I-3-2), Formula (I-3-3), or Formula (I-3-4), or compounds in tables 1 or 2) or pharmaceutically acceptable salts thereof can be conveniently administered in any suitable unit dosage form. Suitable unit dosage form specifications include, but are not limited to, less than 1mg, 1mg to 3000mg, Smg to 1000mg, for example, 5 to 500mg, 25 to 250mg of active ingredient per unit dosage form.

### IV. Kits/Packaged Products

The compounds of the present disclosure (including compounds of any one of Formula (I), Formula (I-1), Formula (I-2), Formula (I-3), Formula (I-2-1), Formula (I-2-2), Formula (I-2-3), Formula (I-2-4), Formula (I-2-5), Formula (I-3-1), Formula (I-3-2), Formula (I-3-3), or Formula (I-3-4), or compounds in tables 1 or 2) or a pharmaceutically acceptable salt, solvate, polymorph, prodrug, stereoisomer (including enantiomer), or mixture of stereoisomers thereof is used as a medicament. The medicament of the present disclosure or the pharmaceutical composition of the present disclosure may be presented in a kit/packaged product. The kit/packaged product may include a package or container including, but not limited to, ampoules, blister packs, pharmaceutical plastic bottles, vials, pharmaceutical glass bottles, containers, syringes, laminated flexible packaging, co-extruded film infusion containers, test tubes and dispensing devices, and the like. The kit/packaged product may contain instructions for use of the product.

### V. Methods and Uses

The compounds of the present disclosure (including compounds of any one of Formula (I), Formula (I-1), Formula (I-2), Formula (I-3), Formula (I-2-1), Formula (I-2-2), Formula (I-2-3), Formula (I-2-4), Formula (I-2-5), Formula (I-3-1), Formula (I-3-2), Formula (I-3-3), or Formula (I-3-4), or compounds in tables 1 or 2) or a pharmaceutically acceptable salt, solvate, isotopically enriched analog, polymorph, prodrug, stereoisomer (including enantiomer), or mixture of stereoisomers thereof can be used as a medicament. Especially the compounds of the present disclosure (including compounds of any one of Formula (I), Formula (I-1), Formula (I-2), Formula (I-3), Formula (I-2-1), Formula (I-2-2), Formula (I-2-3), Formula (I-2-4), Formula (I-2-5), Formula (I-3-1), Formula (I-3-2), Formula (I-3-3), or Formula (I-3-4), or compounds in tables 1 or 2) or a pharmaceutically acceptable salt, solvate, polymorph, prodrug, stereoisomer (including enantiomer), or mixture of stereoisomers thereof can be used for the manufacture of a medicament for the prevention and/or treatment of of a disease or disorder selected from the group consisting of neurodegenerative diseases; angiocardiopathy; autoimmune disease; myelofibrosis; renal fibrosis; hepatic fibrosis; cirrhosis; tumors; multiple-organ dysfunction syndrome (MODS); multiple-organ failure caused by cachexia and tangnsepsis shock; organ (including kidney, heart, lung) or tissue transplant rejection; diabetes mellitus; transplant rejection; retinopathy; and acute liver failure. In some embodiments, the disease or disorder includes, but is not limited to, neurodegenerative diseases, including Parkinson's disease and Alzheimer's disease; angiocardiopathy, including e.g., coronary heart disease, congestive heart failure, myocardial infarction, and atherosclerosis; autoimmune disease, including rheumatoid arthritis, autoimmune encephalomyelitis, ankylosing spondylitis, psoriasis, systemic lupus erythematosus, multiple sclerosis, recurrent oral ulcer, Kawasaki disease, polymyositis/dermatomyositis, Sjogren's syndrome, and atopic dermatitis; myelofibrosis; renal fibrosis; hepatic fibrosis; cirrhosis; tumors, including hematological malignancies, and solid tumors; multiple-organ dysfunction syndrome (MODS), including multiple-organ failure caused by cachexia and tangnsepsis shock; acute liver failure; graft-rejection, including organ (including kidney, heart, lung) or tissue transplant rejection; retinopathy, including diabetic macular edema (DME) and wet age-related macular degeneration (wAMD); and diabetes mellitus. In some embodiments, the tumors include, but are not limited to, e.g., myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplant-related cancer; neutropenia; leukemia, including acute myeloid leukemia, chronic myelogenous leukemia, chronic lymphocytic leukemia, B-cell chronic lymphocytic leukemia, leukemia-related anemia, acute myeloid leukemia (AMI,); lymphomas, including diffuse large B-cell lymphoma, non-Hodgkin lymphoma, anaplastic lymphoma, anaplastic large cell lymphoma, CD20-positive lymphoma, mantle cell lymphoma, primary lymphoma, T-cell lymphoma (including relapsed or refractory peripheral T-cell lymphoma), small lymphocytic lymphoma, B-cell lymphoma, recurrent B-cell non-Hodgkin lymphoma, recurrent diffuse large B-cell lymphoma, recurrent mediastinal (thymic) large B-cell lymphoma, primary mediastinal (thymus) large B-cell lymphoma, recurrent transformed non-Hodgkin lymphoma, refractory B cellular non-Hodgkin's lymphoma, refractory diffuse large B cell lymphoma, refractory primary mediastinal (thymic) large B cell lymphoma, refractory transformed non-Hodgkin's lymphoma, lymphoplasmacytic lymphoma, Waldenstroem's macroglobulinemia; thyroid cancer; melanoma; lung cancer, including non-small cell lung cancer, small cell lung cancer, lung adenocarcinoma, and lung squamous cell carcinoma; inflammatory myofibroblastic tumor; colorectal cancer; glioma; astroblastoma; ovarian cancer; bronchial cancer; prostate cancer; breast cancer, including estrogen-dependent breast cancer, HER2-positive breast cancer, triple-negative breast cancer, incidental breast cancer, and Coden's disease; pancreatic cancer; neuroblastoma; extramedullary plasmacytoma; medulloblastoma; plaemocytoma; gastric cancer; gastrointestinal stromal tumor; esophagus cancer; colorectal cancer; esophageal squamous cell cancer; liver cancer; renal cell cancer; bladder cancer; endometrial cancer; brain cancer; oral cancer; sarcomas, including rhabdomyosarcoma, various adipose-derived tumors, ewing's sarcoma/primitive neuroectodermal tumors (Ewing/PNETs), and leiomyosarcoma; chondrosarcoma; urothelial carcinoma; basal cell carcinoma; oral squamous cell cancer; cholangiocarcinoma; bone cancer; cervical cancer; and skin cancer.

A method for preventing and/or treating a disease or disorder in a subject, comprising administering to the subject a therapeutically effective amount of the compounds of the present disclosure (including compounds of any one of Formula (I), Formula (I-1), Formula (I-2), Formula (I-3), Formula (I-2-1), Formula (I-2-2), Formula (I-2-3), Formula (I-2-4), Formula (I-2-5), Formula (I-3-1), Formula (I-3-2), Formula (I-3-3), or Formula (I-3-4), or compounds in tables 1 or 2), or a pharmaceutically acceptable salt thereof, or the pharmaceutical composition of the present disclosure. In some embodiments, the disease or disorder includes: neurodegenerative diseases; angiocardiopathy; autoimmune disease; myelofibrosis; renal fibrosis; hepatic fibrosis; cirrhosis; tumors; multiple-organ dysfunction syndrome (MODS); multiple-organ failure caused by cachexia and tangnsepsis shock; organ (including kidney, heart, lung) or tissue transplant rejection; diabetes mellitus; transplant rejection; retinopathy; and acute liver failure. In some embodiments, the disease or disorder includes, but is not limited to, neurodegenerative diseases, including Parkinson's disease and Alzheimer's disease; angiocardiopathy, including e.g., coronary heart disease, congestive heart failure, myocardial infarction, and atherosclerosis; autoimmune disease, including rheumatoid arthritis, autoimmune encephalomyelitis, ankylosing spondylitis, psoriasis, systemic lupus erythematosus, multiple sclerosis, recurrent oral ulcer, Kawasaki disease, polymyositis/dermatomyositis, Sjogren's syndrome, and atopic dermatitis; myelofibrosis; renal fibrosis; hepatic fibrosis; cirrhosis; tumors, including hematological malignancies, and solid tumors; multiple-organ dysfunction syndrome (MODS), including multiple-organ failure caused by cachexia and tangnsepsis shock; acute liver failure; graft-rejection, including organ (including kidney, heart, lung) or tissue transplant rejection; retinopathy, including diabetic macular edema (DME) and wet age-related macular degeneration (wAMD); and diabetes mellitus. In some embodiments, the tumors include, but are not limited to, e.g., myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplant-related cancer; neutropenia; leukemia, including acute myeloid leukemia, chronic myelogenous leukemia, chronic lymphocytic leukemia, B-cell chronic lymphocytic leukemia, leukemia-related anemia, acute myeloid leukemia (AML); lymphomas, including diffuse large B-cell lymphoma, non-Hodgkin lymphoma, anaplastic lymphoma, anaplastic large cell lymphoma, CD20-positive lymphoma, mantle cell lymphoma, primary lymphoma, T-cell lymphoma (including relapsed or refractory peripheral T-cell lymphoma), small lymphocytic lymphoma, B-cell lymphoma, recurrent B-cell non-Hodgkin lymphoma, recurrent diffuse large B-cell lymphoma, recurrent mediastinal (thymic) large B-cell lymphoma, primary mediastinal (thymus) large B-cell lymphoma, recurrent transformed non-Hodgkin lymphoma, refractory B cellular non-Hodgkin's lymphoma, refractory diffuse large B cell lymphoma, refractory primary mediastinal (thymic) large B cell lymphoma, refractory transformed non-Hodgkin's lymphoma, lymphoplasmacytic lymphoma, Waldenstroem's macroglobulinemia; thyroid cancer; melanoma; lung cancer, including non-small cell lung cancer, small cell lung cancer, lung adenocarcinoma, and lung squamous cell carcinoma; inflammatory myofibroblastic tumor; colorectal cancer; glioma; astroblastoma; ovarian cancer; bronchial cancer; prostate cancer; breast cancer, including estrogen-dependent breast cancer, HER2-positive breast cancer, triple-negative breast cancer, incidental breast cancer, and Coden's disease; pancreatic cancer; neuroblastoma; extramedullary plasmacytoma; medulloblastoma; plaemocytoma; gastric cancer; gastrointestinal stromal tumor; esophagus cancer; colorectal cancer; esophageal squamous cell cancer; liver cancer; renal cell cancer; bladder cancer; endometrial cancer; brain cancer; oral cancer; sarcomas, including rhabdomyosarcoma, various adipose-derived tumors, ewing's sarcoma/primitive neuroectodermal tumors (Ewing/PNETs), and leiomyosarcoma; chondrosarcoma; urothelial carcinoma; basal cell carcinoma; oral squamous cell cancer; cholangiocarcinoma; bone cancer; cervical cancer; and skin cancer.

In the method for preventing and/or treating the disease or disorder in a subject, the compound of the present disclosure (including compounds of any one of Formula (I), Formula (I-1), Formula (I-2), Formula (I-3), Formula (I-2-1), Formula (I-2-2), Formula (I-2-3), Formula (I-2-4), Formula (I-2-5), Formula (I-3-1), Formula (I-3-2), Formula (I-3-3), or Formula (I-3-4), or compounds in tables 1 or 2) or the pharmaceutical composition of the present disclosure is administered to the subject through at least one mode of administration selected from the group consisting of nasal administration, inhalation administration, topical administration, oral administration, oral mucosal administration, rectal administration, pleural cavity administration, peritoneal administration, vaginal administration, intramuscular administration, subcutaneous administration, transdermal administration, epidural administration, intrathecal administration, and intravenous administration.

The term "treatment" or "treating" refers to the administration of the compound of the present disclosure (including compounds of any one of Formula (I), Formula (I-1), Formula (I-2), Formula (I-3), Formula (I-2-1), Formula (I-2-2), Formula (I-2-3), Formula (I-2-4), Formula (I-2-5), Formula (I-3-1), Formula (I-3-2), Formula (I-3-3), or Formula (I-3-4), or compounds in tables 1 or 2) or a pharmaceutically acceptable salt thereof according to the present disclosure, or the pharmaceutical composition containing, as an active ingredient, the compound of the present disclosure or a pharmaceutically acceptable salt thereof, to a subject to mitigate (alleviate) undesirable diseases or conditions, such as the development of a cancer or tumor. The beneficial or desired clinical results of the present disclosure include, but are not limited to: alleviating symptoms, reducing the severity of the disease, stabilizing the state of the disease, slowing down or delaying the progression of the disease, improving or alleviating the condition, and alleviating the disease.

A "therapeutic effective amount" of the compound of the present disclosure depends on a variety of factors, including the activity of the specific compound used, the metabolic stability of the compound and the duration of its action, the age, sex and weight of the patient, the patient's current medical condition, the route and duration of administration, the excretion rate, the combined administration of additional drugs, and the progression of the diseases or conditions of the patient being treated. Those skilled in the art will be able to determine appropriate dosages based on these and other factors.

It is to be understood that the choice of using one or more active compounds and/or compositions and their dosage depends on the basic situations of the individual (which should generally render the individual situation to achieve the best effect). Dosing and dosing regimens should be within the ability of those skilled in the art, and the appropriate dosage depends on many factors including the knowledge and ability of the physicians, veterinarians or researchers (see e.g., Jun Li (chief editor), "Clinical Pharmacology", 4th edition, People's Public Health Press, 2008).

As used herein, the term "patient" or "subject" to be treated refers to animal, for example mammal, including but not limited to primate (such as human being), cow, sheep, goat, horse, dog, cat, rabbit, guinea pig, rat, mice, etc.

### VI. Definitions

Unless otherwise specified, the following words, phrases and symbols used herein generally have the meanings as described below.

In general, the nomenclature used herein (including the IUPAC nomenclature) and the laboratory procedures described below (including those used in cell culture, organic chemistry, analytical chemistry, and pharmacology, etc.) are those well-known and commonly used in the art. Unless otherwise defined, all scientific and technical terms used herein in connection with the present disclosure described herein have the same meaning as commonly understood by one skill in the art. In addition, the use of the word "a" or "an" when used in conjunction with the term "comprising" or a noun in the claims and/or the specification may mean "one", but it is also consistent with the meaning of "one or more", "at least one", and "one or more than one". Similarly, the terms "another" or "other" can mean at least a second or more.

It should be understood that whenever the term "comprise" or "include" is used herein to describe various aspects, other similar aspects described by "consisting of" and/or "consisting essentially of" are also provided.

As used herein, the term "about" used alone or in combination refers to approximately, roughly, nearly, or around. When the term "about" is used in conjunction with a numerical range, it modifies that range by extending the boundaries above and below the stated numerical value. In general, the term "about" can modify a numerical value above and below the stated value by an upward or downward (increasing or decreasing) variation, e.g., 10%, 5%, 2%, or 1%.

As used herein, the wording "...represents a bond" used alone or in combination means that the referenced group is a bond linker (that is, the referenced group is absent). For example, the wording "R₁₂ represents a bond" means that R₁₂ is a bond linker. In other words, when R₁₂ represents a bond, the group LIN in the structure of Formula (I) is directly connected to the benzene ring in the structure of Formula (II).

As used herein, the term "inserted" of the expression "one or more groups R_{b} and/or one or more groups R_{c} and/or any combination of one or more groups R_{b} and R_{c} are inserted into the backbone carbon chain of the linear or branched C₂₋₃₀ alkylene group", used alone or in combination, has a known definition in the art, which can mean that carbon-carbon bond between one or more pairs of adjacent carbon atoms in the referenced backbone carbon chain is interrupted by the groups R_{b}, R_{c}, or a combination of R_{b} and R_{c}. Herein, examples of the above-mentioned expression "one or more groups...... are inserted into" may include, but are not limited to, that one or more (1-30, 1-20, or 1-15, 1-10, 1-9, 1-8, 1-7, 1-6, 1-5, 1-4, 1-3, or 1-2, or 1) groups R_{b} as defined herein and/or one or more (1-30, 1-20, or 1-15, 1-10, 1-9, 1-8, 1-7, 1-6, 1-5, 1-4, 1-3, or 1-2, or 1) groups R_{c} as defined herein and/or one or more (1-30, 1-20, or 1-15, 1-10, 1-9, 1-8, 1-7, 1-6, 1-5, 1-4, 1-3, or 1-2 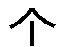 , or 1) combinations of R_{b} with R_{c} are inserted into the backbone carbon chain, and the resulting backbone chain group conforms to the covalent bond theory. For example, the expression "one or more groups R_{b} and/or one or more groups R_{c} and/or any combination of one or more groups R_{b} and R_{c} are inserted into the backbone carbon chain of the linear or branched C₂₋₃₀ alkylene group" can refer to that one or more (e.g., 1-30, 1-20, 1-15, 1-10, 1-8, 1-7, 1-6, 1-5, 1 -4, 1-3, 1-2, or 1) groups R_{b} and/or R_{c} and/or one or more combinations of R_{b} with R_{c} are inserted between one or more pairs of any two adjacent carbon atoms of the backbone carbon chain of the linear or branched C₂₋₃₀ alkylene group, resulting in the formation of a backbone chain group containing one or more (e.g., 1-30, 1-20, 1-15, 1-10, 1-8, 1-7, 1-6, 1-5, 1 -4, 1-3, 1-2, or 1) fragments "-CH₂-R_{b}-CH₂-" and/or one or more (e.g., 1-30, 1-20, 1-15, 1-10, 1-8, 1-7, 1-6, 1-5, 1-4, 1-3, 1-2, or 1) fragments "-CH₂-R_{c}-CH₂-" and/or one or more (e.g., 1-30, 1-20, 1-15, 1-10, 1-8, 1-7, 1-6, 1-5, 1-4, 1-3, 1-2, or 1) fragments "-CH₂-R_{b}-R_{c}-CH₂-", where each R_{b} are the same or different, each R_{c} are the same or different, and are as defined herein.

As used herein, the term "optionally substituted" used alone or in combination means that the referenced group may be unsubstituted or substituted with one or more substituents as defined here. Herein, the wording "optionally substituted by..." and "unsubstituted or substituted" can be used interchangeably. The term "substituted" generally indicates that one or more hydrogens in the referenced structure are replaced by the same or different specific substituents. The number of substituents is not theoretically limited in any way, or is automatically limited by the size of the building units (i.e., the total number of replaceable hydrogen atoms in the building units), or as clearly defined herein.

Herein, a bond interrupted by a wavy line shows the point of attachment of the depicted group to the rest of the molecule. For example, the group of Formula (II) depicted below 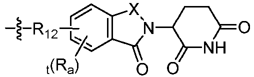 shows the point of attachment of R₁₂ in said group to LIN of the compound of Formula (I).

As used herein, the expression "a hydrogen atom of one or more CH₂ of the linear or branched C_{x-y} alkylene is replaced by...", used alone or in combination, means that a hydrogen atom of any one or more CH₂ of the linear or branched C_{x-y} alkylene is replaced by a substituent(s) as defined herein. Herein, the term "one or more" of "a hydrogen atom of one or more CH₂ of groups #-CH₂-, #-(CH₂)₂-, #-(CH₂)₃-, #-(CH₂)₄-, #-(CH₂)₅-, #-(CH₂)₆-, #-(CH₂)₇-, #-(CH₂)₈-, #-(CH₂)₉-, #-(CH₂)₁₀-, #-(CH₂)₁₁-, #-(CH₂)₁₂-, #-(CH₂)₁₃-, #-(CH₂)₁₄-, #-(CH₂)₁₅-, #-(CH₂)₁₆-, #-(CH₂)₁₇-, #-(CH₂)₁₈-, #-(CH₂)₁₉-, #-(CH₂)₂₀-, #-(CH₂)₂₁-, #-(CH₂)₂₂-, #-(CH₂)₂₅-, or #-(CH₂)₃₀-" may refer to part or all hydrogen atoms of each referenced alkylene group, including but not limited to 1-60 hydrogens. In some embodiments, the expression "a hydrogen atom of one or more CH₂" may refer to part or all of the hydrogen atoms of the referenced alkylene group, including but not limited to 1-30, such as 1-25, 1-20, 1-15, 1-10, 1-5, 1-4, 1-3, 1-2 or 1 hydrogen atoms. In some embodiments, the expression "a hydrogen atom of one or more CH₂" may include 1-3 of the plurality of hydrogen atoms of the referenced alkylene group. The number of hydrogens to be replaced is in principle not limited in any way, or is automatically limited by the size of the building unit.

As used herein, the term "oxo" or "oxo group" refers to =O.

As used herein, the term "halogen atom" or "halogen", used alone or in combination, refers to fluorine, chlorine, bromine, or iodine.

As used herein, the term "alkyl", used alone or in combination, refers to a linear or branched alkyl group. The term "Cₓ-C_{y} alkyl" or "C_{x-y} alkyl" (x and y each being an integer) refers to a linear or branched alkyl group containing from x to y carbon atoms. The term "C₁₋₁₀ alkyl" used alone or in combination in the present disclosure refers to a linear or branched alkyl group containing from 1 to 10 carbon atoms. Examples of the C₁₋₁₀ alkyl of the present disclosure may include a C₁₋₉ alkyl, C₁₋₈ alkyl, C₂₋₈ alkyl, C₁₋₇ alkyl, C₁₋₆ alkyl, C₁₋₅ alkyl, and C₁₋₄ alkyl. Representative examples include methyl, ethyl, n-propyl, isopropyl, n-butyl, isobutyl, sec-butyl, tert-butyl, pentyl, isopentyl, neopentyl, tert-pentyl, hexyl, heptyl, octyl, nonyl, and decyl. The term "C₁₋₃ alkyl" or "C₁-C₃ alkyl" in the present disclosure refers to an alkyl group containing from 1 to 3 carbon atoms, and representative examples thereof include methyl, ethyl, n-propyl, and isopropyl. In the present disclosure, the "alkyl" is optionally substituted with one or more substituents optionally selected from the group consisting of halogen, hydroxyl, cyano, C₁₋₃ alkyl, C₁₋₃ alkoxy, trifluoromethyl, heterocyclyl, or a combination thereof.

As used herein, the term "halogenated alkyl" or "haloalkyl", used alone or in combination, refers to a linear or branched alkyl group substituted with one or more halogens, wherein one or more hydrogen atom(s) of the alkyl group are replaced with one or more halogens. The term "halogenated C_{x-Cy} alkyl" or "halogenated C_{x-y} alkyl" (x and y are each an integer) refers to a linear or branched alkyl containing from x to y carbon atoms substituted with one or more halogens. The term "halogenated C₁₋₁₀ alkyl" used alone or in combination in the present invention refers to a linear or branched alkyl group containing from 1 to 10 carbon atoms substituted with one or more halogens (e.g., fluorine, chlorine, bromine, or iodine). Examples of the halogenated C₁₋₁₀ alkyl group of the present disclosure include halogenated C₁₋₉ alkyl group, e.g., halogenated C₁₋₈ alkyl group, halogenated C₂₋₈ alkyl group, halogenated C₁₋₇ alkyl group, halogenated C₁₋₆ alkyl, halogenated C₁₋₅ alkyl, or halogenated C₁₋₄ alkyl. Representative examples include halomethyl, haloethyl, halo-n-propyl, haloisopropyl, halo-n-butyl, haloisobutyl, halo-sec-butyl, halo-tert-butyl, halopentyl, haloisoamyl, haloneopentyl, halo-tert-pentyl, halohexyl, haloheptyl, halooctyl, halononyl, and halodecyl. The term "halo-C₁₋₃ alkyl" or "halo-C₁₋C₃ alkyl" of the present disclosure refers to an alkyl group containing from 1 to 3 carbon atoms substituted with one or more halogens, and its representative examples include halomethyl, haloethyl, halo-n-propyl and haloisopropyl.

As used herein, the term "alkylene" (which is used interchangeably with "alkylene chain"), used alone or in combination, refers to a linear or branched divalent saturated hydrocarbon group composed of carbon and hydrogen atoms. The term "Cₓ-C_{y} alkylene" or "C_{x-y} alkylene" (x and y each being an integer) refers to a linear or branched alkylene group containing from x to y carbon atoms. Examples of the C₁-C₃₀ alkylene in the present disclosure may include C₁-C₃₀ alkylene, C₁-C₂₉ alkylene, C₁-C₂₈ alkylene, C₁-C₂₇ alkylene, C₁-C₂₆ alkylene, C₁-C₂₅ alkylene, C₁-C₂₄ alkylene, C₁-C₂₃ alkylene, C₁-C₂₂ alkylene, C₁-C₂₁ alkylene, C₁-C₂₀ alkylene, C₁-C₁₉ alkylene, C₁-C₁₈ alkylene, C₁-C₁₇ alkylene, C₁-C₁₆ alkylene, C₁-C₁₅ alkylene, C₁-C₁₄ alkylene, C₁-C₁₃ alkylene, C₁-C₁₂ alkylene, C₁-C₁₁ alkylene, C₁-C₁₀ alkylene, C₁-C₉ alkylene, C₁-C₈ alkylene, C₁-C₇ alkylene, C₁-C₆ alkylene, C₁-C₅ alkylene, C₁-C₄ alkylene, C₁-C₃ alkylene, or C₁-C₂ alkylene. Representative examples include, but are not limited to, methylene, ethylene, propylene, isopropylene, butylene, isobutylene, sec-butylene, tert-butylene, n-pentylene, isopentylene, neopentylidene, tert-pentylene, hexylene, heptylene, octylene, nonylene, decylene, undecylene, dodecylene, tridecylene, tetradecylene, pentadecylene, hexadecylene, heptadecylene, octadecylene, nonadecylene, eicosylene, heneicosylene, docosylene, tricosylene, tetracosylene, pentacosylene, hexacosylene, heptacosylene, octacosylene, nonacosylene, and triacontylene. In the present disclosure, the "alkylene" is optionally substituted with one or more substituents optionally selected from C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof.

As used herein, the term "alkoxy", used alone or in combination, refers to a linear or branched alkoxy group having structural formula of -O-alkyl. Optionally, the alkyl portion of the alkoxy group may contain 1-10 carbon atoms. Representative examples of "alkoxy" include, but are not limited to, methoxy, ethoxy, propoxy, isopropoxy, n-butoxy, isobutoxy, tert-butoxy, pentyloxy, 2-pentyloxy, isopentyloxy, neopentyloxy, hexyloxy, 2-hexyloxy, 3-hexyloxy, 3-methylpentyloxy, etc. The term "C₁-C₃ alkoxy" or "C₁₋₃ alkoxy" refers to a linear or branched alkoxy group containing from 1 to 3 carbon atoms. Representative examples of C₁₋₃ alkoxy include, but are not limited to, methoxy, ethoxy, n-propoxy, and isopropoxy.

As used herein, the term "heteroaryl", used alone or in combination, refers to a 5- to 20-membered (e.g., 5- to 15-membered, 5- to 12-membered, 5- to 11-membered, 5- to 10-membered, 5-to 9-membered, 5- to 8-membered, 5- to 7-membered, 5- to 6-membered, 6- to 15-membered, or 6- to 9-membered) monocyclic, bicyclic, or polycyclic cyclic hydrocarbon radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroaryl groups include bicyclic, tricyclic or tetracyclic heteroaryl groups, which contain one aromatic ring having one or more heteroatoms independently selected from O, S and N, and the remaining rings which may be a saturated, partially unsaturated or aromatic ring and can be carbocyclic ring or contain one or more heteroatoms independently selected from O, S and N. Examples of monocyclic heteroaryl groups include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, tetrazolyl, and triazinyl. Examples of bicyclic heteroaryl groups include, but are not limited to, indolyl, isoindolyl, isoindolinyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, oxazolopyridyl, furopyridyl, pteridyl, purinyl, pyridopyridyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl and imidazo[2,1-b]thiazolyl. Examples of tricyclic heteroaryl groups include, but are not limited to, acridinyl, benzindolyl, carbazolyl, dibenzofuranyl, and xanthyl. The heteroaryl group may be unsubstituted or substituted. A substituted heteroaryl refers to heteroaryl substituted one or more times (e.g., 1-4, 1-3, or 1-2 times) by a substituent(s) optionally selected from the group consisting of C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof.

As used herein, the term "heteroarylene", used alone or in combination, refers to a 5- to 20-membered (e.g., 5- to 15-membered, 5- to 12-membered, 5- to 11-membered, 5- to 10-membered, 5-to 9-membered, 5- to 8-membered, 5- to 7-membered, 5- to 6-membered, 6- to 15-membered, or 6- to 9-membered) bivalent monocyclic, bicyclic, or polycyclic cyclic hydrocarbon radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroarylene groups include bicyclic, tricyclic or tetracyclic heteroarylene groups, which contain one aromatic ring having one or more heteroatoms independently selected from O, S and N, and the remaining ring(s) which may be a saturated, partially unsaturated or aromatic ring and can be carbocyclic ring or contain one or more heteroatoms independently selected from O, S and N. Examples of monocyclic heteroarylene groups include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, tetrazolylene, and triazinylene. Examples of bicyclic heteroarylene groups include, but are not limited to, indolylene, isoindolylene, isoindolinylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, oxazolopyridylene, furopyridylene, pteridylene, purinylene, pyridopyridylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, and imidazo[2,1-b]thiazolylene. Examples of tricyclic heteroarylene groups include, but are not limited to, acridinylene, benzindolylene, carbazolylene, dibenzofuranylene, and xanthylene. The heteroarylene group may be unsubstituted or substituted. A substituted heteroarylene refers to heteroarylene substituted one or more times (e.g., 1-4, 1-3, or 1-2 times) by a substituent(s) optionally selected from the group consisting of C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof.

As used herein, the term "aryl", used alone or in combination, refers to a monovalent aromatic hydrocarbon group containing from 5 to 14 carbon atoms and optionally one or more fused rings, such as phenyl group, naphthyl group, or fluorenyl group. As used herein, the "aryl" is optionally substituted. A substituted aryl group refers to an aryl group substituted one or more times (e.g., 1-4, 1-3, or 1-2 times) with a substituent(s). For example, aryl is mono-, di-, or tri-substituted with a substituent(s) optionally selected from e.g., C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof.

As used herein, the term "arylene", used alone or in combination, refers to a divalent aromatic hydrocarbon group containing from 5 to 14 carbon atoms and optionally one or more fused rings, such as phenylene, naphthylene, or fluorenylene. As used herein, the "arylene" is optionally substituted. A substituted arylene refers to an arylene group optionally substituted one or more times (e.g., 1-4, 1-3, or 1-2 times) with a substituent(s). For example, arylene is mono-, di-, or tri-substituted with a substituent(s) optionally selected from e.g., C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof.

As used herein, the term "cycloalkyl", used alone or in combination, refers to a saturated or partially unsaturated (i.e., containing one or more double bonds, but not having a fully conjugated π-electron system) monocyclic or bicyclic or polycyclic cyclic hydrocarbon radical, which in some embodiments contains from 3 to 20 carbon atoms (i.e., C₃₋₂₀ cycloalkyl), or from 3 to 15 carbon atoms (i.e., C₃₋₁₅ cycloalkyl), or from 3 to 12 carbon atoms (i.e., C₃₋₁₂ cycloalkyl), or from 3 to 11 carbon atoms (i.e., C₃₋₁₁ cycloalkyl), or from 3 to 10 carbon atoms (i.e., C₃₋₁₀ cycloalkyl), or from 3 to 8 carbon atoms ( i.e., C₃₋₈ cycloalkyl), or from 3 to 7 carbon atoms (i.e., C₃₋₇ cycloalkyl), or from 3 to 6 carbon atoms (i.e., C₃₋₆ cycloalkyl). The term "cycloalkyl" includes monocyclic, bicyclic, or tricyclic cyclic hydrocarbon radical having from 3 to 20 carbon atoms. Representative examples of monocyclic cycloalkyl groups include, but are not limited to, cyclopropyl, cyclobutyl, cyclopentyl, cyclopentenyl, cyclohexyl, cyclohexenyl, cycloheptyl, and cyclooctyl. Bicyclic and tricyclic cycloalkyl groups include bridged cycloalkyl, fused cycloalkyl and spiro-cycloalkyl groups such as, but not limited to, decalinyl, octahydropentalenyl, octahydro-1H-indenyl, spiro-cycloalkyl, adamantanyl, noradamantanyl, bornyl, norbornyl (also named as bicyclo[2.2.1]heptyl by the IUPAC system). As used herein, the "cycloalkyl" is optionally mono- or poly-substituted, such as, but not limited to, 2,2-, 2,3-, 2,4-, 2,5-, or 2,6-disubstituted cyclohexyl. The substituents of the substituted "cycloalkyl" can be optionally one or more (e.g., 1-5, 1-4, 1-3, 1-2, or 1) substituents selected from C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof. Examples of "C₃₋₆ cycloalkyl" include, but are not limited to, cyclopropyl, cyclobutyl, cyclopentyl, cyclopentenyl, and cyclohexyl.

As used herein, the term "C_{x-y} spiro-cycloalkyl" (x and y each being an integer), used alone or in combination, refers to a spiro-cycloalkyl group containing from x to y carbon atoms. As used herein, the term "C₇₋₁₁ spiro-cycloalkyl", used alone or in combination, refers to a spiro-cycloalkyl group containing from 7 to 11 carbon atoms (e.g., 7-10, 7-9 carbon atoms). Representative examples of "C₇₋₁₁ spiro-cycloalkyl" include, but are not limited to, spiro[3.3]heptyl, spiro[2.5]octyl, spiro[3.5]nonyl, spiro[4.4]nonyl, spiro[4.5]decyl, or spiro[5.5]undecyl. The "C₇₋₁₁ spiro-cycloalkyl" is optionally further substituted with one or more substituents selected from the group consisting of C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof.

As used herein, the term "cycloalkylene", used alone or in combination, refers to a saturated or partially unsaturated (i.e., containing one or more double bonds, but not having a fully conjugated π-electron system) monocyclic or bicyclic or polycyclic divalent cyclic hydrocarbon radical containing from 3 to 12 carbon atoms (e.g., 3-12, 3-11, 3-10, 3-8, 3-7, 3-6 carbon atoms). The term "cycloalkylene" includes monocyclic, bicyclic or tricyclic cycloalkylene having from 3 to 12 carbon atoms. Representative examples of monocyclic cycloalkylene groups include, but are not limited to, cyclopropylene, cyclobutylene, cyclopentylene, cyclopentenylene, cyclohexylene, cyclohexenylene, cycloheptylene, and cyclooctylene. Bicyclic and tricyclic cycloalkylene groups include bridged cycloalkylene, fused cycloalkylene and spiro-cycloalkylene groups such as, but not limited to, decalinylene, octahydropentalenylene, octahydro-1H-indenylene, 2,3-dihydro-1H-indenylene, spiro-cycloalkylene, adamantanylene, noradamantanylene, and norbornylene (also named as bicyclo[2.2.1]heptylene by the IUPAC system). As used herein, the "cycloalkylene" is optionally mono- or poly-substituted, such as, but not limited to, 2,2-, 2,3-, 2,4-, 2,5-, or 2,6-disubstituted cyclohexylene. The substituents of the substituted "cycloalkylene" can be optionally one or more substituents selected from C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof.

As used herein, the term "C_{x-y} spiro-cycloalkylene" (x and y each being an integer), used alone or in combination, refers to a spiro-cycloalkylene group containing from x to y carbon atoms. As used herein, the term "C₇₋₁₁ spiro-cycloalkylene", used alone or in combination, refers to a spiro-cycloalkylene group containing from 7 to 11 carbon atoms (e.g., 7-10, 7-9 carbon atoms). Representative examples of "C₇₋₁₁ spiro-cycloalkylene" include, but are not limited to, spiro[3.3]heptylene, spiro[2.5]octylene, spiro[3.5]nonylene, spiro[4.4]nonylene, spiro[4.5]decylene, or spiro[5.5]undecylene. The "C₇₋₁₁ spiro-cycloalkylene" is optionally further substituted with one or more substituents selected from the group consisting of C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof.

As used herein, the term "heterocyclyl" or "heterocyclic group", used alone or in combination, refers to a 3- to 20-membered saturated or partially unsaturated (i.e., containing one or more double bonds, but not having a fully conjugated π-electron system) monocyclic, bicyclic, or tricyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. In some embodiments, "heterocyclyl" may refer to a 3- to 15-membered (e.g., 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated π-electron system) monocyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. Representative examples of the monocyclic heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, and diazacycloheptanyl (e.g., 1,4-diazacycloheptan-1-yl), and diazacyclooctyl. Bicyclic and tricyclic heterocyclyl groups include bridged heterocyclyl, fused heterocyclyl and spiro-heterocyclyl groups such as, but not limited to, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, 2,5-diazabicyclo[2.2.2]octan-2-yl, and azaspirocycloalkyl (including 3-azaspiro[5.5]undecan-3-yl). The heterocyclyl may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

As used herein, the term "nitrogen-containing monocyclic heterocyclyl", used alone or in combination, refers to 3- to 20-membered (e.g., 3- to 15-membered, 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated π-electron system) monocyclic monovalent cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Representative examples of the nitrogen-containing monocyclic heterocyclyl include, but are not limited to, azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl (e.g., 1,4-diazacycloheptan-1-yl), and diazacyclooctyl. The nitrogen-containing monocyclic heterocyclyl may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

As used herein, the term "heterocyclylene", used alone or in combination, refers to a 3- to 20-membered saturated or partially unsaturated (i.e., containing one or more double bonds, but not having a fully conjugated π-electron system) monocyclic, bicyclic, or tricyclic bivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. In some embodiments, "heterocyclylene" may refer to e.g., a 3- to 15-membered (optionally 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated π-electron system) monocyclic bivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. Representative examples of the monocyclic heterocyclylene include, but are not limited to, azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, and diazacycloheptanylene (e.g., 1,4-diazacycloheptanylene, 4,5-diazacycloheptanylene, 1,3-diazacycloheptanylene). Bicyclic and tricyclic heterocyclylene groups include bridged heterocyclylene, fused heterocyclylene and spiro-heterocyclylene groups such as, but not limited to, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and azaspirocycloalkylene (e.g., 3-azaspiro[5.5]undecanylene). The heterocyclylene may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

As used herein, the term "nitrogen-containing heterocyclylene", used alone or in combination, refers to 3- to 20-membered (e.g., 3- to 15-membered, 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated π-electron system) monocyclic, bicyclic, or tricyclic bivalent cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Representative examples of the nitrogen-containing heterocyclylene include, but are not limited to, piperidinylene, piperazinylene, morpholinylene, azetidinylene, pyrrolidinylene, imidazolidinylene, pyrazolidylene, oxazolidinylene, thiazolidinylene, thiomorpholinylene, azacycloheptylene, diazacycloheptanylene, azacyclooctylene, and diazacyclooctylene. The nitrogen-containing heterocyclylene may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

As used herein, the term "alkynylene", used alone or in combination, refers to a linear or branched bivalent hydrocarbon group containing from 2 to 8 (e.g., from 2 to 6, from 2 to 5, from 2 to 4, or preferably 2) carbon atoms and having one or more (e.g., from 1 to 3, from 1 to 2, or 1) carbon-carbon triple bonds. Examples of alkynylene include, but are not limited to, ethynylene, 1-propynylene, 1-butynylene, and 1,3-diynylene.

As used herein, the term "alkynyl", used alone or in combination, refers to a linear or branched monovalent hydrocarbon group containing from 2 to 8 (e.g., from 2 to 6, from 2 to 5, from 2 to 4, or preferably 2) carbon atoms and having one or more (e.g., from 1 to 3, from 1 to 2, or 1) carbon-carbon triple bonds. Examples of C₂₋₆ alkynylene include, but are not limited to, ethynyl, 1-propynyl, 1-butynyl, and 1,3-diynyl.

As used herein, the term "alkenylene", used alone or in combination, refers to a linear or branched divalent hydrocarbon group containing from 2 to 8 (e.g., from 2 to 6, from 2 to 5, from 2 to 4, from 2 to 3, or 2) carbon atoms and having one or more (e.g., from 1 to 3, from 1 to 2, or 1) carbon-carbon double bonds. Examples of alkenylene groups include, but are not limited to, vinylene (e.g., -CH=CH-), 1-propenylene, allylidene, 1-butenylene, 2-butenylene, 3-butenylene, isobutenylene, pentenylene, n-pent-2,4-dienylene, 1-methyl-but-1-enylene, 2-methyl-but-1-enylene, 3-methyl-but-1-enylene, 1-methyl-but-2-enylene, 2-methyl-but-2-enylene, 3-methyl-but-2-enylene, 1-methyl-but-3-enylene, 2-methyl-but-3-enylene, 3-methyl-but-3-enylene, and hexenylene.

As used herein, the term "alkenyl", used alone or in combination, refers to a linear or branched monovalent hydrocarbon group containing from 2 to 8 (e.g., from 2 to 6, from 2 to 5, from 2 to 4, from 2 to 3, or 2) carbon atoms and having one or more (e.g., from 1 to 3, from 1 to 2, or 1) carbon-carbon double bonds. Examples of C₂₋₆ alkenyl group include, but are not limited to, vinyl (e.g., CH₂=CH-), 1-propenyl, allyl, 1-butenyl, 2-butenyl, 3-butenyl, isobutenyl, pentenyl, n-pent-2,4-dienyl, 1-methyl-but-1-enyl, 2-methyl-but-1-enyl, 3-methyl-but-1-enyl, 1-methyl-but-2-enyl, 2-methyl-but-2-enyl, 3-methyl-but-2-enyl, 1-methyl-but-3-enyl, 2-methyl-but-3-enyl, 3-methyl-but-3-enyl, and hexenyl.

As used herein, "bornylane" or "bornane" (also known as 1,7,7-trimethylbicyclo[2.2.1]heptane; camphane; bornylane) has a definition known to those skilled in the art. As used herein, "camphanyl" or "bornyl" refers to a monovalent group of bornane, i.e., the group remaining after any one of the hydrogens in bornane is removed. Representative examples of "bornyl" include, but are not limited to, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl,

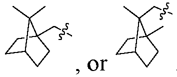

As used herein, "bicyclo[2.2.1]heptane" also known as "norbornane", has a definition known to those skilled in the art. As used herein, "bicyclo[2.2.1]heptyl" or "norbornyl" refers to a monovalent group of bicyclo[2.2.1]heptane, i.e., the group remaining after any hydrogen in bicyclo[2.2.1]heptane is removed. Representative examples of "bicyclo[2.2.1]heptyl" include, but are not limited to, bicyclo[2.2.1]heptan-2-yl, bicyclo[2.2.1]heptan-3-yl, bicyclo[2.2.1]heptan-4-yl, bicyclo[2.2.1]heptan-5-yl, or bicyclo[2.2.1]heptan-6-yl.

As used herein, the term "bicyclo[2.2.1]heptene" has a definition known to those skilled in the art. As used herein, "bicyclo[2.2.1]heptenyl" refers to a monovalent group of bicyclo[2.2.1]heptene, i.e., the group remaining after any hydrogen in bicyclo[2.2.1]heptene is removed. Representative examples of "bicyclo[2.2.1]heptenyl" include, but are not limited to, bicyclo[2.2.1]hept-5-en-2-yl, bicyclo[2.2.1]hept-5-en- 3-yl, or bicyclo[2.2.1]hept-5-en-7-yl.

As used herein, "adamantane" (also known as tricyclo[3.3.1.1^{3,7}]decane) has a definition known to those skilled in the art, and its structural formula is e.g., as follows: 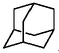 As used herein, "adamantanyl" refers to a monovalent group of adamantane, that is, the group remaining after any hydrogen in adamantane is removed. Representative examples of "adamantanyl" include, but are not limited to, 1-adamantanyl, 2-adamantanyl, 3-adamantanyl, 4-adamantanyl, 5-adamantanyl, 6-adamantanyl, 7-adamantanyl, 8-adamantanyl, 9-adamantanyl, or 10-adamantanyl.

As used herein, "noradamantane" has a definition known to those skilled in the art, and its structural formula is e.g., as follows: 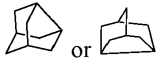 As used herein, "noradamantanyl" refers to a monovalent group of noradamantane, that is, the group remaining after any hydrogen in noradamantane is removed. Representative examples of "noradamantanyl" include, but are not limited to, 1-noradamantanyl, 2-noradamantanyl, 3-noradamantanyl, 4-noradamantanyl, 5-noradamantanyl, 6-noradamantanyl, 7-noradamantanyl, 8-noradamantanyl or 9-noradamantanyl.

As used herein, "adamantanamine" has the definitions known to those skilled in the art, namely referring to an adamantane having an amino substituent, wherein the amino substituent can replace a hydrogen on a carbon at any position in the adamantane. An example of "adamantanamine" can be adamantan-1-amine (corresponding English chemical name is adamantan-1-amine or Tricyclo[3.3.1.1^{3,7}]decan-1-amine; CAS No.: 768-94-5), with the following structural Formula:

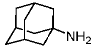

Salts or pharmaceutically acceptable salts, enantiomers, stereoisomers, solvates, polymorphs of the compounds of Formula (I) and compounds in tables 1 or 2 of the present disclosure are also encompassed within the scope of the present invention.

In all embodiments of the present disclosure, the salts or pharmaceutically acceptable salts of the compounds of the present disclosure (including the compounds of any one of Formula (I), Formula (I-1), Formula (I-2), Formula (I-3), Formula (I-2-1), Formula (I-2-2), Formula (I-2-3), Formula (I-2-4), Formula (I-2-5), Formula (I-3-1), Formula (I-3-2), Formula (I-3-3), or Formula (I-3-4), or compounds in tables 1 or 2) refer to non-toxic inorganic or organic acid and/or base addition salts. Examples include: sulfates, hydrochlorides, citrates, maleates, methanesulfonates, citrates, lactates, tartrates, fumarates, phosphates, dihydrophosphates, pyrophosphates, metaphosphates, oxalates, malonates, benzoates, mandelates, succinates, glycolate, or p-toluenesulfonates, etc.

"Pharmaceutically acceptable carrier" refers to a pharmaceutically acceptable material, such as a filler, stabilizer, dispersant, suspending agent, diluent, excipient, thickener, solvent, or encapsulating material, with which the useful compounds according to the present disclosure are carried or transported into or administered to a patient so that they can perform their intended function. Generally, such constructs are carried or transported from one organ or part of the body to another organ or part of the body. The carrier is compatible with the other ingredients of the formulation, including the compounds useful in the present disclosure, and is not harmful to the patient, and the carrier must be "acceptable". Some examples of materials that can be used as pharmaceutically acceptable carriers include sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; and other common non-toxic compatible substances used in pharmaceutical formulations.

As used herein, the term "room temperature" refers to the ambient temperature, such as 20-30°C.

As used herein, "stereoisomer" refers to a compound with the same chemical structural formula, but a different arrangement of atoms or groups in space. Stereoisomers include enantiomers, diastereomers, conformational isomers (rotational isomers), geometric isomers (cis/trans isomers), atropisomers, and so on.

As used herein, the term "solvate" refers to an association or complex formed by the interaction between one or more solvent molecules and compounds of the present invention. Examples of solvents include water, isopropanol, ethanol, methanol, DMSO, ethyl acetate, acetic acid and ethanolamine. The term "hydrate" means that a complex formed with water.

As used herein, the term "chiral" refers to a molecule that is nonsuperimposable on its mirror image; whereas "achiral" refers to a molecule that can be superimposed on its mirror image.

As used herein, the term "enantiomers" refers to two isomers of a compound that are nonsuperimposable mirror images.

As used herein, the term "diastereomers" refers to stereoisomers that have two or more chiral centers but are not mirror images of each other. The diastereomers have different physical properties, such as melting point, boiling point, spectral properties and reactivity. The diastereomeric mixture can be separated by high-resolution analytical operations such as electrophoresis and chromatography, such as HPLC.

### Examples

In the following description, numerous specific details are set forth in order to provide a thorough understanding of the present disclosure. The present disclosure may be practiced without some or all of these specific details. In other cases, well-known process operations have not been described in detail in order not to unnecessarily obscure the present disclosure. Although the present disclosure will be described in conjunction with specific embodiments, it should be understood that this is not intended to limit the present disclosure to these embodiments.

The following abbreviations are used throughout the specification and examples:
- AcOH: acetic acid
- Boc: t-Butyloxy carbonyl
- Con.: Concentration
- DCM: dichloromethane
- DIEA: N, N-diisopropylethylamine
- DMAP: 4-Dimethylaminopyridine
- DMF: N,N-dimethylformamide
- DMSO: dimethyl sulfoxide
- DIPEA: N, N-diisopropylethylamine
- DESS-MARTIN: Dess-Martin Oxidation
- EA: Ethyl acetate
- EDCI: 1-(3-dimethylaminopropyl)-3-ethylcarbodiimide hydrochloride
- ESI: electrospray ionization
- equiv: equivalent
- EtOH: ethanol
- HATU: 2-(7-Azabenzotriazol-1-yl)-N,N,N',N'-tetramethyluronium hexafluorophosphate
- HFIP: Hexafluoroisopropanol
- HPLC: high performance liquid chromatography
- HRMS: high resolution mass spectrometry
- LC-MS: liquid chromatography-mass spectrometry
- LRMS: low resolution mass spectrometry
- LC: liquid chromatography
- Me: methyl
- MeCN: acetonitrile
- MeOH: Methanol
- MS: mass spectrometry
- NBS: N-Bromosuccinimide
- ¹H NMR: Proton nuclear magnetic resonance
- PE: petroleum ether
- (PhCOO)₂: Dibenzoyl peroxide
- Raney Ni: Raney nickel
- rt: room temperature
- tBu: tert-butyl
- TEA: triethylamine
- TFA: trifluoroacetic acid
- THF: Tetrahydrofuran
- TLC: thin layer chromatography
- TMS: tetramethylsilane

In the present disclosure, the ¹H NMR spectrum was recorded on a Bruker-500MHz nuclear magnetic resonance instrument, by using, as a solvent, CD₃OD (δ = 3.31 ppm) containing 0.1% TMS (as an internal standard); or using, as a solvent, CDCl₃ (δ = 7.26 ppm) containing 0.1% TMS (as an internal standard); or using, as a solvent, DMSO-*d*₆ (δ = 2.50 ppm) containing 0.03% TMS (as an internal standard). LRMS spectrum was recorded on an AB Triple 4600 mass spectrometer, HPLC preparation was measured on a SHIMADZU LC-20AP type instrument, and HPLC purity was measured on a SHIMADZU LC-30AP or Waters 1525 type instrument. Unless otherwise specified, all reactions were performed in the air atmosphere. The reactions were monitored via TLC or LC-MS.

Solvents and reagents are processed as follows:
the solvents used in the reactions such as DCM, DMF, anhydrous EtOH, and anhydrous MeOH, were all purchased from Sinopharm Group;
preparative HPLC uses preparative grade CH₃CN and deionized water;
unless otherwise specified, the starting materials, reagents, various carbon chain linkers of different lengths (including compounds used to form the groups represented by LIN), and other reagents and drugs used in the following examples were commercially available and used directly, or synthesized using methods known in the art.

### General synthesis methods

Compounds and/or pharmaceutically acceptable salts thereof of the present disclosure can be synthesized using commercially available raw materials by synthetic techniques known in the art. The synthetic schemes described below illustrate the preparation of most compounds. The starting materials or reagents used in each scheme can be purchased from commercial sources or prepared by methods known to those skilled in the art. One skilled in the art can prepare the salts, racemates, enantiomers, phosphates, sulfates, hydrochlorides and prodrug forms of the compounds of Formula (I) and compounds in tables 1 or 2 of the present disclosure according to routine techniques in the art.

**General synthesis method I of the intermediate corresponding to the R₁₁-LIN part of the compound of Formula (I):**

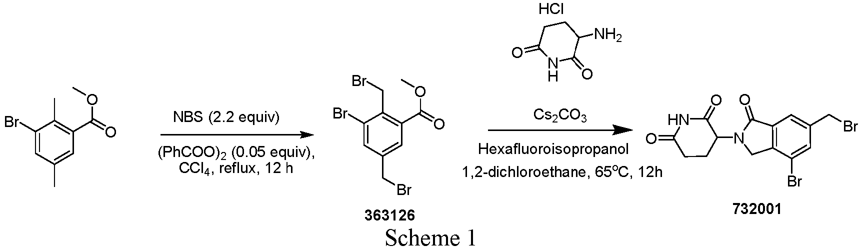

General synthesis method II of the intermediate corresponding to the **R₁₁-LIN** part of the compound of Formula (I):

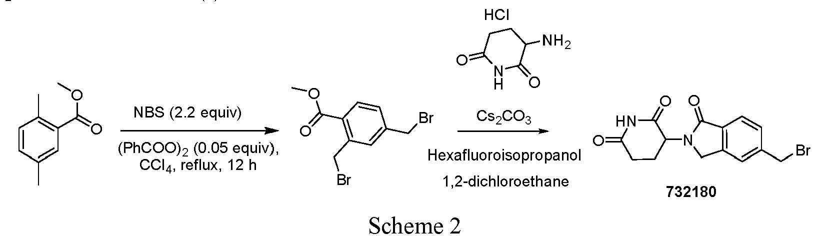

The intermediate compound 732180 was prepared according to the method of Scheme 1.

General synthesis method I of the Binder intermediate compound:

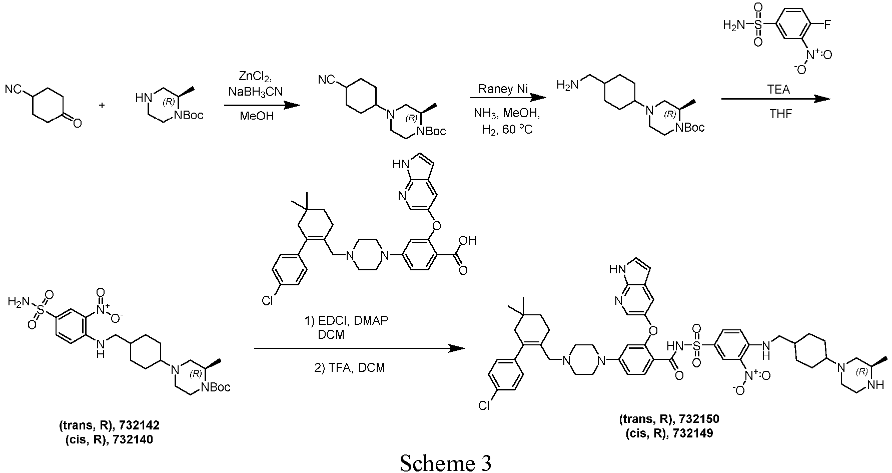

General synthesis method II of the Binder intermediate compound:

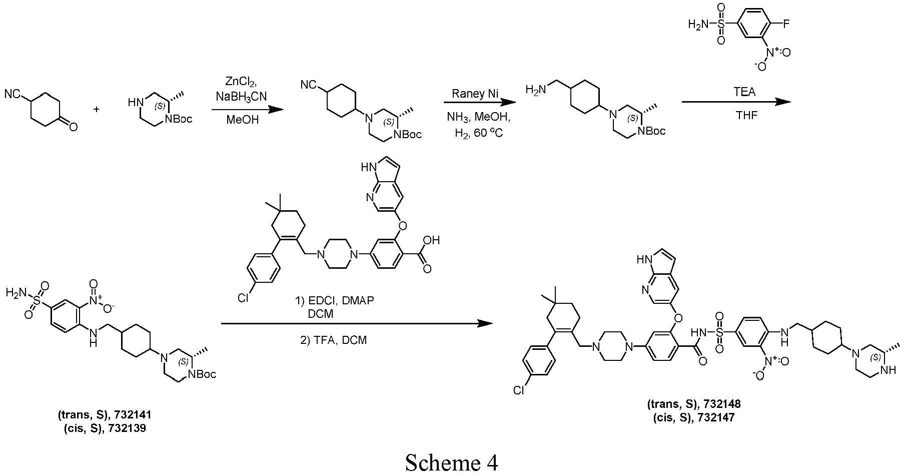

General synthesis method III of the Binder intermediate compound:

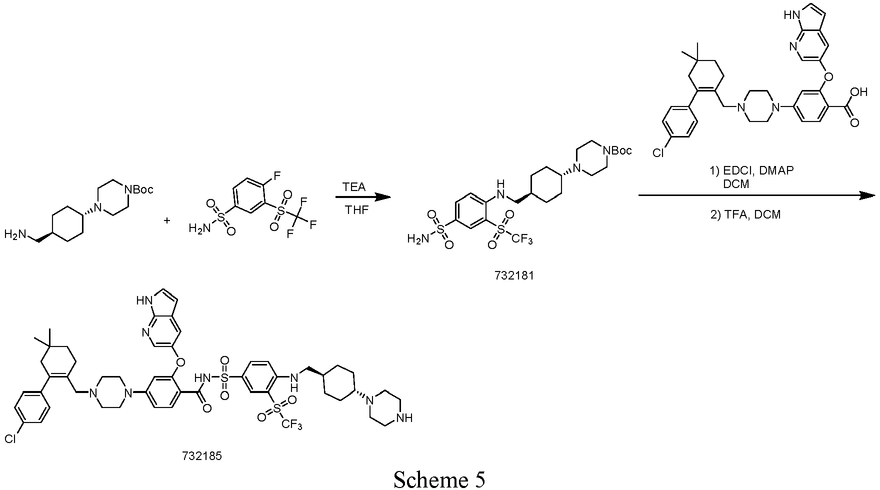

General synthesis method IV of the Binder intermediate compound:

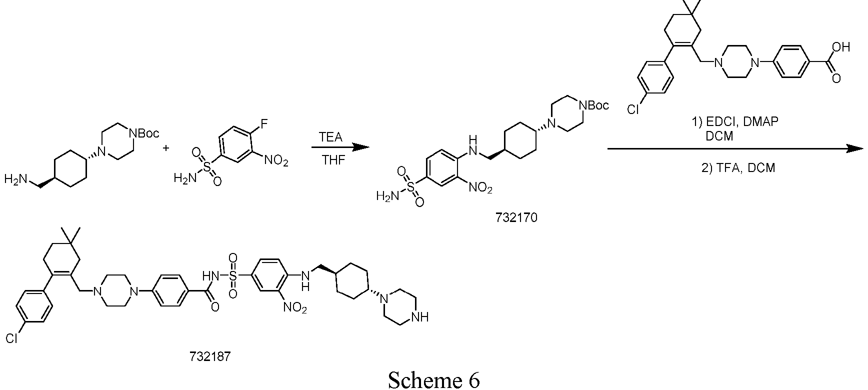

**General synthesis method of the compound of Formula (I):**

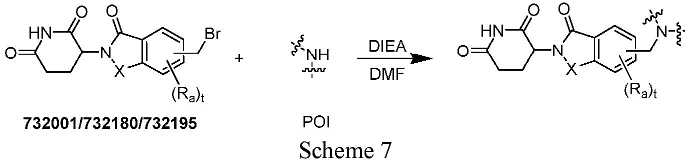

In Scheme 7, POI shows the reactive group of the Binder intermediate, and (Rₐ)ₜ and X are as defined in the compound of Formula (I) of the present disclosure.

To a solution of brominated substrate (1.0 equiv) in DMF were added the corresponding binder intermediate compound (1.0 equiv) and DIEA (2.0 equiv). The reaction mixture was allowed to react at room temperature for 2 hours, and the reaction was complete. The reaction mixture was subjected to a C18 reverse phase column chromatography (eluent (v/v): acetonitrile/(water + 0.05% HCl) = 10% -100%) for separation and purification to give the corresponding target product.

Depending upon the target compounds, the above schemes and their reaction substrates, reaction conditions (including reaction dosage, temperature, duration, etc.), work up, etc. can be appropriately modified and adjusted by techniques and methods well known to those skilled in the art to obtain the desired target compounds. The obtained target compounds can be further modified by the substituents and the like to obtain subsequent target compounds through methods well known to those skilled in the art.

### Examples

### Intermediate Example 1: preparation of 3-(4-bromo-6-(bromomethyl)-1-oxoisoindolin-2-yl)piperidine-2,6-dione (732001)

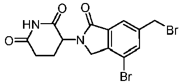

3-(4-bromo-6-(bromomethyl)-1-oxoisoindolin-2-yl)piperidine-2,6-dione (732001) was prepared by referring to the method of Scheme 1.

### Step 1:

To a solution of the raw material methyl 3-bromo-2,5-dimethylbenzoate (5.0 g, 20.6 mmol) in 100 mL of carbon tetrachloride were added sequentially N-Bromosuccinimide (8.0 g, 45 mmol) and dibenzoyl peroxide (0.31 g, 1.26mmol). The reaction mixture was refluxed at 80°C for 12 h, cooled to room temperature, and diluted with 100 mL of petroleum ether. The diluted mixture was washed twice with water and once with saturated saline. The organic phases were separated and combined, dried over anhydrous sodium sulfate, and filtered. The filtrate was distilled under reduced pressure to remove the solvent. The residue was subjected to silica gel chromatography (PE/EA =1/0 - 20/1) for separation to give compound 363126 (6.3 g, yield: 76%) as a white solid.

### Step 2:

Cesium carbonate (16.25 g, 49.89 mmol) was added to a mixed solvent of 500 mL 1,2-dichloroethane and 50 mL hexafluoroisopropanol, and the resulting mixture was stirred at 65°C for 15 min. Then to the resulting mixture were added sequentially 3-aminopiperidine-2,6-dione hydrochloride (8.21 g, 49.89 mmol) and compound 363126 (20 g, 49.89 mmol) obtained in step 1. The reaction mixture was stirred at 65°C for 12h, and then filtered while hot to remove insoluble substances. The filtrate was washed with saturated brine, and concentrated under reduced pressure to remove the organic solvent. The remaining solid was slurried with 50 mL dichloromethane and 200 mL petroleum ether. The resulting mixture was filtered, and the filter cake was washed with 50 mL dichloromethane and 50 mL ether to obtain the product 732001 (11 g, yield: 42%) as gray powder. ¹H NMR (500 MHz, Methanol-*d*₄) δ 7.90 (d, *J =* 1.5 Hz, 1H), 7.84 (d, *J =* 1.5 Hz, 1H), 5.17 (dd, *J =* 13.4, 5.2 Hz, 1H), 4.68 (s, 2H), 4.45 (q, *J =* 17.6 Hz, 2H), 2.91 (ddd, *J =* 17.6, 13.6, 5.4 Hz, 1H), 2.79 (ddd, *J =* 17.6, 4.6, 2.4 Hz, 1H), 2.53 (qd, *J =* 13.4, 4.7 Hz, 1H), 2.19 (dtd, *J =* 13.0, 5.4, 2.4 Hz, 1H). MS (ESI) m/z: calcd for C₁₄H₁₃Br₂N₂O₃⁺ [M+H]⁺, 414.9; found, 415.1.

### Intermediate Example 2: preparation of 3-(5-(bromomethyl)-1-oxoisoindolin-2-yl)piperidine-2,6-dione (732180)

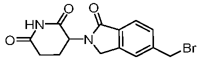

According to Scheme 2, 3-(5-(bromomethyl)-1-oxoisoindolin-2-yl)pipendine-2,6-dione (732180) was prepared by referring to the method of Scheme 1. In addition, the starting material methyl 2,4-bis(bromomethyl)benzoate (CAS No.: 63112-94-7) in step 2 of Scheme 2 can also be commercially available. After the reaction was complete, the reaction mixture was washed with water. The organic phases were separated and concentrated under reduced pressure to remove the solvent. The resulting residue was subjected to silica gel chromatography (DCM/MeOH = 1/0 - 10/1) for separation to give the target product which was slurried with acetonitrile for further purification to give a white powder with a yield of 27%. ¹H NMR (500 MHz, DMSO-*d*₆) δ 11.00 (s, 1H), 7.78 - 7.66 (m, 2H), 7.59 (dd, *J =* 7.9, 1.5 Hz, 1H), 5.11 (dd, *J =* 13.3, 5.1 Hz, 1H), 4.83 (s, 2H), 4.47 (d, *J =* 17.3 Hz, 1H), 4.34 (d, *J =* 17.4 Hz, 1H), 2.91 (ddd, *J =* 17.3, 13.6, 5.4 Hz, 1H), 2.68 - 2.55 (m, 1H), 2.39 (qd, *J* = 13.3, 4.5 Hz, 1H), 2.01 (dtd, *J* = 12.7, 5.3, 4.7, 1.9 Hz, 1H). MS (ESI) m/z: calcd for C₁₄H₁₄BrN₂O₃⁺ [M+H]⁺, 337.0; found, 337.3.

### Intermediate Example 3: preparation of 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((cis)-4-((S)-3-methylpiperazin-1-yl)cyclohexyl)methyl)amino)-3-nitrophenyl)sulfonyl)benzamide (732147) and 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-((S)-3-methylpiperazin-1-yl)cyclohexyl)methyl)amino)-3-nitrophenyl)sulfonyl)benzamide (732148)

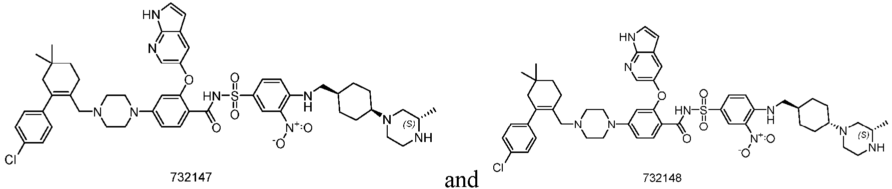

2-((1H-pyrrolo[2,3-b]pyridin-5-yl)oxy)-4-(4-((4'-chloro-5,5-dimethyl-3,4,5,6-tetrahydro-[1,1 '-biphenyl] -2-yl)methyl)piperazin-1 -yl)-N-((4-((((cis)-4-((S)-3 -methylpiperazin-1 - yl)cyclohexyl)methyl)amino)-3-nitrophenyl)sulfonyl)benzamide (732147) and 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-((S)-3-methylpiperazin-1-yl)cyclohexyl)methyl)amino)-3-nitrophenyl)sulfonyl)benzamide (732148) were prepared by referring to the method of Scheme 4.

### Step 1:

To a solution of 4-oxocyclohexane-1-carbonitrile (1 equiv) in methanol were added tert-butyl (S)-2-methylpiperazine-1-carboxylate (1.5 equiv) and zinc chloride (1.5 equiv). The resulting mixture was stirred at room temperature for 15 minutes, cooled to 0°C, followed by addition of sodium cyanoborohydride (3.0 equiv). The reaction mixture was allowed to react overnight. A saturated solution of ammonium chloride was added to the reaction mixture to quench the reaction. The resulting mixture was extracted three times with dichloromethane and washed once with saturated saline. The organic phases were separated and subjected to pressure distillation to remove the solvent. The residue was subjected to column chromatography (DCM/MeOH = 1/0 - 10/1) for separation to give the cyano product as a white solid, which is a mixture of cis- and trans-products, with a yield of 80%.

### Step 2:

A reaction flask containing a solution of the cyano compound obtained in step 1 in methanol was charged with a solution of NH₃ in methanol (7M, 0.5 mL) and Raney nickel. The reaction flask was purged with hydrogen gas. The reaction mixture was heated to 60°C and carried out for hydrogenation under normal pressure and allowed to react overnight. The reaction solution was cooled to room temperature, and filtered by diatomite filter to remove the Raney nickel. The filtrate was rotary evaporated to dryness, giving a crude amine product, which was directly used in the next step.

### Step 3:

To a solution of the amine product obtained from the previous step in THF were added 4-fluoro-3-nitrobenzenesulfonamide (0.9 equiv) and triethylamine (1.5 equiv). The reaction mixture was stirred at room temperature overnight. After the reaction was complete, to the reaction mixture was added a saturated aqueous solution of sodium bicarbonate. The resulting mixture was extracted three times with dichloromethane. The dichloromethane organic phases were combined and washed with saturated saline. The organic phases were separated and concentrated under reduced pressure to remove the solvent. The residue was subjected to column chromatography (DCM/MeOH = 1/0 - 10/1) for separation to successfully give the cis-product (732139) and the trans-product (732141).
the cis-product (732139): tert-butyl (*S*)-2-methyl-4-((cis)-4-(((2-nitro-4-sulfamoylphenyl)amino)methyl)cyclohexyl)piperazine-1-carboxylate
¹H NMR (500 MHz, DMSO-*d*₆) δ 8.54 (dt, *J* = 7.0, 5.1 Hz, 1H), 8.47 (d, *J =* 2.3 Hz, 1H), 7.82 (dd, *J =* 9.1, 2.3 Hz, 1H), 7.33 (s, 2H), 7.26 (d, *J =* 9.3 Hz, 1H), 4.07 (s, 1H), 3.68 (d, *J =* 12.7 Hz, 1H), 3.38 (d, *J* = 6.5 Hz, 2H), 2.99 - 2.83 (m, 2H), 2.77 (d, *J* = 11.3 Hz, 1H), 2.13 (s, 1H), 2.00 (d, *J* = 10.9 Hz, 1H), 1.86 (d, *J =* 9.9 Hz, 2H), 1.81 - 1.66 (m, 2H), 1.53 (d, *J =* 7.0 Hz, 2H), 1.48 - 1.42 (m, 3H), 1.39 (s, 9H), 1.14 (d, *J* = 6.7 Hz, 3H).
the trans-product (732141): tert-butyl (S)-2-methyl-4-((trans)-4-(((2-nitro-4-sulfamoylphenyl)amino)methyl)cyclohexyl)piperazine-1-carboxylate
¹H NMR (500 MHz, DMSO-*d*₆) δ 8.56 (t, *J* = 5.9 Hz, 1H), 8.47 (d, *J* = 2.3 Hz, 1H), 7.81 (dd, *J* = 9.1, 2.3 Hz, 1H), 7.33 (s, 2H), 7.25 (d, *J* = 9.3 Hz, 1H), 4.04 (s, 1H), 3.63 (d, *J* = 12.7 Hz, 1H), 3.29 (t, *J* = 6.4 Hz, 2H), 2.88 (t, *J* = 12.1 Hz, 1H), 2.71 (d, *J* = 10.9 Hz, 1H), 2.60 (d, *J* = 11.1 Hz, 1H), 2.25 (ddt, *J* = 15.6, 11.0, 5.9 Hz, 2H), 2.12 (td, *J* = 11.4, 3.3 Hz, 1H), 1.86 - 1.70 (m, 4H), 1.57 (dt, *J* = 7.7, 3.6 Hz, 1H), 1.38 (s, 9H), 1.18 (ddd, *J* = 25.3, 13.9, 7.4 Hz, 2H), 1.10 (d, *J* = 6.7 Hz, 3H), 1.07 - 0.96 (m, 2H).

### Step 4:

To a solution of 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)benzoic acid (1 equiv; cas: 1235865-77-6) in dichloromethane were added EDCI (1.5 equiv) and DMAP (1.0 equiv). The reaction system becomes clear. To the reaction solution was added the cis product (732139) or the trans product
(732141) obtained in step 3. The reaction was complete after 12 hours. The reaction solution was diluted with 30 mL of dichloromethane. The resulting mixture was washed with saturated saline. The organic phases were separated and concentrated under reduced pressure to remove the solvent. The residue was subjected to column chromatography (DCM/MeOH = 1/0 - 10/1) for separation to give the Boc-protected product.

To a solution of the Boc-protected product in dichloromethane was added trifluoroacetic acid (10 equiv). The reaction mixture was allowed to react at room temperature for 30 minutes. After the reaction was complete, the mixture was concentrated under reduced pressure to remove the organic solvent and trifluoroacetic acid. The residue was lyophilized to give the cis-product (732147) and trans-product (732148) as powder form.
the cis-product (732147): 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((cis)-4-((S)-3-methylpiperazin-1 -yl)cyclohexyl)methyl)amino)-3 -nitrophenyl)sulfonyl)benzamide
¹H NMR (500 MHz, Methanol-*d*₄) δ 8.66 (d, *J* = 2.3 Hz, 1H), 8.06 - 7.96 (m, 1H), 7.86 (dd, *J* = 9.2, 2.3 Hz, 1H), 7.62 (d, *J=* 8.9 Hz, 1H), 7.57 (d, *J* = 2.5 Hz, 1H), 7.47 (d, *J* = 3.4 Hz, 1H), 7.35 (d, *J* = 8.4 Hz, 2H), 7.08 (d, *J* = 8.4 Hz, 2H), 7.01 (d, *J* = 9.4 Hz, 1H), 6.76 (dd, *J* = 8.9, 2.4 Hz, 1H), 6.43 (d, *J =* 3.5 Hz, 1H), 6.34 (d, *J =* 2.3 Hz, 1H), 3.99 - 3.84 (m, 3H), 3.80 - 3.60 (d, *J =* 29.2 Hz, 6H), 3.50 - 3.35 (m, 6H), 3.27 - 3.09 (m, 3H), 2.79 (s, 2H), 2.33 - 2.26 (m, 2H), 2.10 (s, 3H), 1.96 (dt, *J* = 31.7, 9.6 Hz, 7H), 1.72 (ddd, *J* = 15.0, 10.4, 4.7 Hz, 2H), 1.56 (t, *J* = 6.4 Hz, 2H), 1.45 (d, *J* = 6.5 Hz, 3H), 1.00 (s, 6H).
the trans-product (732148): 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-((S)-3-methylpiperazin-1-yl)cyclohexyl)methyl)amino)-3-nitrophenyl)sulfonyl)benzamide
¹H NMR (500 MHz, Methanol-*d*₄) δ 8.66 (d, *J =* 2.3 Hz, 1H), 8.00 (s, 1H), 7.85 (dd, *J* = 9.3, 2.3 Hz, 1H), 7.62 (d, *J* = 8.9 Hz, 1H), 7.57 (d, *J* = 2.5 Hz, 1H), 7.47 (d, *J* = 3.4 Hz, 1H), 7.35 (d, *J* = 8.4 Hz, 2H), 7.08 (d, *J* = 8.4 Hz, 2H), 6.95 (d, *J* = 9.3 Hz, 1H), 6.76 (dd, *J* = 9.0, 2.4 Hz, 1H), 6.43 (d, *J* = 3.4 Hz, 1H), 6.33 (d, *J* = 2.4 Hz, 1H), 3.90 - 3.78 (m, 3H), 3.77 - 3.57 (m, 7H), 3.51 - 3.35 (m, 5H), 3.28 (d, *J =* 6.7 Hz, 3H), 3.17 (d, *J =* 15.8 Hz, 2H), 2.79 (s, 2H), 2.33 - 2.24 (m, 4H), 2.10 (s, 2H), 2.06 (d, *J* = 12.0 Hz, 2H), 1.81 - 1.71 (m, 1H), 1.70 - 1.59 (m, 2H), 1.56 (t, *J* = 6.4 Hz, 2H), 1.45 (d, *J* = 6.5 Hz, 3H), 1.25 (ddt, *J* = 16.2, 12.9, 6.2 Hz, 2H), 1.00 (s, 6H).

### Intermediate Example 4: preparation of 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((cis)-4-((R)-3-methylpiperazin-1-yl)cyclohexyl)methyl)amino)-3-nitrophenyl)sulfonyl)benzamide (732149) and 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-((R)-3-methylpiperazin-1-yl)cyclohexyl)methyl)amino)-3-nitrophenyl)sulfonyl)benzamide (732150)

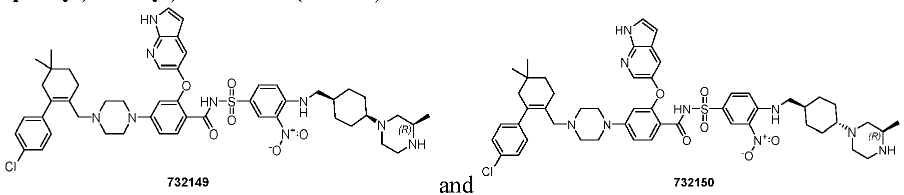

2-((1H-pyrrolo[2,3-b]pyridin-5-yl)oxy)-4-(4-((4'-chloro-5,5-dimethyl-3,4,5,6-tetrahydro-[1,1 '-biphenyl] -2-yl)methyl)piperazin-1 -yl)-N-((4-((((cis)-4-((R)-3 -methylpiperazin-1 - yl)cyclohexyl)methyl)amino)-3-nitrophenyl)sulfonyl)benzamide (732149) and 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-((R)-3-methylpiperazin-1-yl)cyclohexyl)methyl)amino)-3-nitrophenyl)sulfonyl)benzamide (732150) were prepared by referring to the method of Scheme 3.

### Step 1:

To a solution of 4-oxocyclohexane-1-carbonitrile (1 equiv) in methanol were added tert-butyl (R)-2-methylpiperazine-1-carboxylate (1.5 equiv) and zinc chloride (1.5 equiv). The resulting mixture was stirred at room temperature for 15 minutes, cooled to 0°C, followed by addition of sodium cyanoborohydride (3.0 equiv). The reaction mixture was allowed to react overnight. A saturated solution of ammonium chloride was added to the reaction mixture to quench the reaction. The resulting mixture was extracted three times with dichloromethane and washed once with saturated saline. The organic phases were separated and subjected to pressure distillation to remove the solvent. The residue was subjected to column chromatography (DCM/MeOH = 1/0 - 10/1) for separation to give the cyano product as a white solid, which is a mixture of cis- and trans-products, with a yield of 80%.

### Step 2:

A reaction flask containing a solution of the cyano compound obtained in step 1 in methanol was charged with a solution of NH₃ in methanol (7M, 0.5 mL) and Raney nickel. The reaction flask was purged with hydrogen gas. The reaction mixture was heated to 60°C and carried out for hydrogenation under normal pressure and allowed to react overnight. The reaction solution was cooled to room temperature, and filtered by diatomite filter to remove the Raney nickel. The filtrate was rotary evaporated to dryness, giving a crude amine product, which was directly used in the next step.

### Step 3:

To a solution of the amine product obtained from the previous step in THF were added 4-fluoro-3-nitrobenzenesulfonamide (0.9 equiv) and triethylamine (1.5 equiv). The reaction mixture was stirred at room temperature overnight. After the reaction was complete, to the reaction mixture was added a saturated aqueous solution of sodium bicarbonate. The resulting mixture was extracted three times with dichloromethane. The dichloromethane organic phases were combined and washed with saturated saline. The organic phases were separated and concentrated under reduced pressure to remove the solvent. The residue was subjected to column chromatography (DCM/MeOH = 1/0 - 10/1) for separation to successfully give the cis-product (732140) and the trans-product (732142).
the cis-product (732140): tert-butyl (R)-2-methyl-4-((cis)-4-(((2-nitro-4-sulfamoylphenyl)amino)methyl)cyclohexyl)piperazine-1-carboxylate
¹H NMR (500 MHz, DMSO-*d*₆) δ 8.54 (dt, *J* = 7.0, 5.1 Hz, 1H), 8.47 (d, *J =* 2.3 Hz, 1H), 7.82 (dd, *J =* 9.1, 2.3 Hz, 1H), 7.33 (s, 2H), 7.26 (d, *J =* 9.3 Hz, 1H), 4.07 (s, 1H), 3.68 (d, *J =* 12.7 Hz, 1H), 3.38 (d, *J* = 6.5 Hz, 2H), 2.99 - 2.83 (m, 2H), 2.77 (d, *J* = 11.3 Hz, 1H), 2.13 (s, 1H), 2.00 (d, *J* = 10.9 Hz, 1H), 1.86 (d, *J =* 9.9 Hz, 2H), 1.81 - 1.66 (m, 2H), 1.53 (d, *J =* 7.0 Hz, 2H), 1.48 - 1.42 (m, 3H), 1.39 (s, 9H), 1.14 (d, *J* = 6.7 Hz, 3H).
the trans-product (732142): tert-butyl (R)-2-methyl-4-((trans)-4-(((2-nitro-4-sulfamoylphenyl)amino)methyl)cyclohexyl)piperazine-1-carboxylate
¹H NMR (500 MHz, DMSO-*d*₆) δ 8.56 (t, *J* = 5.9 Hz, 1H), 8.47 (d, *J* = 2.3 Hz, 1H), 7.81 (dd, *J* = 9.1, 2.3 Hz, 1H), 7.33 (s, 2H), 7.25 (d, *J* = 9.3 Hz, 1H), 4.04 (s, 1H), 3.63 (d, *J* = 12.7 Hz, 1H), 3.29 (t, *J* = 6.4 Hz, 2H), 2.88 (t, *J* = 12.1 Hz, 1H), 2.71 (d, *J* = 10.9 Hz, 1H), 2.60 (d, *J* = 11.1 Hz, 1H), 2.25 (ddt, *J* = 15.6, 11.0, 5.9 Hz, 2H), 2.12 (td, *J* = 11.4, 3.3 Hz, 1H), 1.86 - 1.70 (m, 4H), 1.57 (dt, *J* = 7.7, 3.6 Hz, 1H), 1.38 (s, 9H), 1.18 (ddd, *J* = 25.3, 13.9, 7.4 Hz, 2H), 1.10 (d, *J* = 6.7 Hz, 3H), 1.07 - 0.96 (m, 2H).

### Step 4:

To a solution of 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)benzoic acid (1 equiv; cas: 1235865-77-6) in dichloromethane were added EDCI (1.5 equiv) and DMAP (1.0 equiv). The reaction system becomes clear. To the reaction solution was added the cis product (732140) or the trans product (732142) obtained in step 3. The reaction was complete after 12 hours. The reaction solution was diluted with 30 mL of dichloromethane. The resulting mixture was washed with saturated saline. The organic phases were separated and concentrated under reduced pressure to remove the solvent. The residue was subjected to column chromatography (DCM/MeOH = 1/0 - 10/1) for separation to give the Boc-protected product.

To a solution of the Boc-protected product in dichloromethane was added trifluoroacetic acid (10 equiv). The reaction mixture was allowed to react at room temperature for 30 minutes. After the reaction was complete, the mixture was concentrated under reduced pressure to remove the organic solvent and trifluoroacetic acid. The residue was lyophilized to give the cis-product (732149) and trans-product (732150) as powder form.
the cis-product (732149): 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((cis)-4-((R)-3-methylpiperazin-1-yl)cyclohexyl)methyl)amino)-3-nitrophenyl)sulfonyl)benzamide (732149)
¹H NMR (500 MHz, Methanol-*d*₄) δ 8.66 (d, *J* = 2.3 Hz, 1H), 8.06 - 7.96 (m, 1H), 7.86 (dd, *J* = 9.2, 2.3 Hz, 1H), 7.62 (d, *J* = 8.9 Hz, 1H), 7.57 (d, *J* = 2.5 Hz, 1H), 7.47 (d, *J* = 3.4 Hz, 1H), 7.35 (d, *J* = 8.4 Hz, 2H), 7.08 (d, *J* = 8.4 Hz, 2H), 7.01 (d, *J* = 9.4 Hz, 1H), 6.76 (dd, *J* = 8.9, 2.4 Hz, 1H), 6.43 (d, *J =* 3.5 Hz, 1H), 6.34 (d, *J =* 2.3 Hz, 1H), 3.99 - 3.84 (m, 3H), 3.80 - 3.60 (d, *J =* 29.2 Hz, 6H), 3.50 - 3.35 (m, 6H), 3.27 - 3.09 (m, 3H), 2.79 (s, 2H), 2.33 - 2.26 (m, 2H), 2.10 (s, 3H), 1.96 (dt, *J* = 31.7, 9.6 Hz, 7H), 1.72 (ddd, *J* = 15.0, 10.4, 4.7 Hz, 2H), 1.56 (t, *J* = 6.4 Hz, 2H), 1.45 (d, *J* = 6.5 Hz, 3H), 1.00 (s, 6H).
the trans-product (732150): 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-((R)-3-methylpiperazin-1-yl)cyclohexyl)methyl)amino)-3-nitrophenyl)sulfonyl)benzamide (732150)
¹H NMR (500 MHz, Methanol-*d*₄) δ 8.66 (d, *J* = 2.3 Hz, 1H), 8.00 (s, 1H), 7.85 (dd, *J* = 9.3, 2.3 Hz, 1H), 7.62 (d, *J* = 8.9 Hz, 1H), 7.57 (d, *J =* 2.5 Hz, 1H), 7.47 (d, *J =* 3.4 Hz, 1H), 7.35 (d, *J* = 8.4 Hz, 2H), 7.08 (d, *J* = 8.4 Hz, 2H), 6.95 (d, *J* = 9.3 Hz, 1H), 6.76 (dd, *J* = 9.0, 2.4 Hz, 1H), 6.43 (d, *J* = 3.4 Hz, 1H), 6.33 (d, *J =* 2.4 Hz, 1H), 3.90 - 3.78 (m, 3H), 3.77 - 3.57 (m, 7H), 3.51 - 3.35 (m, 5H), 3.28 (d, *J =* 6.7 Hz, 3H), 3.17 (d, *J =* 15.8 Hz, 2H), 2.79 (s, 2H), 2.33 - 2.24 (m, 4H), 2.10 (s, 2H), 2.06 (d, *J* = 12.0 Hz, 2H), 1.81 - 1.71 (m, 1H), 1.70 - 1.59 (m, 2H), 1.56 (t, *J* = 6.4 Hz, 2H), 1.45 (d, *J* = 6.5 Hz, 3H), 1.25 (ddt, *J* = 16.2, 12.9, 6.2 Hz, 2H), 1.00 (s, 6H).

### Intermediate Example 5: preparation of 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-(piperazin-1-yl)cyclohexyl)methyl)amino)-3-((trifluoromethyl)sulfonyl)phenyl)sulfonyl)benzamide (732185)

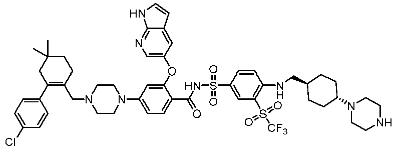

2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-(piperazin-1-yl)cyclohexyl)methyl)amino)-3-((trifluoromethyl)sulfonyl)phenyl)sulfonyl)benzamide (732185) was prepared by referring to the method of Scheme 5.

### Step 1:

To a solution of tert-butyl 4-((trans)-4-(aminomethyl)cyclohexyl)piperazine-1-carboxylate (1.0 equiv; CAS No.: 2357231-00-4) in THF were added 4-fluoro-3-((trifluoromethyl)sulfonyl)benzenesulfonamide (1.0 equiv) and triethylamine (1.5 equiv). The reaction mixture was stirred at room temperature overnight. After the reaction was complete, to the reaction mixture was added a saturated aqueous solution of sodium bicarbonate. The resulting mixture was extracted three times with dichloromethane. The dichloromethane organic phases were combined and washed with saturated saline. The organic phases were separated and concentrated under reduced pressure to remove the solvent. The residue was subjected to column chromatography (DCM/MeOH = 1/0 - 10/1) for separation to give tert-butyl 4-((trans)-4-(((4-sulfamoyl-2-((trifluoromethyl)sulfonyl)phenyl)amino)methyl)cyclohexyl)piperazine-1-carboxylate (732181) as a white powder, with a yield of 98%. ¹H NMR (500 MHz, DMSO-*d*₆) δ 8.01 (d, *J=* 2.3 Hz, 1H), 7.93 (dd, *J* = 9.2, 2.4 Hz, 1H), 7.39 (s, 2H), 7.21 (d, *J* = 9.3 Hz, 1H), 7.16 (t, *J* = 5.8 Hz, 1H), 3.23 (dd, *J* = 13.3, 6.9 Hz, 6H), 2.41 (t, *J* = 5.2 Hz, 4H), 2.22 (t, *J* = 12.0 Hz, 1H), 1.85 - 1.70 (m, 4H), 1.60 - 1.47 (m, 1H), 1.38 (s, 9H), 1.19 (q, *J* = 12.2 Hz, 2H), 1.04 - 0.93 (m, 2H).

### Step 2:

To a solution of 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)benzoic acid (1 equiv; CAS No.: 1235865-77-6) in dichloromethane were added EDCI (1.5 equiv) and DMAP (1.0 equiv). The reaction system becomes clear. To the reaction solution was added the product tert-butyl 4-((trans)-4-(((4-sulfamoyl-2-((trifluoromethyl)sulfonyl)phenyl)amino)methyl)cyclohexyl)piperazine-1-carboxylate (732181) obtained in step 1. The reaction was complete after 12 hours. The reaction solution was diluted with 30 mL of dichloromethane. The resulting mixture was washed with saturated saline. The organic phases were separated and concentrated under reduced pressure to remove the solvent. The residue was subjected to column chromatography (DCM/MeOH = 1/0 - 10/1) for separation to give the Boc-protected product.

To a solution of the Boc-protected product in dichloromethane was added trifluoroacetic acid (10 equiv). The reaction mixture was allowed to react at room temperature for 30 minutes. After the reaction was complete, the mixture was concentrated under reduced pressure to remove the organic solvent and trifluoroacetic acid. The residue was lyophilized to give trifluoroacetate of the target compound (732185) as powder. ¹H NMR (500 MHz, Methanol-*d*₄) δ 8.30 (d, *J* = 2.3 Hz, 1H), 8.03 (d, *J=* 2.6 Hz, 1H), 7.97 (dd, *J* = 9.3, 2.3 Hz, 1H), 7.65 - 7.60 (m, 2H), 7.49 (d, *J* = 3.5 Hz, 1H), 7.35 (d, *J* = 8.2 Hz, 2H), 7.07 (d, *J* = 8.4 Hz, 2H), 6.89 (d, *J =* 9.4 Hz, 1H), 6.75 (d, *J* = 8.9 Hz, 1H), 6.47 (d, *J* = 3.5 Hz, 1H), 6.30 (d, *J* = 2.4 Hz, 1H), 3.66 (s, 3H), 3.59 (s, 10H), 3.20 (d, *J* = 6.8 Hz, 3H), 3.06 (s, 2H), 2.96 - 2.66 (m, 2H), 2.25 (tt, *J =* 6.4, 2.4 Hz, 3H), 2.20 (d, *J =* 10.8 Hz, 2H), 2.11 (d, *J* = 8.2 Hz, 3H), 2.01 (d, *J* = 11.8 Hz, 2H), 1.69 (tdd, *J* = 11.2, 7.7, 4.8 Hz, 1H), 1.64 - 1.52 (m, 5H), 1.25 - 1.15 (m, 2H), 0.99 (s, 6H). HRMS (ESI) m/z: calcd for C₅₁H₆₁ClF₃N₈O₆S₂⁺ [M+H]⁺, 1037.3791; found, 1037.3792.

### Intermediate Example 6: preparation of 4-(4-((4'-chloro-4,4-dimethyl-3,4,5,6-tetrahydro-[1,1'-biphenyl]-2-yl)methyl)piperazin-1-yl)-N-((4-((((trans)-4-(piperazin-1-yl)cyclohexyl)methyl)amino)-3-((trifluoromethyl)sulfonyl)phenyl)sulfonyl)benzamide (732186)

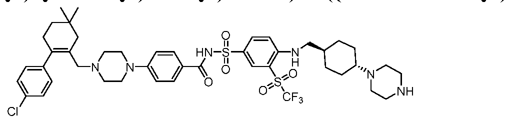

4-(4-((4'-chloro-4,4-dimethyl-3,4,5,6-tetrahydro-[1,1'-biphenyl]-2-yl)methyl)piperazin-1-yl)-N-((4-((((trans)-4-(piperazin-1 -yl)cyclohexyl)methyl)amino)-3 - ((trifluoromethyl)sulfonyl)phenyl)sulfonyl)benzamide (732186) was prepared by referring to the method of step 2 of Scheme 5.

To a solution of 4-(4-((4'-chloro-4,4-dimethyl-3,4,5,6-tetrahydro-[1,1'-biphenyl]-2-yl)methyl)piperazin-1-yl)benzoic acid (1 equiv; CAS No.: 1044598-91-5) in dichloromethane were added EDCI (1.5 equiv) and DMAP (1.0 equiv). The reaction system becomes clear. To the reaction solution was added the product 732181 obtained in step 1 of the intermediate example 7. The reaction was complete after 12 hours. The reaction solution was diluted with 30 mL of dichloromethane. The resulting mixture was washed with saturated saline. The organic phases were separated and concentrated under reduced pressure to remove the solvent. The residue was subjected to column chromatography (DCM/MeOH = 1/0 - 10/1) for separation to give the Boc-protected product.

To a solution of the Boc-protected product in dichloromethane was added trifluoroacetic acid (10 equiv). The reaction mixture was allowed to react at room temperature for 30 minutes. After the reaction was complete, the mixture was concentrated under reduced pressure to remove the organic solvent and trifluoroacetic acid. The residue was lyophilized to give trifluoroacetate of the target product (732186) as powder.
¹H NMR (500 MHz, DMSO-*d*₆) δ 8.05 (d, *J* = 2.1 Hz, 1H), 8.01 (dd, *J* = 9.0, 2.2 Hz, 1H), 7.71 (d, *J =* 8.7 Hz, 2H), 7.38 (d, *J* = 8.5 Hz, 2H), 7.13 (d, *J* = 8.4 Hz, 2H), 6.99 (d, *J* = 9.2 Hz, 1H), 6.85 (t, *J* = 5.7 Hz, 1H), 6.77 (d, *J =* 8.8 Hz, 2H), 3.16 (t, *J* = 6.3 Hz, 3H), 3.12 (t, *J =* 5.0 Hz, 4H), 2.97 - 2.91 (m, 4H), 2.74 (s, 2H), 2.62 (t, *J* = 5.0 Hz, 4H), 2.24 (dt, *J* = 20.9, 5.4 Hz, 7H), 1.99 (s, 2H), 1.78 (dt, *J* = 9.1, 4.3 Hz, 4H), 1.58 - 1.47 (m, 1H), 1.43 (t, *J* = 6.5 Hz, 2H), 1.26 - 1.15 (m, 3H), 1.03 - 0.99 (m, 1H), 0.97 (s, 6H). HRMS (ESI) m/z: calcd for C₄₄H₅₇ClF₃N₆O₅S₂⁺ [M+H]⁺, 905.3467; found, 905.3467.

### Intermediate Example 7: preparation of 4-(4-((4'-chloro-4,4-dimethyl-3,4,5,6-tetrahydro-[1,1'-biphenyl]-2-yl)methyl)piperazin-1-yl)-N-((3-nitro-4-((((trans)-4-(piperazin-1-yl)cyclohexyl)methyl)amino)phenyl)sulfonyl)benzamide (732187)

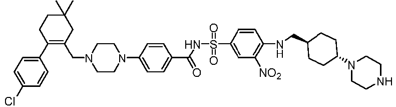

4-(4-((4'-chloro-4,4-dimethyl-3,4,5,6-tetrahydro-[1,1'-biphenyl]-2-yl)methyl)piperazin-1-yl)-N-((3-nitro-4-((((trans)-4-(piperazin-1-yl)cyclohexyl)methyl)amino)phenyl)sulfonyl)benzamide (732187) was prepared by referring to the method of Scheme 6.

### Step 1:

To a solution of tert-butyl 4-((trans)-4-(aminomethyl)cyclohexyl)piperazine-1-carboxylate (1.0 equiv; CAS No.: 2357231-00-4) in THF were added 4-fluoro-3-nitrobenzenesulfonamide (1.0 equiv) and triethylamine (1.5 equiv). The reaction mixture was stirred at room temperature overnight. After the reaction was complete, to the reaction mixture was added a saturated aqueous solution of sodium bicarbonate. The resulting mixture was extracted three times with dichloromethane. The dichloromethane organic phases were combined and washed with saturated saline. The organic phases were separated and concentrated under reduced pressure to remove the solvent. The residue was subjected to column chromatography (DCM/MeOH = 1/0 - 10/1) for separation to give tert-butyl 4-((trans)-4-(((2-nitro-4-sulfamoylphenyl)amino)methyl)cyclohexyl)piperazine-1-carboxylate (732170) as a white powder, with a yield of 95%. ¹H NMR (500 MHz, DMSO-*d*₆) δ 8.56 (t, *J* = 6.0 Hz, 1H), 8.47 (d, *J* = 2.3 Hz, 1H), 7.81 (dd, *J* = 9.1, 2.3 Hz, 1H), 7.33 (s, 2H), 7.26 (d, *J* = 9.3 Hz, 1H), 3.27 (dt, *J* = 14.8, 5.7 Hz, 6H), 2.41 (t, *J* = 5.1 Hz, 4H), 2.25 (t, *J* = 11.6 Hz, 1H), 1.88 - 1.74 (m, 4H), 1.57 (dt, *J* = 7.7, 3.5 Hz, 1H), 1.38 (s, 9H), 1.25 - 1.13 (m, 2H), 1.09 - 0.87 (m, 2H).

### Step 2:

To a solution of 4-(4-((4'-chloro-4,4-dimethyl-3,4,5,6-tetrahydro-[1,1'-biphenyl]-2-yl)methyl)piperazin-1-yl)benzoic acid (1 equiv; CAS No.: 1044598-91-5) in dichloromethane were added EDCI (1.5 equiv) and DMAP (1.0 equiv). The reaction system becomes clear. To the reaction solution was added the product 732170 obtained in step 1. The reaction was complete after 12 hours. The reaction solution was diluted with 30 mL of dichloromethane. The resulting mixture was washed with saturated saline. The organic phases were separated and concentrated under reduced pressure to remove the solvent. The residue was subjected to column chromatography (DCM/MeOH = 1/0 - 10/1) for separation to give the Boc-protected product.

To a solution of the Boc-protected product in dichloromethane was added trifluoroacetic acid (10 equiv). The reaction mixture was allowed to react at room temperature for 30 minutes. After the reaction was complete, the mixture was concentrated under reduced pressure to remove the organic solvent and trifluoroacetic acid. The residue was lyophilized to give trifluoroacetate of the target product (732187) as powder. ¹H NMR (500 MHz, Methanol-*d*₄) δ 8.82 (d, *J* = 2.3 Hz, 1H), 8.03 (dd, *J* = 9.3, 2.3 Hz, 1H), 7.72 (d, *J =* 9.1 Hz, 2H), 7.38 (d, *J* = 8.4 Hz, 2H), 7.16 (t, *J* = 8.5 Hz, 3H), 6.95 (d, *J =* 9.1 Hz, 2H), 3.89 (s, 1H), 3.70 (s, 2H), 3.59 (s, 10H), 3.34 (dd, *J* = 7.5, 5.5 Hz, 3H), 3.22 (s, 2H), 2.89 (s, 2H), 2.40 (t, *J =* 6.5 Hz, 2H), 2.20 (d, *J =* 10.8 Hz, 2H), 2.11 - 2.04 (m, 4H), 1.78 (dqd, *J =* 14.5, 8.4, 6.8, 3.4 Hz, 1H), 1.64 - 1.55 (m, 4H), 1.31 - 1.18 (m, 3H), 1.06 (s, 6H). HRMS (ESI) m/z: calcd for C₄₃H₅₇ClN₇O₅S⁺ [M+H]⁺, 818.3825; found, 818.3822.

### Example 1: preparation of 2-((1H-pyrrolo[2,3-b]pyridin-5-yl)oxy)-N-((4-((((trans)-4-(4-((7-bromo-2-(2,6-dioxopiperidin-3-yl)-3-oxoisoindolin-5-yl)methyl)piperazin-1-yl)cyclohexyl)methyl)amino)-3-nitrophenyl)sulfonyl)-4-(4-((4'-chloro-5,5-dimethyl-3,4,5,6-tetrahydro-[1,1'-biphenyl]-2-yl)methyl)piperazin-1-yl)benzamide (BCL-03146)

Referring to the method of Scheme 7, the target compound (BCL-03146) was prepared using the intermediate compound 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((3-nitro-4-((((trans)-4-(piperazin-1-yl)cyclohexyl)methyl)amino)phenyl)sulfonyl)benzamide (SIAIS364025; which can be prepared by referring to the Preparation Example 11 of intermediate BINDER in Chinese Patent Application Publication No. CN112707900 A, the content of which is incorporated by reference in its entirety) and the compound (732001) prepared according to the intermediate Example 1 as raw materials. ¹H NMR (500 MHz, DMSO-*d*₆) δ 11.78 (s, 1H), 11.71 (s, 1H), 11.05 (s, 1H), 10.78 (s, 1H), 8.64 (t, *J =* 6.0 Hz, 1H), 8.57 (d, *J* = 2.7 Hz, 1H), 8.22 (s, 1H), 8.07 (s, 1H), 8.05 (d, *J* = 2.9 Hz, 1H), 7.81 (dd, *J* = 9.2, 2.6 Hz, 1H), 7.57 (d, *J* = 3.1 Hz, 1H), 7.56 - 7.49 (m, 2H), 7.39 (d, *J* = 8.2 Hz, 2H), 7.15 - 7.06 (m, 3H), 6.72 (dd, *J =* 9.1, 2.4 Hz, 1H), 6.46 - 6.37 (m, 1H), 6.26 (d, *J =* 2.3 Hz, 1H), 5.16 (dd, *J =* 13.3, 5.2 Hz, 1H), 4.46 (d, *J* = 18.0 Hz, 3H), 4.31 (d, *J* = 17.9 Hz, 1H), 3.72 - 3.53 (m, 8H), 3.28 (dd, *J* = 12.3, 6.5 Hz, 9H), 2.92 (ddd, *J* = 17.9, 13.5, 5.6 Hz, 1H), 2.75 - 2.57 (m, 3H), 2.41 - 2.33 (m, 2H), 2.19 - 2.09 (m, 2H), 2.05 - 1.97 (m, 3H), 1.95 - 1.85 (m, 2H), 1.69 - 1.59 (m, 1H), 1.57 - 1.39 (m, 5H), 1.32 - 1.21 (m, 2H), 1.14 - 1.03 (m, 2H), 0.94 (s, 6H). HRMS (ESI) m/z: calcd for C₆₄H₇₂BrClN₁₁O₉S⁺ [M+H]⁺, 1284.4102; found, 1284.4100.

### Example 2: preparation of 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-(4-((2-(2,6-dioxopiperidin-3-yl)-1-oxoisoindolin-5-yl)methyl)piperazin-1-yl)cyclohexyl)methyl)amino)-3-nitrophenyl)sulfonyl)benzamide (BCL-03147)

Referring to the method of Scheme 7, the target compound (BCL-03147) was prepared using the intermediate compound 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((3-nitro-4-((((trans)-4-(piperazin-1-yl)cyclohexyl)methyl)amino)phenyl)sulfonyl)benzamide (SIAIS364025) and the compound (732180) prepared according to the intermediate Example 2 as raw materials. ¹H NMR (500 MHz, DMSO-*d*₆) δ 11.77 (s, 1H), 11.71 (s, 1H), 11.03 (s, 1H), 10.67 (s, 1H), 8.64 (t, *J* = 6.1 Hz, 1H), 8.56 (d, *J* = 2.0 Hz, 1H), 8.05 (d, *J* = 3.1 Hz, 1H), 7.90 (s, 1H), 7.80 (t, *J =* 5.8 Hz, 3H), 7.57 (d, *J =* 2.9 Hz, 1H), 7.55 - 7.48 (m, 2H), 7.39 (d, *J=* 8.2 Hz, 2H), 7.14 - 7.07 (m, 3H), 6.72 (d, *J=* 9.2 Hz, 1H), 6.45 - 6.40 (m, 1H), 6.26 (s, 1H), 5.14 (dd, *J* = 13.3, 5.2 Hz, 1H), 4.50 (d, *J* = 17.7 Hz, 3H), 4.37 (d, *J* = 17.7 Hz, 1H), 3.70 - 3.52 (m, 11H), 3.26 (d, *J =* 10.1 Hz, 8H), 2.92 (td, *J =* 13.0, 6.6 Hz, 1H), 2.76 - 2.57 (m, 3H), 2.43 (dt, *J* = 13.3, 6.6 Hz, 1H), 2.35 (s, 2H), 2.18 - 2.08 (m, 2H), 2.02 (d, *J* = 9.3 Hz, 3H), 1.90 (d, *J* = 11.7 Hz, 2H), 1.63 (t, *J =* 11.3 Hz, 1H), 1.49 (dd, *J =* 12.3, 3.1 Hz, 2H), 1.44 (t, *J =* 6.5 Hz, 2H), 1.08 (q, *J* = 12.1 Hz, 2H), 0.94 (s, 6H). HRMS (ESI) m/z: calcd for C₆₄H₇₃ClN₁₁O₉S⁺ [M+H]⁺, 1206.4996; found, 1206.4990.

### Example 3: preparation of 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((cis)-4-(4-((2-(2,6-dioxopiperidin-3-yl)-1-oxoisoindolin-5-yl)methyl)piperazin-1-yl)cyclohexyl)methyl)amino)-3-nitrophenyl)sulfonyl)benzamide (BCL-03148)

Referring to the method of Scheme 7, the target compound (BCL-03148) was prepared using the intermediate compound 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((3-nitro-4-((((cis)-4-(piperazin-1-yl)cyclohexyl)methyl)amino)phenyl)sulfonyl)benzamide (SIAIS364043; which can be prepared by referring to the Preparation Example 12 of intermediate BINDER in Chinese Patent Application Publication No. CN112707900 A, the content of which is incorporated by reference in its entirety) and the intermediate compound (732180) as raw materials. ¹H NMR (500 MHz, DMSO-*d*₆) δ 11.75 (s, 1H), 11.70 (s, 1H), 11.02 (s, 1H), 8.63 (t, *J* = 6.0 Hz, 1H), 8.57 (d, *J* = 2.4 Hz, 1H), 8.05 (d, *J =* 2.6 Hz, 1H), 7.91 - 7.69 (m, 4H), 7.56 (d, *J* = 2.7 Hz, 1H), 7.54 - 7.49 (m, 2H), 7.39 (d, *J* = 8.5 Hz, 2H), 7.15 (d, *J* = 9.6 Hz, 1H), 7.09 (d, *J* = 8.4 Hz, 2H), 6.72 (dd, *J* = 9.0, 2.4 Hz, 1H), 6.44 - 6.37 (m, 1H), 6.25 (d, *J =* 2.3 Hz, 1H), 5.14 (dd, *J =* 13.3, 5.2 Hz, 1H), 4.55 - 4.29 (m, 4H), 3.63 (d, *J =* 11.3 Hz, 5H), 3.54 (s, 3H), 3.30 - 3.16 (m, 6H), 2.93 (ddd, *J =* 17.5, 13.7, 5.6 Hz, 1H), 2.70 (d, *J =* 10.7 Hz, 2H), 2.62 (d, *J* = 17.4 Hz, 1H), 2.43 (qd, *J* = 12.7, 4.3 Hz, 1H), 2.28 (s, 2H), 2.05 - 1.97 (m, 4H), 1.85 (s, 2H), 1.78 (d, *J =* 12.7 Hz, 4H), 1.54 (d, *J =* 13.0 Hz, 2H), 1.44 (t, *J =* 6.4 Hz, 2H), 0.94 (s, 6H). HRMS (ESI) m/z: calcd for C₆₄H₇₃ClN₁₁O₉S⁺ [M+H]⁺, 1206.4996; found, 1206.4992.

### Example 4: preparation of 2-((1H-pyrrolo[2,3-b]pyridin-5-yl)oxy)-N-((4-((((cis)-4-(4-((7-bromo-2-(2,6-dioxopiperidin-3-yl)-3-oxoisoindolin-5-yl)methyl)piperazin-1-yl)cyclohexyl)methyl)amino)-3-nitrophenyl)sulfonyl)-4-(4-((4'-chloro-5,5-dimethyl-3,4,5,6-tetrahydro-[1,1'-biphenyl]-2-yl)methyl)piperazin-1-yl)benzamide (BCL-03149)

Referring to the method of Scheme 7, the target compound (BCL-03149) was prepared using the compound (SIAIS364043) and the compound (732001) prepared according to the intermediate Example 1 as raw materials. ¹H NMR (500 MHz, DMSO-*d*₆) δ 11.75 (s, 1H), 11.70 (s, 1H), 11.04 (s, 1H), 10.47 (s, 1H), 8.63 (t, *J* = 6.0 Hz, 1H), 8.57 (d, *J* = 2.3 Hz, 1H), 8.15 (s, 1H), 8.05 (d, *J* = 2.6 Hz, 1H), 8.02 (s, 1H), 7.82 (dd, *J* = 9.2, 2.4 Hz, 1H), 7.56 (d, *J* = 2.7 Hz, 1H), 7.54 - 7.49 (m, 2H), 7.42 - 7.38 (m, 2H), 7.15 (d, *J* = 9.6 Hz, 1H), 7.09 (d, *J* = 8.4 Hz, 2H), 6.72 (dd, *J* = 9.1, 2.5 Hz, 1H), 6.42 - 6.38 (m, 1H), 6.25 (d, *J =* 2.4 Hz, 1H), 5.16 (dd, *J =* 13.2, 5.1 Hz, 1H), 4.38 (dd, *J =* 78.7, 17.9 Hz, 4H), 3.63 (d, *J =* 13.9 Hz, 5H), 3.53 (s, 4H), 3.31 - 3.09 (m, 9H), 2.92 (ddd, *J =* 17.4, 13.6, 5.4 Hz, 1H), 2.69 (q, *J =* 13.3, 12.1 Hz, 2H), 2.61 (d, *J =* 17.1 Hz, 1H), 2.46 (dd, *J =* 13.0, 4.8 Hz, 1H), 2.32 (s, 2H), 2.07 - 1.96 (m, 4H), 1.85 (s, 2H), 1.78 (d, *J* = 11.1 Hz, 4H), 1.58 - 1.48 (m, 2H), 1.44 (t, *J* = 6.4 Hz, 2H), 0.94 (s, 6H). HRMS (ESI) m/z: calcd for C₆₄H₇₂BrClN₁₁O₉S⁺ [M+H]⁺, 1284.4102; found, 1284.4105.

### Example 5: preparation of 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((cis)-4-((3S)-4-((2-(2,6-dioxopiperidin-3-yl)-1-oxoisoindolin-5-yl)methyl)-3-methylpiperazin-1-yl)cyclohexyl)methyl)amino)-3-nitrophenyl)sulfonyl)benzamide (BCL-03165)

Referring to the method of Scheme 7, the target compound (BCL-03165) was prepared using the compound (732147) prepared according to the intermediate Example 3 and the compound (732180) prepared according to the intermediate Example 2 as raw materials. ¹H NMR (500 MHz, DMSO-*d*₆) δ 11.74 (s, 1H), 11.70 (s, 1H), 11.02 (s, 1H), 10.32 (s, 1H), 8.63 (t, *J =* 6.0 Hz, 1H), 8.57 (d, *J =* 2.3 Hz, 1H), 8.05 (s, 1H), 7.85 - 7.78 (m, 3H), 7.56 (d, *J =* 2.6 Hz, 1H), 7.53 - 7.50 (m, 2H), 7.39 (d, *J* = 8.5 Hz, 2H), 7.15 (d, *J* = 9.5 Hz, 1H), 7.09 (d, *J* = 8.4 Hz, 2H), 6.72 (dd, *J* = 9.1, 2.4 Hz, 1H), 6.41 (dd, *J* = 3.6, 1.9 Hz, 1H), 6.25 (d, *J* = 2.4 Hz, 1H), 5.14 (dd, *J =* 13.3, 5.2 Hz, 1H), 4.49 (t, *J* = 17.1 Hz, 1H), 4.36 (t, *J* = 17.5 Hz, 1H), 3.63 (d, *J* = 13.4 Hz, 4H), 3.56 (d, *J* = 18.2 Hz, 4H), 3.30 - 3.15 (m, 8H), 2.98 - 2.88 (m, 1H), 2.70 (d, *J =* 10.5 Hz, 2H), 2.61 (d, *J* = 17.1 Hz, 2H), 2.43 (dd, *J* = 13.3, 4.9 Hz, 1H), 2.31 (t, *J* = 6.5 Hz, 2H), 2.01 (d, *J* = 2.3 Hz, 5H), 1.90 - 1.73 (m, 7H), 1.59 - 1.47 (m, 4H), 1.47 - 1.42 (m, 3H), 0.95 (s, 6H). HRMS (ESI) m/z: calcd for C₆₅H₇₅ClN₁₁O₉S⁺ [M+H]⁺, 1220.5153; found, 1220.5150.

### Example 6: preparation of 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-((3S)-4-((2-(2,6-dioxopiperidin-3-yl)-1-oxoisoindolin-5-yl)methyl)-3-methylpiperazin-1-yl)cyclohexyl)methyl)amino)-3-nitrophenyl)sulfonyl)benzamide (BCL-03166)

Referring to the method of Scheme 7, the target compound (BCL-03166) was prepared using the compound (732148) prepared according to the intermediate Example 3 and the intermediate compound (732180) as raw materials. ¹H NMR (500 MHz, DMSO-*d*₆) δ 11.74 (s, 1H), 11.69 (s, 1H), 11.02 (s, 1H), 10.32 (s, 1H), 8.63 (t, *J =* 6.0 Hz, 1H), 8.56 (d, *J =* 2.6 Hz, 1H), 8.04 (d, *J =* 2.9 Hz, 1H), 7.83 - 7.77 (m, 2H), 7.69 (s, 1H), 7.55 (d, *J* = 2.9 Hz, 1H), 7.54 - 7.50 (m, 2H), 7.39 (d, *J=* 8.4 Hz, 2H), 7.10 (dd, *J* = 8.7, 5.5 Hz, 3H), 6.72 (dd, *J =* 9.1, 2.4 Hz, 1H), 6.43 - 6.37 (m, 1H), 6.25 (d, *J* = 2.4 Hz, 1H), 5.13 (dd, *J* = 13.4, 5.2 Hz, 1H), 4.55 - 4.43 (m, 1H), 4.35 (t, *J* = 16.3 Hz, 1H), 3.63 (d, *J=* 12.1 Hz, 3H), 3.54 (s, 3H), 3.30 - 3.10 (m, 11H), 2.92 (ddd, *J=* 18.0, 13.6, 5.6 Hz, 2H), 2.70 (d, *J* = 10.2 Hz, 2H), 2.61 (d, *J =* 17.3 Hz, 2H), 2.47 - 2.35 (m, 2H), 2.31 (s, 2H), 2.19 - 2.08 (m, 2H), 2.01 (s, 3H), 1.90 (d, *J =* 12.9 Hz, 2H), 1.62 (s, 1H), 1.54 - 1.40 (m, 6H), 1.07 (d, *J =* 11.4 Hz, 2H), 0.94 (s, 6H). HRMS (ESI) m/z: calcd for C₆₅H₇₅ClN₁₁O₉S⁺ [M+H]⁺, 1220.5153; found, 1220.5155.

### Example 7: preparation of 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((cis)-4-((3R)-4-((2-(2,6-dioxopiperidin-3-yl)-1-oxoisoindolin-5-yl)methyl)-3-methylpiperazin-1-yl)cyclohexyl)methyl)amino)-3-nitrophenyl)sulfonyl)benzamide (BCL-03167)

Referring to the method of Scheme 7, the target compound (BCL-03167) was prepared using the compound (732149) prepared according to the intermediate Example 4 and the intermediate compound (732180) as raw materials. ¹H NMR (500 MHz, DMSO-*d*₆) δ 11.74 (s, 1H), 11.70 (s, 1H), 11.02 (s, 1H), 10.32 (s, 1H), 8.63 (t, *J =* 6.0 Hz, 1H), 8.57 (d, *J =* 2.3 Hz, 1H), 8.05 (s, 1H), 7.85 - 7.78 (m, 3H), 7.56 (d, *J* = 2.6 Hz, 1H), 7.53 - 7.50 (m, 2H), 7.39 (d, *J* = 8.5 Hz, 2H), 7.15 (d, *J =* 9.5 Hz, 1H), 7.09 (d, *J=* 8.4 Hz, 2H), 6.72 (dd, *J=* 9.1, 2.4 Hz, 1H), 6.41 (dd, *J* = 3.6, 1.9 Hz, 1H), 6.25 (d, *J =* 2.4 Hz, 1H), 5.14 (dd, *J =* 13.3, 5.2 Hz, 1H), 4.49 (t, *J =* 17.1 Hz, 1H), 4.36 (t, *J =* 17.5 Hz, 1H), 3.63 (d, *J =* 13.4 Hz, 4H), 3.56 (d, *J =* 18.2 Hz, 4H), 3.30 - 3.15 (m, 8H), 2.98 - 2.88 (m, 1H), 2.70 (d, *J =* 10.5 Hz, 2H), 2.61 (d, *J =* 17.1 Hz, 2H), 2.43 (dd, *J =* 13.3, 4.9 Hz, 1H), 2.31 (t, *J* = 6.5 Hz, 2H), 2.01 (d, *J =* 2.3 Hz, 5H), 1.90 - 1.73 (m, 7H), 1.59 - 1.47 (m, 4H), 1.47 - 1.42 (m, 3H), 0.95 (s, 6H). HRMS (ESI) m/z: calcd for C₆₅H₇₅ClN₁₁O₉S⁺ [M+H]⁺, 1220.5153; found, 1220.5155.

### Example 8: preparation of 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-((3R)-4-((2-(2,6-dioxopiperidin-3-yl)-1-oxoisoindolin-5-yl)methyl)-3-methylpiperazin-1-yl)cyclohexyl)methyl)amino)-3-nitrophenyl)sulfonyl)benzamide (BCL-03168)

Referring to the method of Scheme 7, the target compound (BCL-03168) was prepared using the compound (732150) prepared according to the intermediate Example 4 and the intermediate compound (732180) as raw materials. ¹H NMR (500 MHz, DMSO-*d*₆) δ 11.74 (s, 1H), 11.69 (s, 1H), 11.02 (s, 1H), 10.32 (s, 1H), 8.63 (t, *J =* 6.0 Hz, 1H), 8.56 (d, *J =* 2.6 Hz, 1H), 8.04 (d, *J =* 2.9 Hz, 1H), 7.83 - 7.77 (m, 2H), 7.69 (s, 1H), 7.55 (d, *J =* 2.9 Hz, 1H), 7.54 - 7.50 (m, 2H), 7.39 (d, *J =* 8.4 Hz, 2H), 7.10 (dd, *J =* 8.7, 5.5 Hz, 3H), 6.72 (dd, *J* = 9.1, 2.4 Hz, 1H), 6.43 - 6.37 (m, 1H), 6.25 (d, *J* = 2.4 Hz, 1H), 5.13 (dd, *J =* 13.4, 5.2 Hz, 1H), 4.55 - 4.43 (m, 1H), 4.35 (t, *J* = 16.3 Hz, 1H), 3.63 (d, *J =* 12.1 Hz, 3H), 3.54 (s, 3H), 3.30 - 3.10 (m, 11H), 2.92 (ddd, *J =* 18.0, 13.6, 5.6 Hz, 2H), 2.70 (d, *J* = 10.2 Hz, 2H), 2.61 (d, *J =* 17.3 Hz, 2H), 2.47 - 2.35 (m, 2H), 2.31 (s, 2H), 2.19 - 2.08 (m, 2H), 2.01 (s, 3H), 1.90 (d, *J =* 12.9 Hz, 2H), 1.62 (s, 1H), 1.54 - 1.40 (m, 6H), 1.07 (d, *J =* 11.4 Hz, 2H), 0.94 (s, 6H). HRMS (ESI) m/z: calcd for C₆₅H₇₅ClN₁₁O₉S⁺ [M+H]⁺, 1220.5153; found, 1220.5154.

### Example 9: preparation of 2-((1H-pyrrolo[2,3-b]pyridin-5-yl)oxy)-N-((4-((((cis)-4-((3S)-4-((7-bromo-2-(2,6-dioxopiperidin-3-yl)-3-oxoisoindolin-5-yl)methyl)-3-methylpiperazin-1-yl)cyclohexyl)methyl)amino)-3-nitrophenyl)sulfonyl)-4-(4-((4'-chloro-5,5-dimethyl-3,4,5,6-tetrahydro-[1,1'-biphenyl]-2-yl)methyl)piperazin-1-yl)benzamide (BCL-03169)

Referring to the method of Scheme 7, the target compound (BCL-03169) was prepared using the compound (732147) prepared according to the intermediate Example 3 and the intermediate compound (732001) as raw materials. ¹H NMR (500 MHz, DMSO-*d*₆) δ 11.76 (s, 1H), 11.71 (s, 1H), 11.05 (s, 1H), 10.58 (s, 1H), 8.63 (t, *J =* 6.0 Hz, 1H), 8.57 (d, *J =* 2.3 Hz, 1H), 8.15 (s, 1H), 8.01 (s, 1H), 7.56 (d, *J* = 2.7 Hz, 1H), 7.54 - 7.50 (m, 2H), 7.39 (d, *J* = 8.4 Hz, 2H), 7.15 (d, *J* = 9.6 Hz, 1H), 7.10 (d, *J* = 8.4 Hz, 2H), 6.72 (dd, *J* = 9.1, 2.5 Hz, 1H), 6.45 - 6.39 (m, 1H), 6.25 (d, *J* = 2.4 Hz, 1H), 5.16 (dd, *J* = 13.2, 5.1 Hz, 1H), 4.46 (d, *J* = 17.9 Hz, 1H), 4.30 (d, *J* = 20.0 Hz, 1H), 3.63 (d, *J* = 11.1 Hz, 4H), 3.53 (s, 5H), 3.27 (d, *J =* 13.0 Hz, 8H), 2.92 (ddd, *J =* 18.0, 13.5, 5.4 Hz, 1H), 2.75 - 2.66 (m, 2H), 2.61 (dt, *J* = 17.2, 3.4 Hz, 1H), 2.46 (dd, *J* = 13.2, 4.8 Hz, 1H), 2.34 (t, *J* = 6.6 Hz, 2H), 2.07 - 1.96 (m, 4H), 1.93 - 1.74 (m, 6H), 1.60 - 1.48 (m, 4H), 1.44 (t, *J =* 6.5 Hz, 2H), 0.94 (s, 6H). HRMS (ESI) m/z: calcd for C₆₅H₇₄BrClN₁₁O₉S⁺ [M+H]⁺, 1298.4258; found, 1298.4260.

### Example 10: preparation of 2-((1H-pyrrolo[2,3-b]pyridin-5-yl)oxy)-N-((4-((((trans)-4-((3S)-4-((7-bromo-2-(2,6-dioxopiperidin-3-yl)-3-oxoisoindolin-5-yl)methyl)-3-methylpiperazin-1-yl)cyclohexyl)methyl)amino)-3-nitrophenyl)sulfonyl)-4-(4-((4'-chloro-5,5-dimethyl-3,4,5,6-tetrahydro-[1,1'-biphenyl]-2-yl)methyl)piperazin-1-yl)benzamide (BCL-03170)

Referring to the method of Scheme 7, the target compound (BCL-03170) was prepared using the compound (732148) prepared according to the intermediate Example 3 and the intermediate compound (732001) as raw materials. ¹H NMR (500 MHz, DMSO-*d*₆) δ 11.75 (s, 1H), 11.70 (s, 1H), 11.04 (s, 1H), 10.59 (s, 1H), 8.64 (t, *J* = 6.0 Hz, 1H), 8.57 (d, *J* = 2.4 Hz, 1H), 8.13 (s, 1H), 8.05 (d, *J* = 2.7 Hz, 1H), 7.98 (s, 1H), 7.81 (dd, *J* = 9.2, 2.7 Hz, 1H), 7.56 (d, *J =* 2.9 Hz, 1H), 7.54 - 7.50 (m, 2H), 7.39 (d, *J =* 8.4 Hz, 2H), 7.10 (dd, *J =* 8.9, 3.1 Hz, 3H), 6.72 (dd, *J =* 9.1, 2.5 Hz, 1H), 6.46 - 6.39 (m, 1H), 6.26 (d, *J* = 2.4 Hz, 1H), 5.15 (dd, *J* = 13.3, 5.2 Hz, 1H), 4.70 (s, 1H), 4.45 (d, *J* = 17.9 Hz, 1H), 4.30 (d, *J* = 17.5 Hz, 1H), 3.63 (d, *J* = 12.7 Hz, 4H), 3.53 (s, 4H), 3.32 - 3.04 (m, 10H), 2.92 (ddd, *J* = 18.0, 13.4, 5.6 Hz, 1H), 2.74 - 2.66 (m, 2H), 2.65 - 2.57 (m, 1H), 2.46 (dd, *J =* 13.1, 4.9 Hz, 1H), 2.34 (t, *J* = 6.6 Hz, 2H), 2.19 - 2.08 (m, 2H), 2.06 - 1.97 (m, 3H), 1.90 (d, *J* = 12.4 Hz, 2H), 1.63 (tq, *J* = 7.9, 4.2 Hz, 1H), 1.55 - 1.39 (m, 6H), 1.07 (q, *J* = 12.5, 11.7 Hz, 2H), 0.94 (s, 6H). HRMS (ESI) m/z: calcd for C₆₅H₇₄BrClN₁₁O₉S⁺ [M+H]⁺, 1298.4258; found, 1298.4250.

### Example 11: preparation of 2-((1H-pyrrolo[2,3-b]pyridin-5-yl)oxy)-N-((4-((((cis)-4-((3R)-4-((7-bromo-2-(2,6-dioxopiperidin-3-yl)-3-oxoisoindolin-5-yl)methyl)-3-methylpiperazin-1-yl)cyclohexyl)methyl)amino)-3-nitrophenyl)sulfonyl)-4-(4-((4'-chloro-5,5-dimethyl-3,4,5,6-tetrahydro-[1,1'-biphenyl]-2-yl)methyl)piperazin-1-yl)benzamide (BCL-03171)

Referring to the method of Scheme 7, the target compound (BCL-03171) was prepared using the compound (732149) prepared according to the intermediate Example 3 and the intermediate compound (732001) as raw materials. ¹H NMR (500 MHz, DMSO-*d*₆) δ 11.76 (s, 1H), 11.71 (s, 1H), 11.05 (s, 1H), 10.58 (s, 1H), 8.63 (t, *J =* 6.0 Hz, 1H), 8.57 (d, *J =* 2.3 Hz, 1H), 8.15 (s, 1H), 8.01 (s, 1H), 7.56 (d, *J* = 2.7 Hz, 1H), 7.54 - 7.50 (m, 2H), 7.39 (d, *J* = 8.4 Hz, 2H), 7.15 (d, *J* = 9.6 Hz, 1H), 7.10 (d, *J* = 8.4 Hz, 2H), 6.72 (dd, *J* = 9.1, 2.5 Hz, 1H), 6.45 - 6.39 (m, 1H), 6.25 (d, *J* = 2.4 Hz, 1H), 5.16 (dd, *J* = 13.2, 5.1 Hz, 1H), 4.46 (d, *J* = 17.9 Hz, 1H), 4.30 (d, *J* = 20.0 Hz, 1H), 3.63 (d, *J* = 11.1 Hz, 4H), 3.53 (s, 5H), 3.27 (d, *J =* 13.0 Hz, 8H), 2.92 (ddd, *J =* 18.0, 13.5, 5.4 Hz, 1H), 2.75 - 2.66 (m, 2H), 2.61 (dt, *J* = 17.2, 3.4 Hz, 1H), 2.46 (dd, *J* = 13.2, 4.8 Hz, 1H), 2.34 (t, *J* = 6.6 Hz, 2H), 2.07 - 1.96 (m, 4H), 1.93 - 1.74 (m, 6H), 1.60 - 1.48 (m, 4H), 1.44 (t, *J =* 6.5 Hz, 2H), 0.94 (s, 6H). HRMS (ESI) m/z: calcd for C₆₅H₇₄BrClN₁₁O₉S⁺ [M+H]⁺, 1298.4258; found, 1298.4250.

### Example 12: preparation of 2-((1H-pyrrolo[2,3-b]pyridin-5-yl)oxy)-N-((4-((((trans)-4-((3R)-4-((7-bromo-2-(2,6-dioxopiperidin-3-yl)-3-oxoisoindolin-5-yl)methyl)-3-methylpiperazin-1-yl)cyclohexyl)methyl)amino)-3-nitrophenyl)sulfonyl)-4-(4-((4'-chloro-5,5-dimethyl-3,4,5,6-tetrahydro-[1,1'-biphenyl]-2-yl)methyl)piperazin-1-yl)benzamide (BCL-03172)

Referring to the method of Scheme 7, the target compound (BCL-03172) was prepared using the compound (732150) prepared according to the intermediate Example 3 and the intermediate compound (732001) as raw materials. ¹H NMR (500 MHz, DMSO-*d*₆) δ 11.75 (s, 1H), 11.70 (s, 1H), 11.04 (s, 1H), 10.59 (s, 1H), 8.64 (t, *J* = 6.0 Hz, 1H), 8.57 (d, *J* = 2.4 Hz, 1H), 8.13 (s, 1H), 8.05 (d, *J* = 2.7 Hz, 1H), 7.98 (s, 1H), 7.81 (dd, *J* = 9.2, 2.7 Hz, 1H), 7.56 (d, *J =* 2.9 Hz, 1H), 7.54 - 7.50 (m, 2H), 7.39 (d, *J =* 8.4 Hz, 2H), 7.10 (dd, *J =* 8.9, 3.1 Hz, 3H), 6.72 (dd, *J =* 9.1, 2.5 Hz, 1H), 6.46 - 6.39 (m, 1H), 6.26 (d, *J* = 2.4 Hz, 1H), 5.15 (dd, *J* = 13.3, 5.2 Hz, 1H), 4.70 (s, 1H), 4.45 (d, *J* = 17.9 Hz, 1H), 4.30 (d, *J* = 17.5 Hz, 1H), 3.63 (d, *J* = 12.7 Hz, 4H), 3.53 (s, 4H), 3.32 - 3.04 (m, 10H), 2.92 (ddd, *J* = 18.0, 13.4, 5.6 Hz, 1H), 2.74 - 2.66 (m, 2H), 2.65 - 2.57 (m, 1H), 2.46 (dd, *J =* 13.1, 4.9 Hz, 1H), 2.34 (t, *J* = 6.6 Hz, 2H), 2.19 - 2.08 (m, 2H), 2.06 - 1.97 (m, 3H), 1.90 (d, *J* = 12.4 Hz, 2H), 1.63 (tq, *J* = 7.9, 4.2 Hz, 1H), 1.55 - 1.39 (m, 6H), 1.07 (q, *J* = 12.5, 11.7 Hz, 2H), 0.94 (s, 6H). HRMS (ESI) m/z: calcd for C₆₅H₇₄BrClN₁₁O₉S⁺ [M+H]⁺, 1298.4258; found, 1298.4255.

### Example 13: preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-oxoisoindolin-5-yl)methyl)piperazin-1-yl)-1-(phenylthio)butan-2-yl)amino)-3-((trifluoromethyl)sulfonyl)phenyl)sulfonyl)benzamide (BCL-03177)

Referring to the method of Scheme 7, the target compound (BCL-03177) was prepared using the intermediate compound (R)-4-(4-((4'-chloro-4,4-dimethyl-3,4,5,6-tetrahydro-[1,1'-biphenyl]-2-yl)methyl)piperazin-1-yl)-N-((4-((1-(phenylthio)-4-(piperazin-1-yl)butan-2-yl)amino)-3-((trifluoromethyl)sulfonyl)phenyl)sulfonyl)benzamide (SIAIS360129; which can be prepared by referring to the Preparation Example 13 of intermediate BINDER in Chinese Patent Application Publication No. CN112707900 A, the content of which is incorporated by reference in its entirety) and the intermediate compound (732180) as raw materials. ¹H NMR (500 MHz, DMSO-*d*₆) δ 11.02 (s, 1H), 8.17 (d, *J =* 2.4 Hz, 1H), 7.99 (d, *J =* 8.6 Hz, 1H), 7.76 (dd, *J =* 9.1, 1.9 Hz, 3H), 7.57 (s, 2H), 7.43 - 7.39 (m, 2H), 7.32 - 7.28 (m, 2H), 7.26 - 7.21 (m, 2H), 7.20 - 7.13 (m, 4H), 6.95 (dd, *J* = 13.6, 9.0 Hz, 3H), 5.13 (dd, *J =* 13.3, 5.2 Hz, 1H), 4.46 (d, *J* = 17.5 Hz, 1H), 4.34 (d, *J* = 17.1 Hz, 1H), 4.18 (s, 1H), 3.90 - 3.62 (m, 3H), 3.50 - 3.10 (m, 14H), 2.99 - 2.85 (m, 4H), 2.73 (d, *J* = 4.6 Hz, 2H), 2.61 (dt, *J* = 17.2, 4.2 Hz, 2H), 2.48 - 2.35 (m, 2H), 2.27 (t, *J* = 6.8 Hz, 2H), 2.16 (s, 3H), 2.00 (ddt, *J =* 7.3, 5.3, 2.7 Hz, 1H), 1.46 (t, *J* = 6.5 Hz, 2H), 1.00 (s, 6H). HRMS (ESI) m/z: calcd for C₆₁H₆₉ClF₃N₈O₈S₃⁺ [M+H]⁺, 1229.4036; found, 1229.4038.

### Example 14: preparation of 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-(4-((2-(2,6-dioxopiperidin-3-yl)-1-oxoisoindolin-5-yl)methyl)piperazin-1-yl)cyclohexyl)methyl)amino)-3-((trifluoromethyl)sulfonyl)phenyl)sulfonyl)benzamide (BCL-03179)

Referring to the method of Scheme 7, the target compound (BCL-03179) was prepared using the compound (732185) prepared according to the intermediate Example 5 and the intermediate compound (732180) as raw materials. ¹H NMR (500 MHz, DMSO-*d*₆) δ 11.81 (s, 2H), 11.74 (s, 1H), 11.03 (s, 1H), 10.66 (s, 1H), 8.17 (d, *J* = 2.6 Hz, 1H), 8.07 (d, *J* = 3.1 Hz, 1H), 7.97 - 7.89 (m, 2H), 7.81 (s, 2H), 7.60 (d, *J* = 3.2 Hz, 1H), 7.55 (t, *J =* 3.3 Hz, 1H), 7.50 (d, *J* = 8.9 Hz, 1H), 7.40 (d, *J* = 8.2 Hz, 2H), 7.35 (t, *J* = 5.9 Hz, 1H), 7.10 (d, *J =* 8.2 Hz, 2H), 7.08 (d, *J =* 9.3 Hz, 1H), 6.76 - 6.70 (m, 1H), 6.47 - 6.41 (m, 1H), 6.28 - 6.21 (m, 1H), 5.15 (dd, *J =* 13.3, 5.2 Hz, 1H), 4.50 (d, *J =* 17.7 Hz, 3H), 4.37 (d, *J =* 17.7 Hz, 1H), 3.61 (s, 9H), 3.33 - 3.11 (m, 9H), 2.93 (ddd, *J =* 17.5, 13.3, 5.3 Hz, 1H), 2.71 (dd, *J =* 20.1, 7.3 Hz, 2H), 2.61 (d, *J =* 17.9 Hz, 1H), 2.45 (tt, *J =* 13.3, 6.5 Hz, 1H), 2.35 (s, 2H), 2.17 - 2.08 (m, 2H), 2.01 (s, 3H), 1.84 (d, *J=* 11.4 Hz, 2H), 1.65 - 1.55 (m, 1H), 1.54 - 1.41 (m, 4H), 1.07 (t, *J* = 11.8 Hz, 2H), 0.94 (s, 6H). HRMS (ESI) m/z: calcd for C₆₅H₇₃ClF₃N₁₀O₉S₂⁺ [M+H]⁺, 1293.4639; found, 1293.4635.

### Example 15: preparation of 4-(4-((4'-chloro-4,4-dimethyl-3,4,5,6-tetrahydro-[1,1'-biphenyl]-2-yl)methyl)piperazin-1-yl)-N-((4-((((trans)-4-(4-((2-(2,6-dioxopiperidin-3-yl)-1-oxoisoindolin-5-yl)methyl)piperazin-1-yl)cyclohexyl)methyl)amino)-3-((trifluoromethyl)sulfonyl)phenyl)sulfonyl)benzamide (BCL-03180)

Referring to the method of Scheme 7, the target compound (BCL-03180) was prepared using the compound (732186) prepared according to the intermediate Example 6 and the intermediate compound (732180) as raw materials. ¹H NMR (500 MHz, DMSO-*d*₆) δ 12.16 (s, 1H), 11.02 (s, 1H), 10.25 (s, 1H), 8.22 (s, 1H), 8.05 (dd, *J* = 9.2, 2.2 Hz, 1H), 7.77 (t, *J* = 9.6 Hz, 4H), 7.70 (s, 1H), 7.42 (d, *J =* 8.2 Hz, 3H), 7.24 (d, *J =* 9.6 Hz, 1H), 7.18 (d, *J =* 8.4 Hz, 2H), 6.96 (d, *J =* 9.0 Hz, 2H), 5.14 (dd, *J =* 13.3, 5.2 Hz, 1H), 4.48 (d, *J =* 17.7 Hz, 1H), 4.36 (d, *J =* 17.5 Hz, 2H), 3.89 (d, *J =* 13.9 Hz, 2H), 3.58 (s, 3H), 3.51 (s, 2H), 3.30 - 3.00 (m, 12H), 2.93 (ddd, *J* = 17.9, 13.4, 5.4 Hz, 1H), 2.78 (q, *J* = 14.2, 12.4 Hz, 2H), 2.62 (d, *J =* 14.4 Hz, 1H), 2.41 (ddd, *J =* 18.6, 11.7, 5.1 Hz, 2H), 2.29 (t, *J* = 6.9 Hz, 2H), 2.16 (s, 2H), 2.15 - 2.07 (m, 2H), 2.02 (q, *J =* 5.8, 5.3 Hz, 1H), 1.84 (d, *J =* 11.1 Hz, 2H), 1.60 (t, *J* = 11.3 Hz, 1H), 1.48 (p, *J* = 7.5, 6.3 Hz, 4H), 1.05 (d, *J* = 12.7 Hz, 2H), 1.01 (s, 6H). HRMS (ESI) m/z: calcd for C₅₈H₆₉ClF₃N₈O₈S₂⁺ [M+H]⁺, 1161.4315; found, 1161.4316.

### Example 16: preparation of 4-(4-((4'-chloro-4,4-dimethyl-3,4,5,6-tetrahydro-[1,1'-biphenyl]-2-yl)methyl)piperazin-1-yl)-N-((4-((((trans)-4-(4-((2-(2,6-dioxopiperidin-3-yl)-1-oxoisoindolin-5-yl)methyl)piperazin-1-yl)cyclohexyl)methyl)amino)-3-nitrophenyl)sulfonyl)benzamide (BCL-03181)

Referring to the method of Scheme 7, the target compound (BCL-03181) was prepared using the compound (732187) prepared according to the intermediate Example 7 and the intermediate compound (732180) as raw materials. ¹H NMR (500 MHz, DMSO-*d*₆) δ 12.15 (s, 1H), 11.03 (s, 1H), 10.69 (s, 1H), 8.70 (t, *J* = 6.2 Hz, 1H), 8.64 (d, *J* = 2.4 Hz, 1H), 7.94 (dd, *J* = 9.2, 2.6 Hz, 1H), 7.88 (s, 1H), 7.82 - 7.73 (m, 4H), 7.41 (d, *J* = 8.4 Hz, 2H), 7.28 (d, *J* = 9.6 Hz, 1H), 7.18 (d, *J* = 8.4 Hz, 2H), 6.95 (d, *J =* 9.3 Hz, 2H), 5.14 (dd, *J =* 13.3, 5.0 Hz, 1H), 4.54 - 4.31 (m, 4H), 3.87 (d, *J =* 13.6 Hz, 2H), 3.56 (s, 6H), 3.40 - 3.11 (m, 11H), 2.93 (ddd, *J =* 17.7, 13.6, 5.5 Hz, 1H), 2.83 - 2.69 (m, 2H), 2.66 - 2.58 (m, 1H), 2.42 (tt, *J* = 13.1, 6.5 Hz, 1H), 2.27 (t, *J* = 6.9 Hz, 2H), 2.21 (s, 2H), 2.18 - 2.08 (m, 2H), 2.02 (dq, *J* = 7.5, 3.6, 2.3 Hz, 1H), 1.95 - 1.87 (m, 2H), 1.64 (dd, *J* = 7.2, 3.6 Hz, 1H), 1.46 (q, *J* = 8.2, 6.7 Hz, 4H), 1.15 - 1.04 (m, 2H), 0.99 (s, 6H). HRMS (ESI) m/z: calcd for C₅₇H₆₉ClN₉O₈S⁺ [M+H]⁺, 1074.4673; found, 1074.4673.

### Example 17: preparation of 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-(4-((2-(2,6-dioxopiperidin-3-yl)-1,3-dioxoisoindolin-5-yl)methyl)piperazin-1-yl)cyclohexyl)methyl)amino)-3-((trifluoromethyl)sulfonyl)phenyl)sulfonyl)benzamide (BCL-03185)

Referring to the method of Scheme 7, the target compound (BCL-03185) was prepared using the compound (732185) prepared according to the intermediate Example 5 and the intermediate compound 5-(bromomethyl)-2-(2,6-dioxopiperidin-3-yl)isoindoline-1,3-dione (732195; CAS No.: 1312023-72-5) as raw materials. ¹H NMR (500 MHz, DMSO-*d*₆) δ 11.84 (s, 1H), 11.75 (s, 1H), 11.17 (s, 1H), 10.77 (s, 1H), 8.28 (s, 1H), 8.17 (d, *J =* 2.4 Hz, 1H), 8.15 (s, 1H), 8.07 (d, *J =* 2.7 Hz, 1H), 8.02 (d, *J =* 7.8 Hz, 1H), 7.93 (dd, *J =* 9.3, 2.6 Hz, 1H), 7.62 (d, *J =* 2.7 Hz, 1H), 7.55 (t, *J =* 3.1 Hz, 1H), 7.50 (d, *J =* 8.9 Hz, 1H), 7.39 (d, *J* = 8.5 Hz, 2H), 7.34 (t, *J =* 5.9 Hz, 1H), 7.10 (d, *J =* 8.5 Hz, 2H), 7.08 (d, *J =* 9.5 Hz, 1H), 6.73 (dd, *J* = 9.0, 2.4 Hz, 1H), 6.48 - 6.42 (m, 1H), 6.25 (d, *J* = 2.3 Hz, 1H), 5.19 (dd, *J =* 12.8, 5.5 Hz, 1H), 4.57 (s, 2H), 3.75 - 3.40 (m, 11H), 3.34 - 3.13 (m, 8H), 2.91 (ddd, *J =* 16.8, 13.6, 5.4 Hz, 1H), 2.75 - 2.66 (m, 2H), 2.65 - 2.53 (m, 2H), 2.36 (t, *J =* 6.6 Hz, 2H), 2.18 - 2.05 (m, 3H), 2.01 (s, 2H), 1.83 (d, *J =* 11.1 Hz, 2H), 1.59 (tt, *J* = 8.2, 4.2 Hz, 1H), 1.50 (d, *J* = 8.7 Hz, 2H), 1.44 (t, *J =* 6.4 Hz, 2H), 1.07 (t, *J =* 12.7 Hz, 2H), 0.94 (s, 6H). HRMS (ESI) m/z: calcd for C₆₅H₇₁ClF₃N₁₀O₁₀S₂⁺ [M+H]⁺, 1307.4431; found, 1307.4430.

### Example 18: preparation of 4-(4-((4'-chloro-4,4-dimethyl-3,4,5,6-tetrahydro-[1,1'-biphenyl]-2-yl)methyl)piperazin-1-yl)-N-((4-((((trans)-4-(4-((2-(2,6-dioxopiperidin-3-yl)-1,3-dioxoisoindolin-5-yl)methyl)piperazin-1-yl)cyclohexyl)methyl)amino)-3-((trifluoromethyl)sulfonyl)phenyl)sulfonyl)benzamide (BCL-03186)

Referring to the method of Scheme 7, the target compound (BCL-03186) was prepared using the compound (732186) prepared according to the intermediate Example 6 and the intermediate compound 5-(bromomethyl)-2-(2,6-dioxopiperidin-3-yl)isoindoline-1,3-dione (732195; CAS No.: 1312023-72-5) as raw materials. ¹H NMR (500 MHz, DMSO-*d*₆) δ 12.17 (s, 1H), 11.61 (s, 1H), 11.16 (s, 1H), 10.58 (s, 1H), 8.22 (d, *J =* 2.4 Hz, 1H), 8.18 (s, 1H), 8.09 (s, 1H), 8.05 (dd, *J =* 9.3, 2.6 Hz, 1H), 7.99 (d, *J* = 7.6 Hz, 1H), 7.76 (d, *J* = 9.0 Hz, 2H), 7.41 (td, *J* = 7.3, 6.8, 3.6 Hz, 3H), 7.24 (d, *J* = 9.6 Hz, 1H), 7.18 (d, *J* = 8.5 Hz, 2H), 6.96 (d, *J* = 9.3 Hz, 2H), 5.18 (dd, *J* = 12.9, 5.4 Hz, 1H), 4.36 (s, 1H), 3.88 (d, *J* = 13.3 Hz, 2H), 3.65 - 3.13 (m, 18H), 2.91 (ddd, *J* = 16.9, 13.8, 5.5 Hz, 1H), 2.76 (q, *J* = 10.0, 9.2 Hz, 2H), 2.67 - 2.58 (m, 1H), 2.57 - 2.53 (m, 1H), 2.28 (t, *J =* 6.8 Hz, 2H), 2.20 (s, 2H), 2.13 (d, *J* = 10.5 Hz, 2H), 2.11 - 2.04 (m, 1H), 1.84 (d, *J* = 11.0 Hz, 2H), 1.60 (tdq, *J* = 11.0, 7.2, 4.3, 3.7 Hz, 1H), 1.47 (q, *J =* 6.3 Hz, 4H), 1.07 (t, *J =* 12.1 Hz, 2H), 1.00 (s, 6H). HRMS (ESI) m/z: calcd for C₅₈H₆₇ClF₃N₈O₉S₂⁺ [M+H]⁺, 1175.4108; found, 1175.4101.

### Example 19: preparation of 4-(4-(4-(4-((4'-chloro-4,4-dimethyl-3,4,5,6-tetrahydro-[1,1'-biphenyl]-2-yl)methyl)piperazin-1-yl)-N-((4-((((trans)-4-(4-((2-(2,6-dioxopiperidin-3-yl)-1,3-dioxoisoindolin-5-yl)methyl)piperazin-1-yl)cyclohexyl)methyl)amino)-3-nitrophenyl)sulfonyl)benzamide (BCL-03187)

Referring to the method of Scheme 7, the target compound (BCL-03187) was prepared using the compound (732187) prepared according to the intermediate Example 7 and the intermediate compound 5-(bromomethyl)-2-(2,6-dioxopiperidin-3-yl)isoindoline-1,3-dione (732195; CAS No.: 1312023-72-5) as raw materials. ¹H NMR (500 MHz, DMSO-*d*₆) δ 12.13 (s, 1H), 11.15 (s, 1H), 10.26 (s, 1H), 8.70 (t, *J* = 6.1 Hz, 1H), 8.64 (d, *J* = 2.3 Hz, 1H), 8.10 (s, 1H), 7.98 (s, 2H), 7.93 (dd, *J* = 9.2, 2.5 Hz, 1H), 7.76 (d, *J* = 9.0 Hz, 2H), 7.42 (d, *J* = 8.4 Hz, 2H), 7.28 (d, *J* = 9.5 Hz, 1H), 7.17 (d, *J* = 8.5 Hz, 2H), 6.95 (d, *J* = 9.3 Hz, 2H), 5.17 (dd, *J* = 12.8, 5.5 Hz, 1H), 4.22 (s, 1H), 3.88 (d, *J* = 14.2 Hz, 2H), 3.57 (s, 2H), 3.55 - 3.10 (m, 15H), 2.90 (ddd, *J =* 16.9, 13.8, 5.5 Hz, 1H), 2.81 - 2.72 (m, 3H), 2.65 - 2.58 (m, 1H), 2.55 (d, *J* = 5.2 Hz, 1H), 2.28 (t, *J* = 6.8 Hz, 2H), 2.20 - 2.03 (m, 5H), 1.90 (d, *J =* 12.2 Hz, 2H), 1.63 (qt, *J =* 10.8, 5.8 Hz, 1H), 1.47 (t, *J* = 6.6 Hz, 4H), 1.07 (q, *J* = 12.1, 11.1 Hz, 2H), 1.00 (s, 6H). HRMS (ESI) m/z: calcd for C₅₇H₆₇ClN₉O₉S⁺ [M+H]⁺, 1088.4465; found, 1088.4469.

### Example 20: preparation of 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-(4-((2-(2,6-dioxopiperidin-3-yl)-1,3-dioxoisoindolin-5-yl)methyl)piperazin-1-yl)cyclohexyl)methyl)amino)-3-nitrophenyl)sulfonyl)benzamide (BCL-03188)

Referring to the method of Scheme 7, the target compound (BCL-03188) was prepared using the intermediate compound 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((3-nitro-4-((((trans)-4-(piperazin-1-yl)cyclohexyl)methyl)amino)phenyl)sulfonyl)benzamide (SIAIS364025) and the intermediate compound 5-(bromomethyl)-2-(2,6-dioxopiperidin-3-yl)isoindoline-1,3-dione (732195; CAS No.: 1312023-72-5) as raw materials. ¹H NMR (500 MHz, DMSO-*d*₆) δ 11.76 (s, 1H), 11.70 (s, 1H), 11.16 (s, 1H), 10.52 (s, 1H), 8.64 (t, *J* = 6.1 Hz, 1H), 8.56 (d, *J* = 2.4 Hz, 1H), 8.17 (s, 1H), 8.09 - 8.04 (m, 2H), 8.00 (d, *J* = 7.6 Hz, 1H), 7.81 (dd, *J* = 9.3, 2.6 Hz, 1H), 7.56 (d, *J* = 2.7 Hz, 1H), 7.54 - 7.49 (m, 2H), 7.39 (d, *J =* 8.4 Hz, 2H), 7.10 (dd, *J =* 9.2, 5.3 Hz, 3H), 6.72 (dd, *J =* 9.0, 2.4 Hz, 1H), 6.44 - 6.36 (m, 1H), 6.25 (d, *J* = 2.4 Hz, 1H), 5.18 (dd, *J* = 12.8, 5.5 Hz, 1H), 4.35 (s, 1H), 3.63 (d, *J* = 13.9 Hz, 2H), 3.53 (d, *J =* 4.4 Hz, 5H), 3.32 - 3.06 (m, 12H), 2.90 (ddd, *J =* 16.9, 13.7, 5.4 Hz, 1H), 2.74 (d, *J =* 5.0 Hz, 1H), 2.70 (t, *J =* 10.9 Hz, 2H), 2.65 - 2.58 (m, 1H), 2.57 - 2.53 (m, 1H), 2.33 (t, *J =* 6.6 Hz, 2H), 2.13 (d, *J =* 10.4 Hz, 2H), 2.10 - 2.04 (m, 1H), 2.01 (s, 2H), 1.90 (d, *J =* 11.6 Hz, 2H), 1.62 (dq, *J* = 8.1, 5.5, 4.0 Hz, 1H), 1.55 - 1.42 (m, 4H), 1.07 (q, *J* = 11.5 Hz, 2H), 0.94 (s, 6H). HRMS (ESI) m/z: calcd for C₆₄H₇₁ClN₁₁O₁₀S⁺ [M+H]⁺, 1220.4789; found, 1220.4785.

### Example 21: preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1,3-dioxoisoindolin-5-yl)methyl)piperazin-1-yl)-1-(phenylthio)butan-2-yl)amino)-3-((trifluoromethyl)sulfonyl)phenyl)sulfonyl)benzamide (BCL-03189)

Referring to the method of Scheme 7, the target compound (BCL-03189) was prepared using the intermediate compound (R)-4-(4-((4'-chloro-4,4-dimethyl-3,4,5,6-tetrahydro-[1,1'-biphenyl]-2-yl)methyl)piperazin-1-yl)-N-((4-((1-(phenylthio)-4-(piperazin-1-yl)butan-2-yl)amino)-3-((trifluoromethyl)sulfonyl)phenyl)sulfonyl)benzamide (SIAIS360129) and the intermediate compound 5-(bromomethyl)-2-(2,6-dioxopiperidin-3-yl)isoindoline-1,3-dione (732195; CAS No.: 1312023-72-5) as raw materials. ¹H NMR (500 MHz, DMSO-*d*₆) δ 12.18 (s, 1H), 11.15 (s, 1H), 10.08 (s, 1H), 8.17 (d, *J* = 2.4 Hz, 1H), 7.99 (dd, *J* = 9.3, 2.4 Hz, 1H), 7.92 (s, 2H), 7.84 (s, 1H), 7.77 (d, *J* = 9.0 Hz, 2H), 7.44 - 7.39 (m, 2H), 7.31 - 7.27 (m, 2H), 7.22 (t, *J* = 7.6 Hz, 3H), 7.17 (d, *J* = 8.7 Hz, 3H), 7.15 - 7.13 (m, 1H), 6.97 (t, *J* = 10.1 Hz, 3H), 5.16 (dd, *J* = 12.9, 5.4 Hz, 1H), 4.18 (s, 1H), 3.95 - 3.84 (m, 2H), 3.79 (s, 1H), 3.58 (s, 2H), 3.40 - 3.25 (m, 7H), 3.17 (s, 1H), 3.00 (s, 3H), 2.90 (ddd, *J* = 17.1, 14.2, 5.4 Hz, 3H), 2.76 (dd, *J* = 19.2, 6.9 Hz, 2H), 2.65 - 2.58 (m, 2H), 2.56 - 2.54 (m, 1H), 2.28 (t, *J* = 6.5 Hz, 2H), 2.14 (s, 4H), 2.10 - 2.03 (m, 1H), 1.47 (t, *J* = 6.6 Hz, 2H), 1.00 (s, 6H). HRMS (ESI) m/z: calcd for C₆₁H₆₇ClF₃N₈O₉S₃⁺ [M+H]⁺, 1243.3828; found, 1243.3829.

### Example 22: preparation of 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((cis)-4-((3S)-4-((2-(2,6-dioxopiperidin-3-yl)-1,3-dioxoisoindolin-5-yl)methyl)-3-methylpiperazin-1-yl)cyclohexyl)methyl)amino)-3-nitrophenyl)sulfonyl)benzamide (BCL-03190)

Referring to the method of Scheme 7, the target compound (BCL-03190) was prepared using the intermediate compound (732147) prepared according to the intermediate Example 3 and the intermediate compound 5-(bromomethyl)-2-(2,6-dioxopiperidin-3-yl)isoindoline-1,3-dione (732195; CAS No.: 1312023-72-5) as raw materials. ¹H NMR (500 MHz, DMSO-*d*₆) δ 11.79 (s, 1H), 11.71 (s, 1H), 11.17 (s, 1H), 10.67 (s, 1H), 8.63 (t, *J* = 6.0 Hz, 1H), 8.57 (d, *J* = 2.6 Hz, 1H), 8.27 (s, 1H), 8.15 (s, 1H), 8.06 (d, *J* = 2.9 Hz, 1H), 8.02 (d, *J* = 7.6 Hz, 1H), 7.82 (dd, *J* = 9.2, 2.7 Hz, 1H), 7.58 (d, *J* = 2.9 Hz, 1H), 7.56 - 7.48 (m, 2H), 7.39 (d, *J =* 8.4 Hz, 2H), 7.16 (d, *J =* 9.6 Hz, 1H), 7.10 (d, *J =* 8.4 Hz, 2H), 6.72 (dd, *J =* 9.1, 2.5 Hz, 1H), 6.45 - 6.36 (m, 1H), 6.26 (d, *J =* 2.4 Hz, 1H), 5.19 (dd, *J =* 12.8, 5.5 Hz, 1H), 4.88 (s, 1H), 4.30 (s, 1H), 3.97 - 3.50 (m, 10H), 3.27 (d, *J =* 11.7 Hz, 8H), 2.91 (ddd, *J* = 17.4, 13.6, 5.5 Hz, 1H), 2.69 (q, *J* = 10.9, 9.5 Hz, 2H), 2.65 - 2.58 (m, 1H), 2.58 - 2.53 (m, 1H), 2.35 (t, *J* = 6.9 Hz, 2H), 2.09 (td, *J* = 7.6, 3.7 Hz, 1H), 2.01 (s, 3H), 1.93 - 1.74 (m, 6H), 1.66 - 1.47 (m, 5H), 1.44 (t, *J =* 6.4 Hz, 2H), 0.94 (s, 6H). HRMS (ESI) m/z: calcd for C₆₅H₇₃ClN₁₁O₁₀S⁺ [M+H]⁺, 1234.4946; found, 1234.4940.

### Example 23: preparation of 2-((1H-pyrrolo[2,3-b]pyridin-5-yl)oxy)-N-((4-((((trans)-4-(4-((7-bromo-2-(2,6-dioxopiperidin-3-yl)-3-oxoisoindolin-5-yl)methyl)piperazin-1-yl)cyclohexyl)methyl)amino)-3-((trifluoromethyl)sulfonyl)phenyl)sulfonyl)-4-(4-((4'-chloro-5,5-dimethyl-3,4,5,6-tetrahydro-[1,1'-biphenyl]-2-yl)methyl)piperazin-1-yl)benzamide (BCL-03182)

Referring to the method of Scheme 7, the target compound (BCL-03182) was prepared using the intermediate compound (732185) and the intermediate compound (732001) as raw materials. ¹H NMR (500 MHz, DMSO-*d*₆) δ 11.82 (t, *J =* 2.4 Hz, 1H), 11.75 (s, 1H), 11.05 (s, 1H), 10.80 (s, 1H), 8.23 (s, 1H), 8.17 (d, *J* = 2.6 Hz, 1H), 8.08 (s, 1H), 8.07 (d, *J* = 2.7 Hz, 1H), 7.93 (dd, *J* = 9.3, 2.6 Hz, 1H), 7.60 (d, *J =* 2.7 Hz, 1H), 7.55 (t, *J =* 3.0 Hz, 1H), 7.50 (d, *J =* 8.9 Hz, 1H), 7.39 (d, *J =* 8.4 Hz, 2H), 7.34 (t, *J=* 6.0 Hz, 1H), 7.10 (d, *J=* 8.5 Hz, 2H), 7.08 (d, *J=* 9.8 Hz, 1H), 6.73 (dd, *J* = 9.0, 2.4 Hz, 1H), 6.47 - 6.40 (m, 1H), 6.24 (d, *J* = 2.3 Hz, 1H), 5.16 (dd, *J* = 13.2, 5.1 Hz, 1H), 4.50 (s, 1H), 4.46 (d, *J* = 18.0 Hz, 1H), 4.31 (d, *J* = 17.9 Hz, 1H), 3.69 - 3.57 (m, 12H), 3.29 (dd, *J* = 22.6, 11.6 Hz, 5H), 3.22 (t, *J* = 6.3 Hz, 3H), 2.92 (ddd, *J* = 17.4, 13.4, 5.2 Hz, 1H), 2.69 (d, *J* = 11.7 Hz, 2H), 2.61 (d, *J* = 17.5 Hz, 1H), 2.46 (dd, *J* = 13.1, 4.9 Hz, 1H), 2.36 (s, 2H), 2.17 - 2.10 (m, 2H), 2.02 (d, *J* = 11.0 Hz, 3H), 1.84 (d, *J* = 11.4 Hz, 2H), 1.59 (tt, *J* = 7.8, 3.9 Hz, 1H), 1.49 (d, *J* = 12.3 Hz, 2H), 1.43 (t, *J* = 6.4 Hz, 2H), 1.06 (d, *J* = 13.2 Hz, 2H), 0.94 (s, 6H). HRMS (ESI) m/z: calcd for C₆₅H₇₂BrClF₃N₁₀O₉S₂⁺ [M+H]⁺, 1371.3744; found, 1371.3740.

### Example 24: preparation of N-((4-((((trans)-4-(4-((7-bromo-2-(2,6-dioxopiperidin-3-yl)-3-oxoisoindolin-5-yl)methyl)piperazin-1-yl)cyclohexyl)methyl)amino)-3-((trifluoromethyl)sulfonyl)phenyl)sulfonyl)-4-(4-((4'-chloro-4,4-dimethyl-3,4,5,6-tetrahydro-[1,1'-biphenyl]-2-yl)methyl)piperazin-1-yl)benzamide (BCL-03183)

Referring to the method of Scheme 7, the target compound (BCL-03183) was prepared using the intermediate compound (732186) and the intermediate compound (732001) as raw materials. ¹H NMR (500 MHz, DMSO-*d*₆) δ 12.18 (s, 1H), 11.04 (s, 1H), 10.68 (s, 1H), 8.22 (d, *J =* 2.6 Hz, 1H), 8.16 (s, 1H), 8.05 (dd, *J* = 9.2, 2.5 Hz, 1H), 8.02 (s, 1H), 7.76 (d, *J* = 9.0 Hz, 2H), 7.44 - 7.38 (m, 3H), 7.24 (d, *J* = 9.6 Hz, 1H), 7.18 (d, *J* = 8.4 Hz, 2H), 6.95 (d, *J* = 9.2 Hz, 2H), 5.16 (dd, *J* = 13.3, 5.2 Hz, 1H), 4.37 (dd, *J* = 76.8, 17.9 Hz, 4H), 3.88 (d, *J =* 13.3 Hz, 2H), 3.56 (s, 6H), 3.36 - 3.07 (m, 11H), 2.92 (ddd, *J =* 17.5, 13.5, 5.2 Hz, 1H), 2.77 (s, 2H), 2.61 (dd, *J* = 13.9, 3.5 Hz, 1H), 2.46 (dd, *J* = 13.1, 4.9 Hz, 1H), 2.27 (s, 2H), 2.21 (s, 2H), 2.12 (s, 2H), 2.03 (td, *J =* 7.6, 3.8 Hz, 1H), 1.84 (d, *J =* 11.4 Hz, 2H), 1.60 (td, *J* = 9.5, 7.9, 5.8 Hz, 1H), 1.47 (q, *J* = 7.4, 6.7 Hz, 4H), 1.11 - 1.02 (m, 2H), 1.00 (s, 6H). HRMS (ESI) m/z: calcd for C₅₈H₆₈BrClF₃N₈O₈S₂⁺ [M+H]⁺, 1239.3420; found, 1239.3421.

### Example 25: preparation of N-((4-((((trans)-4-(4-((7-bromo-2-(2,6-dioxopiperidin-3-yl)-3-oxoisoindolin-5-yl)methyl)piperazin-1-yl)cyclohexyl)methyl)amino)-3-nitrophenyl)sulfonyl)-4-(4-((4'-chloro-4,4-dimethyl-3,4,5,6-tetrahydro-[1,1'-biphenyl]-2-yl)methyl)piperazin-1-yl)benzamide (BCL-03184)

Referring to the method of Scheme 7, the target compound (BCL-03184) was prepared using the intermediate compound (732187) and the intermediate compound (732001) as raw materials. ¹H NMR (500 MHz, DMSO-*d*₆) δ 12.14 (s, 1H), 11.04 (s, 1H), 10.63 (s, 1H), 8.70 (t, *J =* 6.1 Hz, 1H), 8.64 (d, *J* = 2.3 Hz, 1H), 8.15 (s, 1H), 8.01 (s, 1H), 7.94 (dd, *J* = 9.2, 2.5 Hz, 1H), 7.77 (d, *J* = 9.0 Hz, 2H), 7.42 (d, *J =* 8.5 Hz, 2H), 7.28 (d, *J =* 9.6 Hz, 1H), 7.18 (d, *J =* 8.5 Hz, 2H), 6.95 (d, *J =* 9.2 Hz, 2H), 5.16 (dd, *J* = 13.3, 5.2 Hz, 1H), 4.37 (dd, *J* = 77.3, 17.8 Hz, 4H), 3.88 (d, *J =* 13.0 Hz, 2H), 3.56 (s, 5H), 3.37 - 3.13 (m, 12H), 2.96 - 2.86 (m, 1H), 2.81 - 2.70 (m, 2H), 2.61 (d, *J =* 17.3 Hz, 1H), 2.47 (dd, *J =* 13.4, 8.2 Hz, 1H), 2.28 (t, *J* = 6.8 Hz, 2H), 2.21 (s, 2H), 2.17 - 2.09 (m, 2H), 2.07 - 2.00 (m, 1H), 1.91 (d, *J* = 11.3 Hz, 2H), 1.64 (tdd, *J* = 14.8, 7.0, 3.4 Hz, 1H), 1.47 (q, *J* = 7.5, 6.7 Hz, 4H), 1.08 (q, *J* = 12.5, 11.3 Hz, 2H), 1.00 (s, 6H). HRMS (ESI) m/z: calcd for C₅₇H₆₈BrClN₉O₈S⁺ [M+H]⁺, 1152.3778; found, 1152.3781.

### Example 26: preparation of N-((4-(((2R)-4-(4-((7-bromo-2-(2,6-dioxopiperidin-3-yl)-3-oxoisoindolin-5-yl)methyl)piperazin-1-yl)-1-(phenylthio)butan-2-yl)amino)-3-((trifluoromethyl)sulfonyl)phenyl)sulfonyl)-4-(4-((4'-chloro-4,4-dimethyl-3,4,5,6-tetrahydro-[1,1'-biphenyl]-2-yl)methyl)piperazin-1-yl)benzamide (BCL-03178)

Referring to the method of Scheme 7, the target compound (BCL-03178) was prepared using the intermediate compound (SIAIS360129) and the intermediate compound (732001) as raw materials. ¹H NMR (500 MHz, DMSO-*d*₆) δ 12.19 (s, 1H), 11.04 (s, 1H), 10.49 (s, 1H), 8.18 (d, *J* = 2.6 Hz, 1H), 8.05 (s, 1H), 7.98 (dd, *J* = 9.3, 2.4 Hz, 1H), 7.94 (s, 1H), 7.77 (d, *J* = 9.0 Hz, 2H), 7.41 (d, *J* = 8.5 Hz, 2H), 7.29 (d, *J* = 7.6 Hz, 2H), 7.22 (t, *J* = 7.7 Hz, 3H), 7.20 - 7.12 (m, 3H), 6.99 (d, *J* = 8.9 Hz, 1H), 6.96 (d, *J =* 9.2 Hz, 2H), 5.15 (dd, *J =* 13.3, 5.2 Hz, 1H), 4.44 (d, *J =* 17.9 Hz, 1H), 4.28 (d, *J =* 17.7 Hz, 1H), 4.20 (s, 2H), 3.89 (d, *J =* 12.7 Hz, 2H), 3.57 (s, 2H), 3.50 - 3.00 (m, 15H), 2.97 - 2.86 (m, 2H), 2.77 (d, *J* = 11.3 Hz, 2H), 2.62 (d, *J =* 3.5 Hz, 1H), 2.59 (s, 1H), 2.46 (dd, *J* = 13.2, 4.8 Hz, 1H), 2.28 (t, *J =* 6.9 Hz, 2H), 2.21 - 2.07 (m, 4H), 2.06 - 1.97 (m, 1H), 1.47 (t, *J =* 6.5 Hz, 2H), 1.00 (s, 6H). HRMS (ESI) m/z: calcd for C₆₁H₆₈BrClF₃N₈O₈S₃⁺ [M+H]⁺, 1307.3141; found, 1307.3139.

**Biological activity assays**

### Reagents and Materials

- Reagents and materials: Suppliers or Source
- RPMI 1640 cell culture medium: HyClone Company
- IMDM cell culture medium: Gibco Company
- DMEM cell culture medium: HyClone Company
- Penicillin and streptomycin solutions: Gibco Company
- G418: Sigma Company
- Zeocin: Invitrogen Company
- Fetal bovine serum (FBS): HyClone Company
- Thiazolyl blue: Genview Company
- Sodium dodecyl sulfate (SDS): Genview Company
- Isopropanol: Sinopharm Chemical Reagent Co., Ltd
- Hydrochloric acid: Sinopharm Chemical Reagent Co., Ltd
- Dual luciferase assay reporter kit: Promega Company
- IP lysis buffer: Biyuntian Biotechnology Co., Ltd
- PMSF: Biyuntian Biotechnology Co., Ltd
- Halt^{™} Protease and Phosphatase Inhibitors: Thermo Fisher Company
- 4×LDS: Thermo Fisher Company
- β-mercaptoethanol: Genview Company
- 30% polyacrylamide solution: Beijing Dingguo Changsheng Biotechnology Co.,Ltd.
- 0.22 µm PVDF membrane: Millipore Company
- sodium chloride: Sinopharm Chemical Reagent Co., Ltd
- Tris(hydroxymethyl)aminoethane (Tris): Genview Company
- Tween-20: Beijing Dingguo Changsheng Biotechnology Co.,Ltd.
- SAG: Selleck Company

### Antibody:

The BCL-XL antibody used in the experiment was purchased from Abcam Company, and the Tubulin antibody was purchased from Proteintech Company.

### Cell culture

The cell lines RS4;11 (human acute lymphocytic leukemia cells), Molt-4 cells (human acute lymphocytic leukemia cells), MV4;11 (human myeloid monocytic leukemia cells), and Light II cells were commercially available or purchased from ATCC (American Type Culture Collection) and routinely cultured according to instructions. The cells used were shown to be authentic using STR and were negative for mycoplasma by routine examination.

### Evaluation of the inhibitory activity against tumor cells proliferation in vitro

The compounds of the present disclosure were subjected to preliminary evaluation of tumor cell growth inhibition rate at single or double concentrations, and determination of IC₅₀.

**Determination of half maximal inhibitory concentration (IC₅₀) of the compounds of the present disclosure: Thiazolyl Blue cell proliferation assay**
1) Study on the proliferation inhibition effects induced by the compounds of the present disclosure designed based on ABT199/263 against BCL-XL-dependent RS4;11 cells: 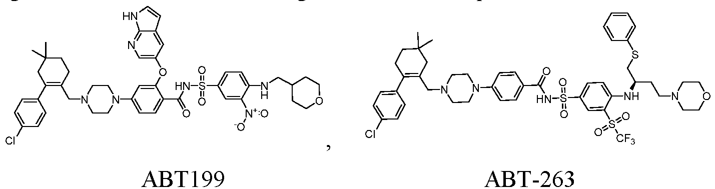 The specific steps were as follows: RS4;11 cells were seeded at a density of 3×10⁴ cells/well in 90 µL of serum-containing RPMI-1640 culture medium. DT2216 and the compounds of the present disclosure were added to the cell suspension at corresponding concentration gradients. DT2216 and DMSO were used as positive and vehicle control, respectively. The positive control and negative control were treated with the same treatment method as the compounds of the present disclosure. After treating the cells with the compounds of the present disclosure for 48 hours, thiazole blue solution (5 mg/mL) was added at 10 µL/well to the cell suspension, followed by incubation for 3 hours at 37°C in an incubator with 5% CO₂. Then, triple solution (0.1 g/mL SDS, 5% isopropyl alcohol, 10 mM hydrochloric acid) was added, followed by incubation for 4-6 hours at 37°C in an incubator with 5% CO₂. After confirming that all the crystal violets were dissolved under inverted biological microscopy, the absorbance was measured at 490 nm using an Epoch microplate reader (Biotek Corporation). The growth inhibition of the compounds of the present disclosure on the cells was plotted by GraphPad Prism 8 software, and the IC₅₀ values of the compounds of the present disclosure were calculated therefrom. The experiment had 3 replicates per group and was repeated at least three times. Results were shown in Table 3 below.
2) Study on the proliferation inhibition effects induced by the compounds of the present disclosure designed based on ABT199/263 against BCL-XL-dependent MV4;11 cells:
   The specific steps were as follows: MV4;11 cells were seeded at a density of 2×10⁴ cells/well in 90 µL of serum-containing IMDM culture medium. DT2216 and the compounds of the present disclosure were added to the cell suspension at corresponding concentration gradients. DT2216 and DMSO were used as positive and vehicle control, respectively. The positive control and negative control were treated with the same treatment method as the compounds of the present disclosure. After treating the cells with the compounds of the present disclosure for 48 hours, thiazole blue solution (5 mg/mL) was added at 10 µL/well to the cell suspension, followed by incubation for 3 hours at 37°C in an incubator with 5% CO₂. Then, triple solution (0.1 g/mL SDS, 5% isopropyl alcohol, 10 mM hydrochloric acid) was added, followed by incubation for 4-6 hours at 37°C in an incubator with 5% CO₂. After confirming that all the crystal violets were dissolved under inverted biological microscopy, the absorbance was measured at 490 nm using an Epoch microplate reader (Biotek Corporation). The growth inhibition of the compounds of the present disclosure on the cells was plotted by GraphPad Prism 8 software, and the IC₅₀ values of the compounds of the present disclosure were calculated therefrom. The experiment had 3 replicates per group and was repeated at least three times. Results were shown in Table 4 below.
3) Study on the proliferation inhibition effects induced by the compounds of the present disclosure designed based on ABT199/263 against BCL-XL-dependent Molt-4 cells:

The specific steps were as follows: Molt-4 cells were seeded at a density of 2×10⁴ cells/well in 180 µL of serum-containing RPMI 1640 culture medium. DT2216 and the compounds of the present disclosure were added to the cell suspension at corresponding concentration gradients. DT2216 and DMSO were used as positive and vehicle control, respectively. The positive control and negative control were treated with the same treatment method as the compounds of the present disclosure. After treating the cells with the compounds of the present disclosure for 72 hours, thiazole blue solution (5 mg/mL) was added at 20 µL/well to the cell suspension, followed by incubation for 3 hours at 37°C in an incubator with 5% CO₂. Then, triple solution (0.1 g/mL SDS, 5% isopropyl alcohol, 10 mM hydrochloric acid) was added, followed by incubation for 4-6 hours at 37°C in an incubator with 5% CO₂. After confirming that all the crystal violets were dissolved under inverted biological microscopy, the absorbance was measured at 490 nm using an Epoch microplate reader (Biotek Corporation). The growth inhibition of the compounds of the present disclosure on the cells was plotted by GraphPad Prism 8 software, and the IC₅₀ values of the compounds of the present disclosure were calculated therefrom. The experiment had 3 replicates per group and was repeated at least three times. Results were shown in Table 5 below.

### Determination of the IC₅₀ values of compounds inhibiting Hedgehog activity---Dual-luciferase reporter assay:

Light II cells were NIH3T3 cell lines that stably express the Gli-luciferase and TK-renilla reporters. They were cultured with DMEM medium containing 10% (v/v) fetal bovine serum (FBS), 0.4 mg/ml G418, 0.15 mg/ml bleomycin (Zeocin), and penicillin/streptomycin at 37°C in an incubator with 5% CO₂. Light II cells in the logarithmic growth phase were seeded at a density of 2.5×10⁴ cells/well in a 96-well plate. After 24h incubation, the cells were subjected to treatment with SAG with or without different concentrations of the compounds of the present disclosure for 36h. After that, the Hedgehog activity was meaured using the dual fluorescence reporter kit according to its instructions. The experiment had 3 replicates per group and was repeated at least three times. Results were shown in Table 6 below.

### Western Blot assays

Molt-4 cells in logarithmic growth phase seeded at a density of 3×10⁶ cells/well in a 6-well plate were subjected to treatment with the compounds of the present disclosure, and DMSO was used as the vehicle control group. After 10 hours of treatment, the cells were harvested and washed once in PBS. Cells were placed on ice and treated with IP protein lysis buffer containing PMSF protease inhibitors. After centrifugation at 4 °C (10 k rpm, 5 min), the supernatant was collected and mixed with 4 × LDS protein loading buffer at a volume ratio of 3:1. The mixture of protein supernatant with loading buffer were further added with 5% β-mercaptoethanol. After incubation in a metal bath at 95°C for 10 minutes, the protein samples was stored at -80°C or cooled directly on ice for protein electrophoresis using 10% homemade polyacrylamide gel as separation gel. Electrophoresis tank and other related components were purchased from Bio-rad company. The electrophoresis conditions were voltage 80 V for 45 minutes, then switch to voltage 120V for further electrophoresis for 1 hour. Then protein in gel was transferred onto PVDF membrane at an isocurrent of 300 mA in an icy water bath for one hour. Afterwards, the membrane was blocked with 5% skim milk for 1 hour at room temperature. Subsequently the membrane was incubated with primary antibody at 4°C overnight or at room temperature for 2 h, then washed with 1×TBST buffer (sodium chloride, 150 mM, Tris, 10 mM, Tween 20, 0.1%; PH 7.4) for three times, and then added with secondary antibody and incubated at room temperature for 45 min, washed with 1×TBST buffer (sodium chloride, 150 mM, T ris, 10 mM, Tween 20, 0.1%; PH 7.4) three times, and subjected to color development. The antibody dilution ratio was determined according to the antibody instructions.

### Results

The experimental data were shown below.

### 1. Study on the proliferation inhibition effects induced by the compounds of the present disclosure designed based on ABT199/263 against BCL-XL-dependent cells

We conducted dose-dependent experiments on the designed and synthesized compounds of the present disclosure in RS4;11 cells, which are highly sensitive to BCL-XL inhibitors. The cells were treated with 7 different concentrations (starting at the highest concentration of 1000 nM or 100nM; 10-fold serial dilutions) of the compounds for 48 hours, and then tested using the thiazolyl blue cell proliferation assay. The experiments were repeated more than 3 times, and the specific results were shown in Table 3 below.

The compounds of the present disclosure developed based on ABT-199/263 (including compounds in Tables 1-2 and compounds of Examples 1-26) significantly inhibited the proliferation of RS4;11 tumor cells (Table 3). The IC₅₀ value of DT2216 for inhibiting the proliferation of RS4;11 cells was 81.04±24.89nM, and the inhibitory effects of the compounds of the present disclosure that we developed were significantly better than that of DT2216.

**Table 3. IC₅₀ (nM, half-inhibitory concentration) values of the compounds of the present disclosure based on the ABT-199/263 for inhibiting the proliferation of RS4;11 cells (acute lymphoblastic-leukemia cells)**

| Cells | Tested compounds | Reagents | IC₅₀ (nM) |
|---|---|---|---|
| RS4;11 | DT2216 | Thiazolyl blue | 81.04±24.89 |
| RS4;11 | BCL-03146 | Thiazolyl blue | <20 |
| RS4;11 | BCL-03149 | Thiazolyl blue | <20 |
| RS4;11 | BCL-03165 | Thiazolyl blue | <20 |
| RS4;11 | BCL-03166 | Thiazolyl blue | <20 |
| RS4;11 | BCL-03167 | Thiazolyl blue | <20 |
| RS4;11 | BCL-03168 | Thiazolyl blue | <20 |
| RS4;11 | BCL-03169 | Thiazolyl blue | <20 |
| RS4;11 | BCL-03177 | Thiazolyl blue | <20 |
| RS4;11 | BCL-03179 | Thiazolyl blue | <20 |
| RS4;11 | BCL-03180 | Thiazolyl blue | <20 |
| RS4;11 | BCL-03181 | Thiazolyl blue | <20 |
| RS4;11 | BCL-03185 | Thiazolyl blue | <20 |
| RS4;11 | BCL-03188 | Thiazolyl blue | <20 |
| RS4;11 | BCL-03189 | Thiazolyl blue | <20 |
| RS4;11 | BCL-03190 | Thiazolyl blue | <20 |
| RS4;11 | BCL-03147 | Thiazolyl blue | <20 |
| RS4;11 | BCL-03148 | Thiazolyl blue | <20 |
| RS4;11 | BCL-03182 | Thiazolyl blue | <20 |

DT2216 (CAS No.: 2365172-42-3) is a reported BCL-XL degradation agent, with the following structure:

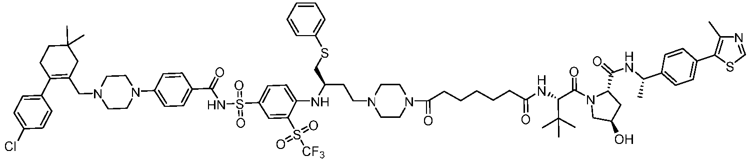

The compounds of the present disclosure developed based on ABT-199/263 (including compounds in Tables 1-2 and compounds of Examples 1-26) significantly inhibited the proliferation of myeloid monocytic leukemia cells MV4;11 (Table 4). The IC₅₀ value of DT2216 for inhibiting the proliferation of MV4; 11 cells was 164.9±70.71 nM, and the inhibitory effects of the compounds of the present disclosure that we developed were significantly better than that of DT2216.

**Table 4. IC₅₀ (nM, half-inhibitory concentration) values of the compounds of the present disclosure based on the ABT-199/263 for inhibiting the proliferation of myeloid monocytic leukemia cells MV4;11**

| Cells | Tested compounds | Reagents | IC₅₀ (nM) |
|---|---|---|---|
| MV4;11 | DT2216 | Thiazolyl blue | 164.9±70.71 |
| MV4;11 | BCL-03146 | Thiazolyl blue | <20 |
| MV4;11 | BCL-03147 | Thiazolyl blue | <20 |
| MV4;11 | BCL-03148 | Thiazolyl blue | <20 |
| MV4;11 | BCL-03149 | Thiazolyl blue | <20 |
| MV4;11 | BCL-03177 | Thiazolyl blue | <20 |
| MV4;11 | BCL-03165 | Thiazolyl blue | <20 |
| MV4;11 | BCL-03166 | Thiazolyl blue | <20 |
| MV4;11 | BCL-03167 | Thiazolyl blue | <20 |
| MV4;11 | BCL-03168 | Thiazolyl blue | <20 |
| MV4;11 | BCL-03169 | Thiazolyl blue | <20 |
| MV4;11 | BCL-03179 | Thiazolyl blue | <20 |
| MV4;11 | BCL-03185 | Thiazolyl blue | <20 |
| MV4;11 | BCL-03188 | Thiazolyl blue | <20 |
| MV4;11 | BCL-03189 | Thiazolyl blue | <20 |
| MV4;11 | BCL-03190 | Thiazolyl blue | <20 |
| MV4;11 | BCL-03182 | Thiazolyl blue | <20 |

The compounds of the present disclosure developed based on ABT-199/263 (including compounds in Tables 1-2 and compounds of Examples 1-26) significantly inhibited the proliferation of human acute lymphocytic leukemia cells Molt-4 (Table 5). The inhibitory effects of the compounds of the present disclosure that we developed were significantly better than that of DT2216.

**Table 5. IC₅₀ (nM, half-inhibitory concentration) values of the compounds of the present disclosure based on the ABT-199/263 for inhibiting the proliferation of acute lymphocytic leukemia cells Molt-4**

| Cells | Tested compounds | Reagents | IC₅₀ (nM) |
|---|---|---|---|
| Molt-4 | DT2216 | Thiazolyl blue | 17.3±9.57 |
| Molt-4 | BCL-03146 | Thiazolyl blue | <10 |
| Molt-4 | BCL-03147 | Thiazolyl blue | <10 |
| Molt-4 | BCL-03148 | Thiazolyl blue | <10 |
| Molt-4 | BCL-03149 | Thiazolyl blue | <10 |
| Molt-4 | BCL-03165 | Thiazolyl blue | <10 |
| Molt-4 | BCL-03166 | Thiazolyl blue | <10 |
| Molt-4 | BCL-03167 | Thiazolyl blue | <10 |
| Molt-4 | BCL-03168 | Thiazolyl blue | <10 |
| Molt-4 | BCL-03169 | Thiazolyl blue | <10 |
| Molt-4 | BCL-03170 | Thiazolyl blue | <10 |
| Molt-4 | BCL-03171 | Thiazolyl blue | <10 |
| Molt-4 | BCL-03172 | Thiazolyl blue | <10 |
| Molt-4 | BCL-03177 | Thiazolyl blue | <10 |
| Molt-4 | BCL-03179 | Thiazolyl blue | <10 |
| Molt-4 | BCL-03185 | Thiazolyl blue | <10 |
| Molt-4 | BCL-03188 | Thiazolyl blue | <10 |
| Molt-4 | BCL-03189 | Thiazolyl blue | <10 |
| Molt-4 | BCL-03190 | Thiazolyl blue | <10 |
| Molt-4 | BCL-03182 | Thiazolyl blue | <10 |
| Molt-4 | BCL-03178 | Thiazolyl blue | <10 |

**2. the IC₅₀ values of the compounds of the present disclosure based on ABT199/263 for inhibiting (Hh) signaling pathway Hedgehog activity:**

The compounds of the present disclosure based on ABT-199/263 (including compounds in Tables 1-2 and compounds of Examples 1-26) were subjected to screening for their activity in inhibiting the Hedgehog (Hh) signaling pathway (Table 6). The IC₅₀ values of the compounds of the present disclosure based on ABT-199/263 reached below 10 nM.

**Table 6. IC₅₀ (nM, half-inhibitory concentration) values of the compounds of the present disclosure based on the ABT-199/263 in Light II cells**

| Cells | Tested compounds | Reagents | IC₅₀ (nM) |
|---|---|---|---|
| LightII | BCL-03146 | Dual-luciferase assay reporter kit | <10 |
| LightII | BCL-03147 | Dual-luciferase assay reporter kit | <10 |
| LightII | BCL-03148 | Dual-luciferase assay reporter kit | <10 |
| LightII | BCL-03149 | Dual-luciferase assay reporter kit | <10 |
| LightII | BCL-03165 | Dual-luciferase assay reporter kit | <10 |
| LightII | BCL-03166 | Dual-luciferase assay reporter kit | <10 |
| LightII | BCL-03167 | Dual-luciferase assay reporter kit | <10 |
| LightII | BCL-03168 | Dual-luciferase assay reporter kit | <10 |
| LightII | BCL-03169 | Dual-luciferase assay reporter kit | <10 |
| LightII | BCL-03177 | Dual-luciferase assay reporter kit | <10 |
| LightII | BCL-03189 | Dual-luciferase assay reporter kit | <10 |

### 3. The compound of the present disclosure based on ABT199/263 can effectively degrade the protein level of BCL-XL in Molt-4 cells

Molt-4 cells were seeded into a 6-well plate at a density of 3×10⁶ cells per well. The next day, the cells were treated with the compounds of the present disclosure at a concentration of 100 nM, and the control groups were treated with DMSO and DT2216, respectively. Protein were extracted after 8 hours treatment, and BCL-XL protein expression was assessed by the western blot. The results showed that treatment with the compounds of the present disclosure (including the compounds in Tables 1-2 and compounds of Examples 1-26) resulted in significant reduction of BCL-XL protein expression. Compounds of the present disclosure such as BCL-03146, BCL-03147, BCL-03148, and BCL-03149 etc. developed in the present invention significantly degraded BCL-XL protein at a concentration of around 100 nM (as shown in Figures 1-3). Compared with DT2216, the compounds of the present disclosure exhibited a much more potent ability in in protein degradation.

The basic principles, main features and advantages of the present disclosure are shown and described above. Those skilled in the art should underst and that the present disclosure is not limited by the foregoing embodiments, and they can make various changes and alterations herein without departing from the spirit and scope of the present disclosure. These changes and alterations fall within the scope of the present disclosure. The claimed scope of the present disclosure is defined by the appended claims and their equivalents.

## Claims

1. A compound of Formula (I) 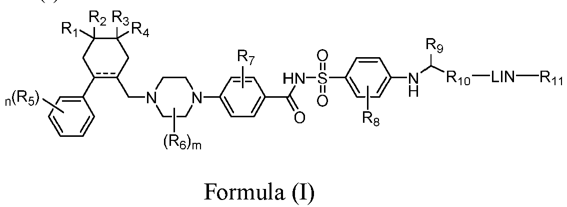 or salts, enantiomers, stereoisomers, solvates, isotopically enriched analogs, prodrugs, or polymorphs thereof,
wherein dotted line indicates the optional presence of a double bond;
R₁, R₂, R₃, R₄ are the same or different and each independently represents hydrogen, C₁₋₄ alkyl, halogen, or halogenated C₁₋₄ alkyl;
(R₅)ₙ indicates that the benzene ring is substituted with n R₅, with each R₅ being the same or different and each independently representing halogen, C₁₋₆ alkyl, or halogenated C₁₋₆ alkyl; n represents an integer of 1, 2, 3, 4 or 5;
(R₆)ₘ indicates that piperazine ring is substituted with m R₆, with each R₆ being the same or different and each independently representing halogen, C₁₋₆ alkyl, or halogenated C₁₋₆ alkyl; m represents an integer of 0, 1, 2, 3, 4, 5, 6, 7 or 8;
R₇ represents hydrogen or 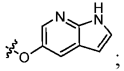
R₈ represents hydrogen or -SO₂CF₃ or -NO₂; R₉ represents hydrogen or C₁₋₄ alkyl;
R₁₀ represents the following group: 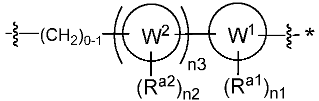
wherein ring W¹ represents 4- to 6-membered nitrogen-containing heterocyclylene; and (R^{a1})ₙ₁ indicates that ring W¹ is substituted with n1 R^{a1}, with each R^{a1} being the same or different and each independently representing C₁₋₆ alkyl, halogenated C₁₋₆ alkyl, C₁₋₆ alkoxy, cyano, halogen, or oxo; and n1 represents an integer of 0-8;
ring W² represents 4- to 6-membered nitrogen-containing heterocyclylene, or C₃₋₆ cycloalkylene; n3 represents an integer of 0 or 1; and (R^{a2})ₙ₂ indicates that ring W² is substituted with n2 R^{a2}, with each R^{a2} being the same or different and each independently representing C₁₋₆ alkyl, halogenated C₁₋₆ alkyl, C₁₋₆ alkoxy, cyano, halogen, or oxo; and n2 represent an integer of 0-8; and
symbol * indicates the point of attachment to LIN;
LIN represents:
optionally substituted linear or branched C₁₋₃₀ alkylene; or
optionally substituted linear or branched C₂₋₃₀ alkylene, wherein one or more groups R_{b} and/or one or more groups R_{c} and/or any combination of one or more groups R_{b} and R_{c} are inserted into the backbone carbon chain of the linear or branched C₂₋₃₀ alkylene group, wherein carbon-carbon bond between one or more pairs of adjacent carbon atoms in the backbone carbon chain is interrupted by the group R_{b}, R_{c}, or a combination of R_{b} and R_{c}; wherein each R_{b} is selected from the group consisting of O, N(Rₐ), C(O), C(O)O, S(O), S(O)₂, S(O)₂NH, NHS(O)₂, OC(O), C(O)N(R_{d}), N(R_{d})C(O), or N(R_{d})C(O)N(R_{d}), where each R_{b} independently represents H or C₁₋₆ alkyl, and in case that two or more groups R_{b} are inserted into the backbone carbon chain of the linear or branched C₂₋₃₀ alkylene group, the two or more groups R_{b} are not directly connected to each other; and wherein each R_{c} is selected from the group consisting of cycloalkylene, arylene, heterocyclylene, heteroarylene, alkynylene, alkenylene, or any combination thereof, wherein the cycloalkylene, arylene, heterocyclylene, and heteroarylene are each independently optionally substituted with a substituent selected from the group consisting of C₁₋₆ alkyl, halogenated C₁₋₆ alkyl, halogen, C₁₋₆ alkoxy, or any combination thereof; and
R₁₁ represents the following structure of Formula (II): 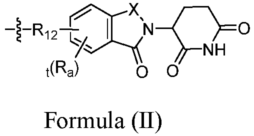
wherein R₁₂ represents N(Rₑ), O, S, C₂₋₆ alkynylene, or C₂₋₆ alkenylene, where Rₑ represents H or C₁₋₆ alkyl, or R₁₂ represents a bond;
(Rₐ)ₜ indicates that the benzene ring is substituted with t Rₐ, with each Rₐ being the same or different and each independently representing bromine, and t represents an integer of 0, 1, 2 or 3; and
X represents C(O) or CH₂;
wherein in case that t represents an integer of 0:
when R₇ represents 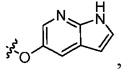 n1 represents an integer of 1-8, and R^{a1} are the same or different and each independently represents C₁₋₆ alkyl, halogenated C₁₋₆ alkyl, C₁₋₆ alkoxy, or halogen; or
when R₇ represents 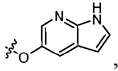 Rs represents -SO₂CF₃.

2. The compound of Formula (I) or salts, enantiomers, stereoisomers, solvates, isotopically enriched analogs, prodrugs, or polymorphs thereof as claimed in claim 1, which is also of Formula (I-1): 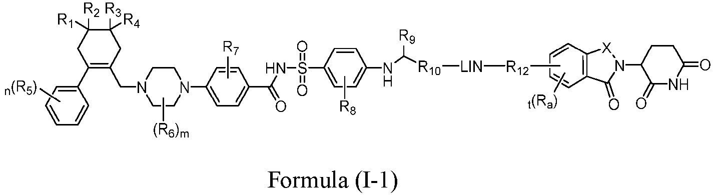 wherein R₁, R₂, R₃, R₄, (R₅)ₙ, (R₆)ₘ, R₇, R₈, R₉, R₁₀, LIN, R₁₂, (Rₐ)ₜ and X are as defined in claim 1.

3. The compound of Formula (I) or salts, enantiomers, stereoisomers, solvates, isotopically enriched analogs, prodrugs, or polymorphs thereof as claimed in claim 1, which is also of Formula (I-2) or Formula (I-3): 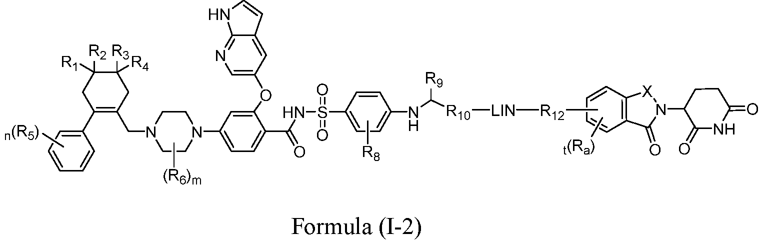 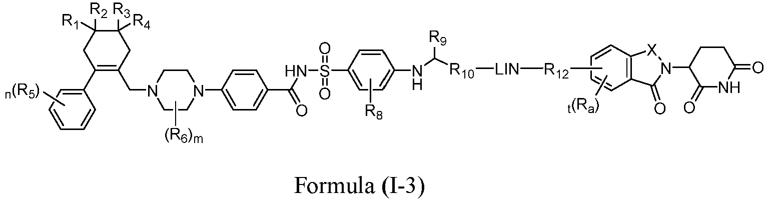 wherein R₁, R₂, R₃, R₄, (R₅)ₙ, (R₆)ₘ, R₈, R₉, R₁₀, LIN, R₁₂, (Rₐ)ₜ and X are as defined in claim 1.

4. The compound of Formula (I) or salts, enantiomers, stereoisomers, solvates, isotopically enriched analogs, prodrugs, or polymorphs thereof as claimed in claim 1, wherein t represents an integer of 1, 2 or 3.

5. The compound of Formula (I) or salts, enantiomers, stereoisomers, solvates, isotopically enriched analogs, prodrugs, or polymorphs thereof as claimed in claim 1, wherein t represents an integer of 0.

6. The compound of Formula (I) or salts, enantiomers, stereoisomers, solvates, isotopically enriched analogs, prodrugs, or polymorphs thereof as claimed in claim 5, which is also of Formula (I-2-1): 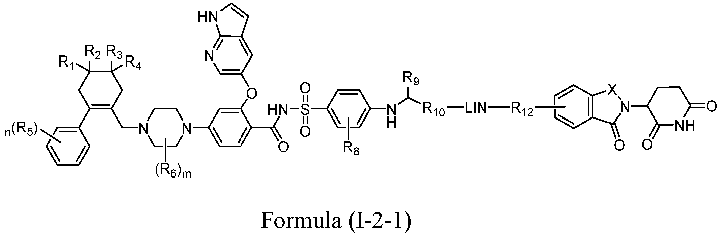 wherein R₁₀ represents the following group: 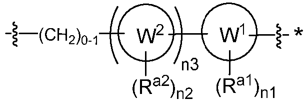
wherein ring W¹ represents 4- to 6-membered nitrogen-containing heterocyclylene; and (R^{a1})ₙ₁ indicates that ring W¹ is substituted with n1 R^{a1}, with each R^{a1} being the same or different and each independently representing C₁₋₆ alkyl, halogenated C₁₋₆ alkyl, C₁₋₆ alkoxy, cyano, or halogen; and n1 represents an integer of 1-8;
ring W² represents 4- to 6-membered nitrogen-containing heterocyclylene, or C₃₋₆ cycloalkylene; n3 represents an integer of 0 or 1; and (R^{a2})ₙ₂ indicates that ring W² is substituted with n2 R^{a2}, with each R^{a2} being the same or different and each independently representing C₁₋₆ alkyl, halogenated C₁₋₆ alkyl, C₁₋₆ alkoxy, cyano, halogen, or oxo; and n2 represents an integer of 0-8; and
symbol * indicates the point of attachment to LIN; and
R₁, R₂, R₃, R₄, (R₅)ₙ, (R₆)ₘ, R₈, R₉, LIN, R₁₂ and X are as defined in claim 1.

7. The compound of Formula (I) or salts, enantiomers, stereoisomers, solvates, isotopically enriched analogs, prodrugs, or polymorphs thereof as claimed in claim 6, wherein R₁₀ represents the following groups: 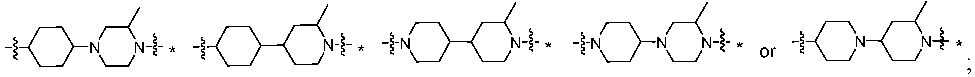 wherein symbol * indicates the point of attachment to LIN.

8. The compound of Formula (I) or salts, enantiomers, stereoisomers, solvates, isotopically enriched analogs, prodrugs, or polymorphs thereof as claimed in claim 5, which is also of Formula (I-2-1): 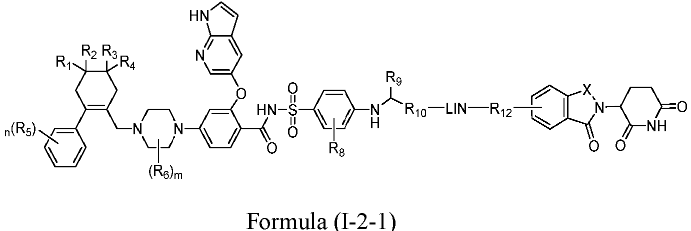 wherein R₈ represents -SO₂CF₃; and
R₁, R₂, R₃, R₄, (R₅)ₙ, (R₆)ₘ, R₉, R₁₀, LIN, R₁₂ and X are as defined in claim 1.

9. The compound of Formula (I) or salts, enantiomers, stereoisomers, solvates, isotopically enriched analogs, prodrugs, or polymorphs thereof as claimed in claim 1, wherein R₁₀ represents the following groups: 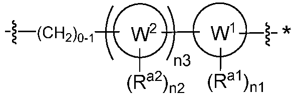
wherein ring W¹ represents 4- to 6-membered nitrogen-containing heterocyclylene, e.g., piperidinylene, piperazinylene, morpholinylene, azetidinylene, pyrrolidinylene, imidazolidylene, pyrazolidylene, oxazolidinylene, thiazolidinylene, or thiomorpholinylene;
(R^{a1})ₙ₁ indicates that ring W¹ is substituted with n1 R^{a1}, with each R^{a1} being the same or different and each independently representing C₁₋₆ alkyl, halogenated C₁₋₆ alkyl, C₁₋₆ alkoxy, cyano, halogen, or oxo; n1 represents an integer of 0, 1, 2, 3, or 4;
ring W² represents 4- to 6-membered nitrogen-containing heterocyclylene, or C₃₋₆ cycloalkylene,
and the 4- to 6-membered nitrogen-containing heterocyclylene includes e.g., piperidinylene, piperazinylene, morpholinylene, azetidinylene, pyrrolidinylene, imidazolidylene, pyrazolidylene, oxazolidinylene, thiazolidinylene, or thiomorpholinylene, and the 4- to 6-membered cycloalkylene includes e.g., cyclopropylene, cyclobutylene, cyclopentylene, or cyclohexylene;
n3 represents an integer of 0 or 1; (R^{a2})ₙ₂ indicates that ring W² is substituted with n2 R^{a2}, with each R^{a2} being the same or different and each independently representing C₁₋₆ alkyl, halogenated C₁₋₆ alkyl, C₁₋₆ alkoxy, cyano, halogen, or oxo; n2 represents an integer of 0, 1, 2, 3, or 4; and
symbol * indicates the point of attachment to LIN.

10. The compound of Formula (I) or salts, enantiomers, stereoisomers, solvates, isotopically enriched analogs, prodrugs, or polymorphs thereof as claimed in any one of claims 1-5 and 8-9, wherein R₁₀ represents the following groups: 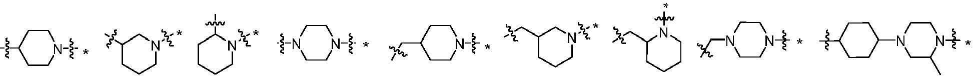 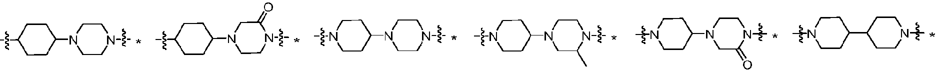 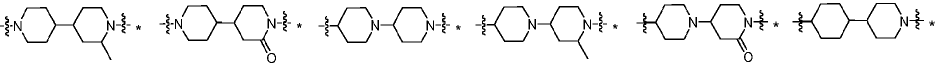 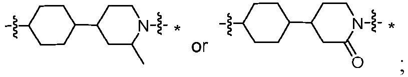 wherein symbol * indicates the point of attachment to LIN.

11. The compound of Formula (I) or salts, enantiomers, stereoisomers, solvates, isotopically enriched analogs, prodrugs, or polymorphs thereof as claimed in claim 1, wherein R₁ and R₂ represents hydrogen, and R₃ and R₄ represents methyl.

12. The compound of Formula (I) or salts, enantiomers, stereoisomers, solvates, isotopically enriched analogs, prodrugs, or polymorphs thereof as claimed in claim 1, wherein R₁ and R₂ represents methyl, and R₃ and R₄ represents hydrogen.

13. The compound of Formula (I) or salts, enantiomers, stereoisomers, solvates, isotopically enriched analogs, prodrugs, or polymorphs thereof as claimed in claim 1, wherein R₁₁ represents the following structure of Formula (II-1), Formula (II-2), Formula (II-3), Formula (II-4), Formula (II-5), Formula (II-6), Formula (II-7), Formula (II-8), Formula (II-9), Formula (II-10), Formula (II-11), or Formula (II-12): 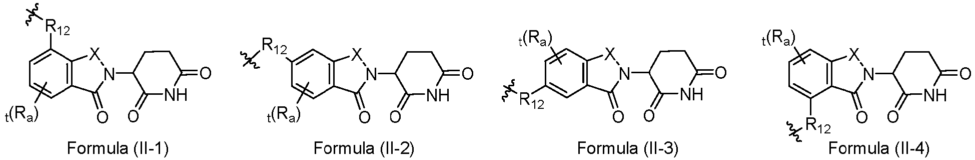 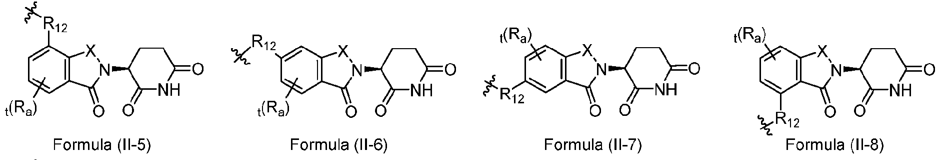 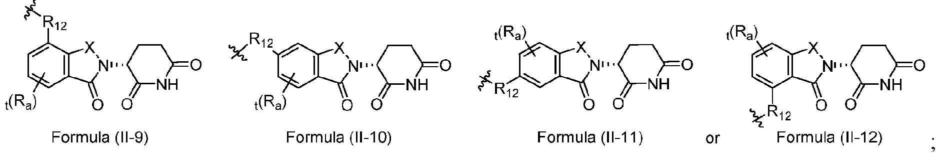
wherein R₁₂ represents N(Rₑ), O, S, C₂₋₆ alkynylene, or C₂₋₆ alkenylene, wherein Rₑ represents H or C₁₋₆ alkyl, or R₁₂ represents a bond;
X represents C(O) or CH₂; and
(Rₐ)ₜ indicates that the benzene ring is substituted with t Rₐ, with each Rₐ being the same or different and each independently representing bromine, and t represents an integer of 0, 1, 2 or 3.

14. The compound of Formula (I) or salts, enantiomers, stereoisomers, solvates, isotopically enriched analogs, prodrugs, or polymorphs thereof as claimed in claim 1, wherein R₁₁ represents the following structures:

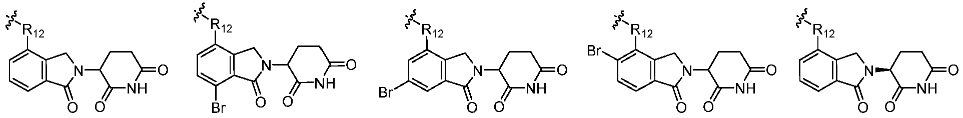

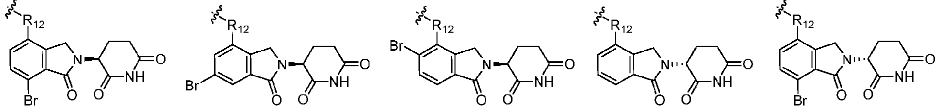

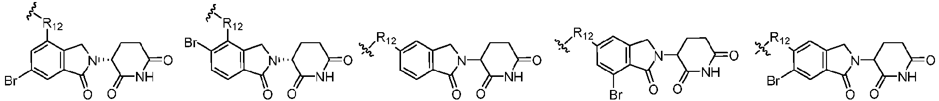

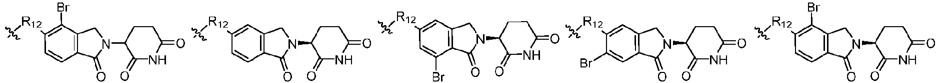

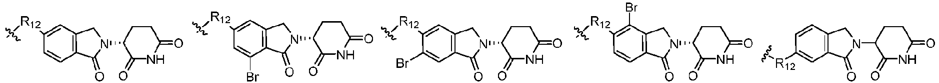

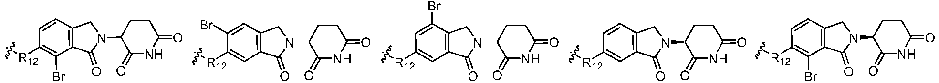

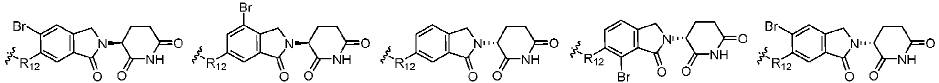

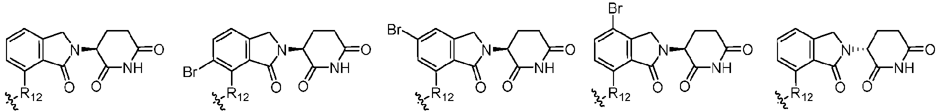

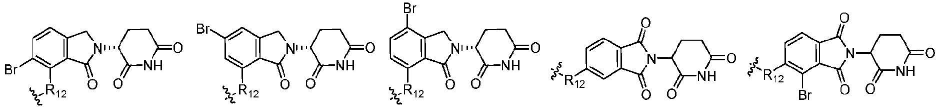

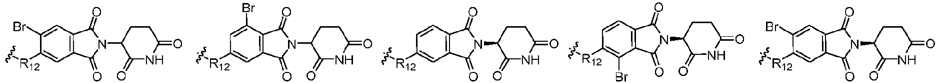

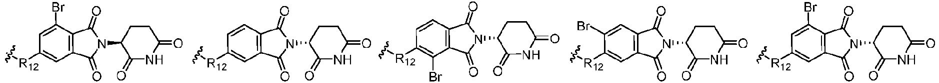

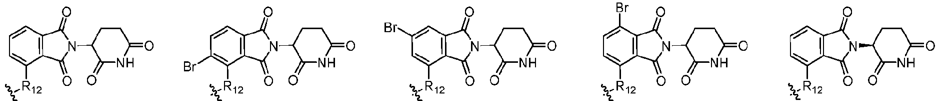

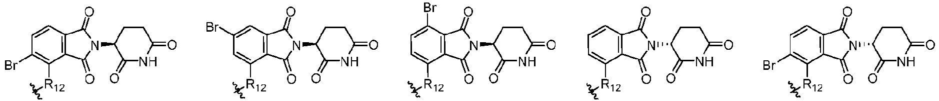

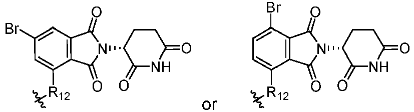

15. The compound of Formula (I) or salts, enantiomers, stereoisomers, solvates, isotopically enriched analogs, prodrugs, or polymorphs thereof as claimed in any one of claims 1-14, wherein the LIN represents the following structures:
#-C₁₋₃₀ alkylene -;
#-(C(R^{a3})(R^{a4}))ₙ₄-(R_{b}-(C(R^{a5})(R^{a6}))ₙ₅)ₘ₁-;
#-(C(R^{a3})(R^{a4}))ₙ₄-(R_{b}-(C(R^{a5})(R^{a6}))ₙ₅)ₘ₁-(R_{b}-(C(R^{a7})(R^{a8}))ₙ₆)ₘ₂-;
#-(C(R^{a3})(R^{a4}))ₙ₄-(R_{b}-(C(R^{a5})(R^{a6}))ₙ₅)ₘ₁-(R_{b}-(C(R^{a7})(R^{a8}))ₙ₆)ₘ₂-(R_{b}-(C(R^{a9})(R^{a10}))ₙ₇)ₘ₃-;
#-(C(R^{a3})(R^{a4}))ₙ₄-(R_{c}-(C(R^{a5})(R^{a6}))ₙ₅)ₘ₁-;
#-(C(R^{a3}))ₙ₄-(R_{c}-(C(R^{a5})(R^{a6}))ₙ₅)ₘ₁-(R_{c}(C(R^{a7})(R^{a8}))ₙ₆)ₘ₂-;
#-(C(R^{a3})(R^{a4}))ₙ₄-(R_{c}-(C(R^{a5})(R^{a6}))ₙ₅)ₘ₁-(R_{c}-(C(R^{a7})(R^{a8}))ₙ₆)ₘ₂-(R_{c}-(C(R^{a9})(R^{a10}))ₙ₇)ₘ₃-;
#-(C(R^{a3})(R^{a4}))ₙ₄-(R_{b}-R_{c}-(C(R^{a5})(R^{a6}))ₙ₅)ₘ₁-;
#-(C(R^{a3})(R^{a4}))ₙ₄-(R_{b}-(C(R^{a5})(R^{a6}))ₙ₅)ₘ₁-(R_{c}-(C(R^{a7})(R^{a8}))ₙ₆)ₘ₂-;
#-(C(R^{a3})(R^{a4}))ₙ₄-(R_{c}-R_{b}-(C(R^{a5})(R^{a6}))ₙ₅)ₘ₁-; or
#-(C(R^{a3})(R^{a4}))ₙ₄-(R_{c}-(C(R^{a5})(R^{a6}))ₙ₅)ₘ₁-(R_{b}-(C(R^{a7})(R^{a8}))ₙ₆)ₘ₂-;
wherein each group R_{b} is selected from the group consisting of O, N(Ra), C(O), C(O)O, OC(O), S(O), S(O)₂, S(O)₂NH, NHS(O)₂, C(O)N(R_{d}), N(R_{d})C(O), or N(R_{d})C(O)N(R_{d}), wherein each R_{d} independently represents H or C₁₋₆ alkyl; each group R_{c} is selected from the group consisting of cycloalkylene (e.g., C₃₋₂₀ cycloalkylene), arylene (e.g., C₃₋₂₀ arylene), heterocyclylene (e.g., 4- to 20-membered heterocyclylene), heteroarylene (e.g., 4- to 20-membered heteroarylene), alkynylene (e.g., C₂₋₆ alkynylene), alkenylene (e.g., C₂₋₆ alkenylene) or any combination thereof, wherein the cycloalkylene, the arylene, the heterocyclylene and the heteroarylene are each independently optionally substituted with a substituent selected from the group consisting of C₁₋₆ alkyl, halogenated C₁₋₆ alkyl, halogen, C₁₋₆ alkoxy, or any combination thereof;
a hydrogen atom of one or more CH₂ of the C₁₋₃₀ alkylene is optionally replaced by a substituent selected from the group consisting of: C₁₋₆ alkyl, halogenated C₁₋₆ alkyl, halogen, C₁₋₆ alkoxy, or any combination thereof;
R^{a3}, R^{a4}, R^{a5}, R^{a6}, R^{a7}, R^{a8}, R^{a9}, and R^{a10} are the same or different and each independently represents H, C₁₋₆ alkyl, halogenated C₁₋₆ alkyl, halogen, or C₁₋₆ alkoxy;
n4, n5, n6, n7, m1, m2, and m3 are each independently selected from the group consisting of integers of 1, 2, 3, 4, 5, 6, 7, 8, 9, 10, 11, 12, 13, 14, or 15; and
symbol # indicates the point of attachment to R₁₀.

16. The compound of Formula (I) or salts, enantiomers, stereoisomers, solvates, isotopically enriched analogs, prodrugs, or polymorphs thereof as claimed in any one of claims 1-14, wherein the LIN represents the following structures:
#-(C(R^{a3})(R^{a4}))ₙ₄-(O-(C(R^{a5})(R^{a6}))ₙ₅)ₘ₁-;
#-(C(R^{a3})(R^{a4}))ₙ₄-(O-(C(R^{a5})(R^{a6}))ₙ₅)ₘ₁-(O-(C(R^{a7})(R^{a8}))ₙ₆)ₘ₂-;
#-(C(R^{a3})(R^{a4}))ₙ₄-(O-(C(R^{a5})(R^{a6}))ₙ₅)ₘ₁-(O-(C(R^{a7})(R^{a8}))ₙ₆)ₘ₂-(O(C(R^{a9})(R^{a10}))ₙ₇)ₘ₃-;
#-(C(R^{a3})(R^{a4}))ₙ₄-(N(R_{d})-(C(R^{a5})(R^{a6}))ₙ₅)ₘ₁-;
#-(C(R^{a3})(R^{a4}))ₙ₄-(N(R_{d})-(C(R^{a5})(R^{a6}))ₙ₅)ₘ₁-(N(R_{d})-(C(R^{a7})(R^{a8}))ₙ₆)ₘ₂-;
#-(C(R^{a3})(R^{a4}))ₙ₄-(N(R_{d})-(C(R^{a5})(R^{a6}))ₙ₅)ₘ₁-(N(R_{d})-(C(R^{a7})(R^{a8}))ₙ₆)ₘ₂-(N(R_{d})-(C(R^{a9})(R^{a10}))ₙ₇)ₘ₃-;
#-(C(R^{a3})(R^{a4}))ₙ₄-(C(O)N(R_{d})-(C(R^{a5})(R^{a6}))ₙ₅)ₘ₁-;
#-(C(R^{a3})(R^{a4}))ₙ₄-(C(O)N(R_{d})-(C(R^{a5})(R^{a6}))ₙ₅)ₘ₁-(C(O)N(R_{d})-(C(R^{a7})(R^{a8}))ₙ₆)ₘ₂-;
#-(C(R^{a3})(R^{a4}))ₙ₄-(C(O)N(R_{d})-(C(R^{a5})(R^{a6}))ₙ₅)ₘ₁-(C(O)N(R_{d})-(C(R^{a7})(R^{a8}))ₙ₆)ₘ₂-(C(O)N(R_{d})- (C(R^{a9})(R^{a10}))ₙ₇)ₘ₃-;
#-(C(R^{a3})(R^{a4}))ₙ₄-(C(O)N(R_{d})-(C(R^{a5})(R^{a6}))ₙ₅)ₘ₁-(O-(C(R^{a7})(R^{a8}))ₙ₆)ₘ₂-;
#-(C(R^{a3})(R^{a4}))ₙ₄-C(O)N(R_{d})-(C(R^{a5})(R^{a6}))ₙ₅-(O-(C(R^{a7})(R^{a8}))ₙ₆)ₘ₁-;
#-(C(R^{a3})(R^{a4}))ₙ₄-(N(R_{d})C(O)-(C(R^{a5})(R^{a6}))ₙ₅)ₘ₁-;
#-(C(R^{a3})(R^{a4}))ₙ₄-(N(R_{d})C(O)-(C(R^{a5})(R^{a6}))ₙ₅)ₘ₁-(O-(C(R^{a7})(R^{a8}))ₙ₆)ₘ₂-;
#-(C(R^{a3})(R^{a4}))ₙ₄-(N(R_{d})C(O)-(C(R^{a5})(R^{a6}))ₙ₅)ₘ₁-(O-(C(R^{a7})(R^{a8}))ₙ₆)ₘ₂-(O-(C(R^{a9})(R^{a10}))ₙ₇)ₘ₃-;
#-(C(R^{a3})(R^{a4}))ₙ₄-N(R_{d})C(O)-(C(R^{a5})(R^{a6}))ₙ₅-(O-(C(R^{a7})(R^{a8}))ₙ₆)ₘ₁-;
#-(C(R^{a3})(R^{a4}))ₙ₄-N(R_{d})C(O)N(R_{d})-(C(R^{a5})(R^{a6}))ₙ₅-;
#-(C(R^{a3})(R^{a4}))ₙ₄-C(O)-(C(R^{a5})(R^{a6}))ₙ₅-;
#-(C(R^{a3})(R^{a4}))ₙ₄-(arylene-(C(R^{a5})(R^{a6}))ₙ₅)ₘ₁-;
#-(C(R^{a3})(R^{a4}))ₙ₄-(arylene-(C(R^{a5})(R^{a6}))ₙ₅)ₘ₁-arylene-(C(R^{a7})(R^{a8}))ₙ₆-;
#-(C(R^{a3})(R^{a4}))ₙ₄-(heterocyclylene-(C(R^{a5})(R^{a6}))ₙ₅)ₘ₁-;
#-(C(R^{a3})(R^{a4}))ₙ₄-(heterocyclylene-(C(R^{a5})(R^{a6}))ₙ₅)ₘ₁-(heterocyclylene-(C(R^{a7})(R^{a8}))ₙ₆)ₘ₂-;
#-(C(R^{a3})(R^{a4}))ₙ₄-(heteroarylene-(C(R^{a5})(R^{a6}))ₙ₅)ₘ₁-;
#-(C(R^{a3})(R^{a4}))ₙ₄-(heteroarylene-(C(R^{a5})(R^{a6}))ₙ₅)ₘ₁-(heteroarylene-(C(R^{a7})(R^{a8}))ₙ₆)ₘ₂-;
#-(C(R^{a3})(R^{a4}))ₙ₄-(cycloalkylene-(C(R^{a5})(R^{a6}))ₙ₅)ₘ₁-; or
#-(C(R^{a3})(R^{a4}))ₙ₄-(cycloalkylene-(C(R^{a5})(R^{a6}))ₙ₅)ₘ₁-(cycloalkylene-(C(R^{a7})(R^{a8}))ₙ₆)ₘ₂-;
wherein each R_{d} independently represents H or C₁₋₆ alkyl;
the cycloalkylene (e.g., C₃₋₂₀ cycloalkylene), the arylene (e.g., C₃₋₂₀ arylene), the heterocyclylene (e.g., 4- to 20-membered heterocyclylene) and the heteroarylene (e.g., 4- to 20-membered heteroarylene) are each independently optionally substituted with a substituent selected from the group consisting of C₁₋₆ alkyl, halogenated C₁₋₆ alkyl, halogen, C₁₋₆ alkoxy, or any combination thereof;
R^{a3}, R^{a4}, R^{a5}, R^{a6}, R^{a7}, R^{a8}, R^{a9}, and R^{a10} are the same or different and each independently represent H, C₁₋₆ alkyl, halogenated C₁₋₆ alkyl, halogen, or C₁₋₆ alkoxy;
n4, n5, n6, n7, m1, m2, and m3 are each independently selected from the group consisting of integers of 1, 2, 3, 4, 5, 6, 7, 8, 9, 10, 11, 12, 13, 14, or 15; and
symbol # indicates the point of attachment to R₁₀.

17. The compound of Formula (I) or salts, enantiomers, stereoisomers, solvates, isotopically enriched analogs, prodrugs, or polymorphs thereof as claimed in any one of claims 1-14, wherein the LIN represents the following groups:
#-CH₂-, #-(CH₂)₂-, #-(CH₂)₃-, #-(CH₂)₄-, #-(CH₂)₅-, #-(CH₂)₆-, #-(CH₂)₇-, #-(CH₂)₈-, #-(CH₂)₉-, #-(CH₂)₁₀-, #-(CH₂)₁₁-, #-(CH₂)₁₂-, #-(CH₂)₁₃-, #-(CH₂)₁₄-, #-(CH₂)₁₅-, #-(CH₂)₁₆-, #-(CH₂)₁₇-, #- (CH₂)₁₈-, #-(CH₂)₁₉-, #-(CH₂)₂₀-, #-(CH₂)₂₁-, #-(CH₂)₂₂-, #-(CH₂)₂₅-, or #-(CH₂)₃₀-;#-CH₂-O-CH₂-, #- CH₂-O-(CH₂)₂-, #-(CH₂)₁-O-(CH₂)₃-, #-(CH₂)₁-O-(CH₂)₄-, #-(CH₂)₁-O-(CH₂)₅-, #-(CH₂)₁-O-(CH₂)₆-, #-(CH₂)₁-O-(CH₂)₇-, #-(CH₂)₁-O-(CH₂)₈-, #-(CH₂)₁-O-(CH₂)₉-, #-(CH₂)₁-O-(CH₂)₁₀-, #-(CH₂)₂-O- (CH₂)₁-, #-(CH₂)₂-O-(CH₂)₂-, #-(CH₂)₂-O-(CH₂)₃-, #-(CH₂)₂-O-(CH₂)₄-, #-(CH₂)₂-O-(CH₂)₅-, #- (CH₂)₂-O-(CH₂)₆-, #-(CH₂)₂-O-(CH₂)₇-, #-(CH₂)₂-O-(CH₂)₅-, #-(CH₂)₂-O-(CH₂)₉-, #-(CH₂)₂-O- (CH₂)₁₀-, #-(CH₂)₂-O-(CH₂)₁₁-, #-(CH₂)₂-O-(CH₂)₁₂-, #-(CH₂)₃-O-(CH₂)₁-, #-(CH₂)₃-O-(CH₂)₂-, #- (CH₂)₃-O-(CH₂)₃-, #-(CH₂)₃-O-(CH₂)₄-, #-(CH₂)₃-O-(CH₂)₅-, #-(CH₂)₃-O-(CH₂)₆-, #-(CH₂)₃-O- (CH₂)₇-, #-(CH₂)₄-O-(CH₂)₁-, #-(CH₂)₄-O-(CH₂)₂-, #-(CH₂)₄-O-(CH₂)₃-, #-(CH₂)₄-O-(CH₂)₄-, #- (CH₂)₄-O-(CH₂)₅-, #-(CH₂)₄-O-(CH₂)₆-, #-(CH₂)₅-O-(CH₂)₁-, #-(CH₂)₅-O-(CH₂)₂-, #-(CH₂)₅-O- (CH₂)₃-, #-(CH₂)₅-O-(CH₂)₄-, #-(CH₂)₅-O-(CH₂)₅-, #-(CH₂)₆-O-(CH₂)₁-, #-(CH₂)₆-O-(CH₂)₂-, #- (CH₂)₆-O-(CH₂)₃-, #-(CH₂)₆-O-(CH₂)₄-, #-(CH₂)₇-O-(CH₂)₁-, #-(CH₂)₇-O-(CH₂)₂-, #-(CH₂)₇-O- (CH₂)₃-, #-(CH₂)₈-O-(CH₂)₁-, #-(CH₂)₈-O-(CH₂)₂-, #-CH(CH₃)-O-(CH₂)₁-, #-CH(CH₃)-O-(CH₂)₂-, #- CH(CH₃)-O-(CH₂)₃-, #-CH(CH₃)-O-(CH₂)₄-, #-CH(CH₃)-O-(CH₂)₅-, #-CH(CH₃)-O-(CH₂)₆-, #- CH(CH₃)-O-(CH₂)₇-, #-CH(CH₃)-O-(CH₂)₈-, #-CH(CH₃)-O-(CH₂)₉-, #-CH(CH₃)-O-(CH₂)₁₀-, #-CH₂- (O(CH₂)₂)₂-, #-CH₂-(O(CH₂)₂)₃-, #-CH₂-(O(CH₂)₂)₄-, #-CH₂-(O(CH₂)₂)₅-, #-CH₂-(O(CH₂)₂)₆-, #-CH₂- (O(CH₂)₂)₇-, #-CH₂-(O(CH₂)₂)₈-, #-CH₂-(O(CH₂)₂)₉-, #-CH₂-(O(CH₂)₂)₁₀-, #-CH₂-(O(CH₂)₂)₁-OCH₂-, #-CH₂-(O(CH₂)₂)₂-OCH₂-, #-CH₂-(O(CH₂)₂)₃-OCH₂-, #-CH₂-(O(CH₂)₂)₄-OCH₂-, #-CH₂-(O(CH₂)₂)₅- OCH₂-, #-CH₂-(O(CH₂)₂)₆-OCH₂-, #-CH₂-(O(CH₂)₂)₇-OCH₂-, #-CH₂-(O(CH_{Z})₂)₈-OCH₂-, #-CH₂- (O(CH₂)₂)₉-OCH₂-, #-CH₂-(O(CH₂)₂)₁₀-OCH₂-, #-(CH₂)₂-(O(CH₂)₂)₂-, #-(CH₂)₂-(O(CH₂)₂)₃-, #- (CH₂)₂-(O(CH₂)₂)₄-, #-(CH₂)₂-(O(CH₂)₂)₅-, #-(CH₂)₂-(O(CH₂)₂)₆-, #-(CH₂)₂-(O(CH₂)₂)₇-, #-(CH₂)₂- (O(CH₂)₂)₈-, #-(CH₂)₂-(O(CH₂)₂)₉-, #-(CH₂)₂-(O(CH₂)₂)₁₀-, #-(CH₂)₃-(O(CH₂)₂)₂-, #-(CH₂)₃- (O(CH₂)₂)₃-, #-(CH₂)₃-(O(CH₂)₂)₄-, #-(CH₂)₃-(O(CH₂)₂)₅-, #-(CH₂)₃-(O(CH₂)₂)₆-, #-(CH₂)₃- (O(CH₂)₂)₇-, #-(CH₂)₃-(O(CH₂)₂)₈-, #-(CH₂)₃-(O(CH₂)₂)₉-, #-(CH₂)₃-(O(CH₂)₂)₁₀-, #-(CH₂)₄- (O(CH₂)₂)₂-, #-(CH₂)₄-(O(CH₂)₂)₃-, #-(CH₂)₄-(O(CH₂)₂)₄-, #-(CH₂)₄-(O(CH₂)₂)₅-, #-(CH₂)₄- (O(CH₂)₂)₆-, #-(CH₂)₄-(O(CH₂)₂)₇-, #-(CH₂)₄-(O(CH₂)₂)₈-, #-(CH₂)₄-(O(CH₂)₂)₉-, #-(CH₂)₄- (O(CH₂)₂)₁₀-, #-CH₂-(O(CH₂)₃)₂-, #-CH₂-(O(CH₂)₃)₃-, #-CH₂-(O(CH₂)₃)₄-, #-CH₂-(O(CH₂)₃)₅-, #- CH₂-(O(CH₂)₃)₆-, #-CH₂-(O(CH₂)₃)₇-, #-CH₂-(O(CH₂)₃)₈-, #-CH₂-(O(CH₂)₃)₉-, #-CH₂-(O(CH₂)₃)₁₀-, #-(CH₂)₂-(O(CH₂)₃)₂-, #-(CH₂)₂-(O(CH₂)₃)₃-, #-(CH₂)₂-(O(CH₂)₃)₄-, #-(CH₂)₂-(O(CH₂)₃)₅-, #-(CH₂)₂- (O(CH₂)₃)₆-, #-(CH₂)₂-(O(CH₂)₃)₇-, #-(CH₂)₂-(O(CH₂)₃)₈-, #-(CH₂)₂-(O(CH₂)₃)₉-, #-(CH₂)₂- (O(CH₂)₃)₁₀-, #-(CH₂)₃-(O(CH₂)₃)₂-, #-(CH₂)₃-(O(CH₂)₃)₃-, #-(CH₂)₃-(O(CH₂)₃)₄-, #-(CH₂)₃- (O(CH₂)₃)₅-, #-(CH₂)₃-(O(CH₂)₃)₆-, #-(CH₂)₃-(O(CH₂)₃)₇-, #-(CH₂)₃-(O(CH₂)₃)₈-, #-(CH₂)₃- (O(CH₂)₃)₉-, #-(CH₂)₃-(O(CH₂)₃)₁₀-, #-CH₂-O-(CH₂)₂-O-(CH₂)₃-, #-CH₂-(O(CH₂)₂)₂-(O(CH₂)₃)₂-, #- CH₂-(O(CH₂)₂)₃-(O(CH₂)₃)₃-, #-CH₂-(O(CH₂)₂)₄-(O(CH₂)₃)₄-, #-CH₂-(O(CH₂)₂)₅-(O(CH₂)₃)₅-, #-CH₂- (O(CH₂)₂)₆-(O(CH₂)₃)₆-, #-(CH₂)₂-O-(CH₂)₂-O-(CH₂)₃-, #-(CH₂)₂-(O(CH₂)₂)₂-(O(CH₂)₃)₂-, #-(CH₂)₂- (O(CH₂)₂)₃-(O(CH₂)₃)₃-, #-(CH₂)₂-(O(CH₂)₂)₄-(O(CH₂)₃)₄-, #-(CH₂)₂-(O(CH₂)₂)₅-(O(CH₂)₃)₅-, #- (CH₂)₂-(O(CH₂)₂)₆-(O(CH₂)₃)₆-, #-(CH₂)₃-O-(CH₂)₂-O-(CH₂)₃-, #-(CH₂)₃-(O(CH₂)₂)₂-(O(CH₂)₃)₂-, #- (CH₂)₃-(O(CH₂)₂)₃-(O(CH₂)₃)₃-, #-(CH₂)₃-(O(CH₂)₂)₄-(O(CH₂)₃)₄-, #-(CH₂)₃-(O(CH₂)₂)₅-(O(CH₂)₃)₅-, #-(CH₂)₃-(O(CH₂)₂)₆-(O(CH₂)₃)₆-, #-CH₂-O-(CH₂)₃-O-(CH₂)₂-, #-CH₂-(O(CH₂)₃)₂-(O(CH₂)₂)₂-, #- CH₂-(O(CH₂)₃)₃-(O(CH₂)₂)₃-, #-CH₂-(O(CH₂)₃)₄-(O(CH₂)₂)₄-, #-CH₂-(O(CH₂)₃)₅-(O(CH₂)₂)₅-, #-CH₂- (O(CH₂)₃)₆-(O(CH₂)₂)₆-, #-(CH₂)₂-O-(CH₂)₃-O-(CH₂)₂-, #-(CH₂)₂-(O(CH₂)₃)₂-(O(CH₂)₂)₂-, #-(CH₂)₂- (O(CH₂)₃)₃-(O(CH₂)₂)₃-, #-(CH₂)₂-(O(CH₂)₃)₄-(O(CH₂)₂)₄-, #-(CH₂)₂-(O(CH₂)₃)₅-(O(CH₂)₂)₅-, #- (CH₂)₂-(O(CH₂)₃)₆-(O(CH₂)₂)₆-, #-(CH₂)₃-O-(CH₂)₃-O-(CH₂)₂-, #-(CH₂)₃-(O(CH₂)₃)₂-(O(CH₂)₂)₂-, #- (CH₂)₃-(O(CH₂)₃)₃-(O(CH₂)₂)₃-, #-(CH₂)₃-(O(CH₂)₃)₄-(O(CH₂)₂)₄-, #-(CH₂)₃-(O(CH₂)₃)₅-(O(CH₂)₂)₅-, #-(CH₂)₃-(O(CH₂)₃)₆-(O(CH₂)₂)₆-, #-CH₂-O-(CH₂)₂-O-CH₂-, #-(CH₂)₂-O-(CH₂)₂-O-CH₂-, #-(CH₂)₂- (O(CH₂)₂)₂-O-(CH₂)₃-, #-(CH₂)₂-(O(CH₂)₂)₃-O-(CH₂)₃-, #-(CH₂)₂-(O(CH₂)₂)₄-O-(CH₂)₃-, #-(CH₂)₅- (O(CH₂)₂)₂-O-(CH₂)₅-, #-(CH₂)₅-(O(CH₂)₂)₂-O-(CH₂)₆-, #-(CH₂)₁-N(R_{g})-(CH₂)₁-, #-(CH₂)₁-N(R_{g})- (CH₂)₂-, #-(CH₂)₁-N(R_{g})-(CH₂)₃-, #-(CH₂)₁-N(R_{g})-(CH₂)₄-, #-(CH₂)₁-N(R_{g})-(CH₂)₅-, #-(CH₂)₁-N(R_{g})- (CH₂)₆-, #-(CH₂)₁-N(R_{g})-(CH₂)₇-, #-(CH₂)₁-N(R_{g})-(CH₂)₈-, #-(CH₂)₁-N(R_{g})-(CH₂)₉-, #-(CH₂)₁-N(R_{g})- (CH₂)₁₀-, #-(CH₂)₂-N(R_{g})-(CH₂)₁-, #-(CH₂)₂-N(R_{g})-(CH₂)₂-, #-(CH₂)₂-N(R_{g})-(CH₂)₃-, #-(CH₂)₂-N(R_{g})- (CH₂)₄-, #-(CH₂)₂-N(R_{g})-(CH₂)₅-, #-(CH₂)₂-N(R_{g})-(CH₂)₆-, #-(CH₂)₂-N(R_{g})-(CH₂)₇-, #-(CH₂)₂-N(R_{g})- (CH₂)₈-, #-(CH₂)₂-N(R_{g})-(CH₂)₉-, #-(CH₂)₂-N(R_{g})-(CH₂)₁₀-, #-(CH₂)₂-N(R_{g})-(CH₂)₁₁-, #-(CH₂)₂- N(R_{g})-(CH₂)₁₂-, #-(CH₂)₃-N(R_{g})-(CH₂)₁-, #-(CH₂)₃-N(R_{g})-(CH₂)₂-, #-(CH₂)₃-N(R_{g})-(CH₂)₃-, #-(CH₂)₄- N(R_{g})-(CH₂)₁-, #-(CH₂)₄-N(R_{g})-(CH₂)₂-, #-(CH₂)₄-N(R_{g})-(CH₂)₃-, #-(CH₂)₄-N(R_{g})-(CH₂)₄-, #-(CH₂)₅- N(R_{g})-(CH₂)₁-, #-(CH₂)₅-N(R_{g})-(CH₂)₂-, #-(CH₂)₅-N(R_{g})-(CH₂)₃-, #-(CH₂)₅-N(R_{g})-(CH₂)₄-, #-(CH₂)₅- N(R_{g})-(CH₂)₅-, #-(CH₂)₆-N(R_{g})-(CH₂)₁-, #-(CH₂)₆-N(R_{g})-(CH₂)₂-, #-(CH₂)₆-N(R_{g})-(CH₂)₃-, #-(CH₂)₇- N(R_{g})-(CH₂)₁-, #-(CH₂)₇-N(R_{g})-(CH₂)₂-, #-(CH₂)₇-N(R_{g})-(CH₂)₃-, #-(CH₂)₈-N(R_{g})-(CH₂)₁-, #-(CH₂)₈- N(R_{g})-(CH₂)₂-, #-(CH₂)₈-N(R_{g})-(CH₂)₃-, #-CH(CH₃)-N(R_{g})-(CH₂)₁-, #-CH(CH₃)-N(R_{g})-(CH₂)₂-, #- CH(CH₃)-N(R_{g})-(CH₂)₃-, #-CH(CH₃)-N(R_{g})-(CH₂)₄-, #-CH(CH₃)-N(R_{g})-(CH₂)₅-, #-CH(CH₃)-N(R_{g})- (CH₂)₆-, #-CH(CH₃)-N(R_{g})-(CH₂)₇-, #-CH(CH₃)-N(R_{g})-(CH₂)₈-, #-CH(CH₃)-N(R_{g})-(CH₂)₉-, #- CH(CH₃)-N(R_{g})-(CH₂)₁₀-, #-CH₂C(O)NHCH₂-, #-(CH₂)₂C(O)NH(CH₂)₂-, #-(CH₂)₂C(O)NH(CH₂)₃-, #-(CH₂)₂C(O)NH(CH₂)₄-, #-(CH₂)₂C(O)NH(CH₂)₅-, #-(CH₂)₃C(O)NH(CH₂)₃-, #- (CH₂)₃C(O)NH(CH₂)₄-, #-(CH₂)₄C(O)NH(CH₂)₄-, #-(CH₂)₅C(O)NH(CH₂)₅-, #- (CH₂)₆C(O)NH(CH₂)₇-, #-(CH₂)₆C(O)NH(CH₂)₆-, #-(CH₂)₇C(O)NH(CH₂)₇-, #-(CH₂)₈C(O)NH(CH₂)₈, U-(CH₂)₉C(O)NH(CH₂)₉-, #-(CH₂)₁₀C(O)NH(CH₂)₁₀-, #-(CH₂)₂C(O)NH(CH₂)₂-O-(CH₂)₂-, #- CH₂NHC(O)CH₂-, #-(CH₂)₂NHC(O)(CH₂)₂-, #-(CH₂)₂NHC(O)(CH₂)₃-, #-(CH₂)₂NHC(O)(CH₂)₄-, #- (CH₂)₂NHC(O)(CH₂)₅-, #-(CH₂)₃NHC(O)(CH₂)₃-, #-(CH₂)₃NHC(O)(CH₂)₄-, #- (CH₂)₄NHC(O)(CH₂)₄-, #-(CH₂)₅NHC(O)(CH₂)₅-, #-(CH₂)₆NHC(O)(CH₂)₇-, #- (CH₂)₆NHC(O)(CH₂)₆-, #-(CH₂)₇NHC(O)(CH₂)₇-, #-(CH₂)₈NHC(O)(CH₂)₈, #-(CH₂)₉NHC(O)(CH₂)₉-, #-(CH₂)₁₀NHC(O)(CH₂)₁₀-, #-(CH₂)₄NHC(O)(CH₂)₈-, #-(CH₂)₂NHC(O)(CH₂)₂-O-(CH₂)₂-, #- (CH₂)₄NHC(O)CH₂-, #-CH₂-piperidinylene-CH₂-, #-CH₂-piperidinylene-(CH₂)₂-, #-CH₂- piperidinylene-(CH₂)₃-, %-CH₂-piperidinylene-(CH₂)₄-, #-CH₂-piperidinylene-(CH₂)₅-, #-CH₂- piperidinylene-(CH₂)₆-, #-CH₂-piperidinylene-(CH₂)₇-, #-CH₂-piperidinylene-(CH₂)₈-, #-(CH₂)₂- piperidinylene-(CH₂)₁-, #-(CH₂)₂-piperidinylene-(CH₂)₂-, #-(CH₂)₂-piperidinylene-(CH₂)₃-, #-(CH₂)₂- piperidinylene-(CH₂)₄-, #-(CH₂)₂-piperidinylene-(CH₂)₅-, #-(CH₂)₂-piperidinylene-(CH₂)₆-, #-(CH₂)₂- piperidinylene-(CH₂)₇-, #-(CH₂)₂-piperidinylene-(CH₂)₈-, #-(CH₂)₃-piperidinylene-CH₂-, #-(CH₂)₃- piperidinylene-(CH₂)₂-, #-(CH₂)₃-piperidinylene-(CH₂)₃-, #-(CH₂)₃-piperidinylene-(CH₂)₄-, #-(CH₂)₃- piperidinylene-(CH₂)₅-, #-(CH₂)₃-piperidinylene-(CH₂)₆-, #-(CH₂)₃-piperidinylene-(CH₂)₇-, #-(CH₂)₃- piperidinylene-(CH₂)₈-, #-(CH₂)₄-piperidinylene-CH₂-, #-(CH₂)₄-piperidinylene-(CH₂)₂-, #-(CH₂)₄- piperidinylene-(CH₂)₃-, #-(CH₂)₄-piperidinylene-(CH₂)₄-, #-(CH₂)₄-piperidinylene-(CH₂)₅-, #-(CH₂)₄- piperidinylene-(CH₂)₆-, #-(CH₂)₄-piperidinylene-(CH₂)₇-, #-(CH₂)₄-piperidinylene-(CH₂)₈-, #-(CH₂)₅- piperidinylene-(CH₂)₁-, #-(CH₂)₅-piperidinylene-(CH₂)₂-, #-(CH₂)₅-piperidinylene-(CH₂)₃-, #-(CH₂)₅- piperidinylene-(CH₂)₄-, #-(CH₂)₅-piperidinylene-(CH₂)₅-, #-(CH₂)₅-piperidinylene-(CH₂)₆-, #-(CH₂)₅- piperidinylene-(CH₂)₇-, #-(CH₂)₅-piperidinylene-(CH₂)₈-, #-(CH₂)₆-piperidinylene-(CH₂)₁-, #-(CH₂)₆- piperidinylene-(CH₂)₂-, #-(CH₂)₆-piperidinylene-(CH₂)₃-, #-(CH₂)₆-piperidinylene-(CH₂)₄-, #-(CH₂)₆- piperidinylene-(CH₂)₅-, #-(CH₂)₆-piperidinylene-(CH₂)₆-, #-(CH₂)₆-piperidinylene-(CH₂)₇-, #-(CH₂)₆- piperidinylene-(CH₂)₈-, #-(CH₂)₇-piperidinylene-(CH₂)₁-, #-(CH₂)₇-piperidinylene-(CH₂)₂-, #-(CH₂)₇- piperidinylene-(CH₂)₃-, #-(CH₂)₇-piperidinylene-(CH₂)₄-, #-(CH₂)₇-piperidinylene-(CH₂)₈-, #-(CH₂)₈- piperidinylene-CH₂-, #-(CH₂)₈-piperidinylene-(CH₂)₂-, #-(CH₂)₈-piperidinylene-(CH₂)₃-, #-(CH₂)₈- piperidinylene-(CH₂)₄-, #-(CH₂)₈-pipendinylene-(CH₂)₅-, #-(CH₂)₈-piperidinylene-(CH₂)₆-, #-(CH₂)₈- piperidinylene-(CH₂)₇-, #-(CH₂)₈-piperidinylene-(CH₂)₈-, #-CH₂-N(R_{g})-CH₂-piperidinylene-CH₂-, #- CH₂-N(R_{g})-CH₂-piperidinylene-(CH₂)₂-, #-CH₂-N(R_{g})-CH₂-piperidinylene-(CH₂)₃-, #-CH₂-N(R_{g})- CH₂-piperidinylene-(CH₂)₄-, #-CH₂-N(R_{g})-CH₂-piperidinylene-(CH₂)₅-, #-CH₂-N(R_{g})-CH₂- piperidinylene-(CH₂)₆-, #-CH₂-N(R_{g})-CH₂-piperidinylene-(CH₂)₇-, #-CH₂-N(R_{g})-CH₂-piperidinylene- (CH₂)₈-, #-CH₂-N(R_{g})-(CH₂)₂-piperidinylene-CH₂-, #-CH₂-N(R_{g})-(CH₂)₂-piperidinylene-(CH₂)₂-, #- CH₂-N(R_{g})-(CH₂)₂-piperidinylene-(CH₂)₃-, #-CH₂-N(R_{g})-(CH₂)₂-piperidinylene-(CH₂)₄-, #-CH₂- N(R_{g})-(CH₂)₂-piperidinylene-(CH₂)₅-, #-CH₂-N(R_{g})-(CH₂)₂-piperidinylene-(CH₂)₆-, #-CH₂-N(R_{g})- (CH₂)₂-piperidinylene-(CH₂)₇-, #-CH₂-N(R_{g})-(CH₂)₂-piperidinylene-(CH₂)₈-, #-(CH₂)₂-N(R_{g})-CH₂- piperidinylene-CH₂-, #-(CH₂)₂-N(R_{g})-CH₂-piperidinylene-(CH₂)₂-, #-(CH₂)₂-N(R_{g})-CH₂- piperidinylene-(CH₂)₃-, #-(CH₂)₂-N(R_{g})-CH₂-piperidinylene-(CH₂)₄-, #-(CH₂)₂-N(R_{g})-CH₂- piperidinylene-(CH₂)₅-, #-(CH₂)₂-N(R_{g})-CH₂-piperidinylene-(CH₂)₆-, #-(CH₂)₂-N(R_{g})-CH₂- piperidinylene-(CH₂)₇-, #-(CH₂)₂-N(R_{g})-CH₂-piperidinylene-(CH₂)₈-, #-(CH₂)₃-N(R_{g})-CH₂- piperidinylene-CH₂-, #-(CH₂)₃-N(R_{g})-CH₂-piperidinylene-(CH₂)₂-, #-(CH₂)₃-N(R_{g})-CH₂- piperidinylene-(CH₂)₃-, #-(CH₂)₃-N(R_{g})-CH₂-piperidinylene-(CH₂)₈-, #-(CH₂)₄-N(R_{g})-CH₂- piperidinylene-CH₂-, #-(CH₂)₄-N(R_{g})-CH₂-piperidinylene-(CH₂)₂-, #-(CH₂)₄-N(R_{g})-CH₂- piperidinylene-(CH₂)₃-, #-(CH₂)₄-N(R_{g})-CH₂-piperidinylene-(CH₂)₈-, #-(CH₂)₅-N(R_{g})-CH₂- piperidinylene-(CH₂)₃-, #-(CH₂)₅-N(R_{g})-CH₂-piperidinylene-(CH₂)₈-, #-(CH₂)₆-N(R_{g})-CH₂- piperidinylene-(CH₂)₃-, #-(CH₂)₆-N(R_{g})-CH₂-piperidinylene-(CH₂)₈-, #-(CH₂)₇-N(R_{g})-CH₂- piperidinylene-(CH₂)₃-, #-(CH₂)₇-N(R_{g})-CH₂-piperidinylene-(CH₂)₈-, #-(CH₂)₈-N(R_{g})-CH₂- piperidinylene-CH₂-, #-(CH₂)₈-N(R_{g})-CH₂-piperidinylene-(CH₂)₂-, #-(CH₂)₈-N(R_{g})-CH₂- piperidinylene-(CH₂)₃-, #-(CH₂)₈-N(R_{g})-CH₂-piperidinylene-(CH₂)₄-, #-(CH₂)₈-N(R_{g})-CH₂- piperidinylene-(CH₂)₅-, #-(CH₂)₈-N(R_{g})-CH₂-piperidinylene-(CH₂)₆-, #-(CH₂)₈-N(R_{g})-CH₂- piperidinylene-(CH₂)₇-, #-(CH₂)₈-N(R_{g})-CH₂-piperidinylene-(CH₂)₈-, #-CH₂-piperazinylene-CH₂-, #- CH₂-piperazinylene-(CH₂)₂-, #-CH₂-piperazinylene-(CH₂)₃-, #-CH₂-piperazinylene-(CH₂)₄-, #-CH₂- piperazinylene-(CH₂)₅-, #-CH₂-piperazinylene-(CH₂)₆-, #-CH₂-piperazinylene-(CH₂)₇-, #-CH₂- piperazinylene-(CH₂)₈-, #-(CH₂)₂-piperazinylene-(CH₂)₁-, #-(CH₂)₂-piperazinylene-(CH₂)₂-, #-(CH₂)₂- piperazinylene-(CH₂)₃-, #-(CH₂)₂-piperazinylene-(CH₂)₄-, #-(CH₂)₂-piperazinylene-(CH₂)₅-, #-(CH₂)₂- piperazinylene-(CH₂)₆-, #-(CH₂)₂-piperazinylene-(CH₂)₇-, #-(CH₂)₂-piperazinylene-(CH₂)₈-, #-(CH₂)₃- piperazinylene-CH₂-, #-(CH₂)₃-piperazinylene-(CH₂)₂-, #-(CH₂)₃-piperazinylene-(CH₂)₃-, #-(CH₂)₃- piperazinylene-(CH₂)₄-, #-(CH₂)₃-piperazinylene-(CH₂)₅-, #-(CH₂)₃-piperazinylene-(CH₂)₆-, #-(CH₂)₃- piperazinylene-(CH₂)₇-, #-(CH₂)₃-piperazinylene-(CH₂)₈-, #-(CH₂)₄-piperazinylene-CH₂-, #-(CH₂)₄- piperazinylene-(CH₂)₂-, #-(CH₂)₄-piperazinylene-(CH₂)₃-, #-(CH₂)₄-piperazinylene-(CH₂)₄-, #-(CH₂)₄- piperazinylene-(CH₂)₅-, #-(CH₂)₄-piperazinylene-(CH₂)₆-, #-(CH₂)₄-piperazinylene-(CH₂)₇-, #-(CH₂)₄- piperazinylene-(CH₂)₈-, #-(CH₂)₅-piperazinylene-(CH₂)₁-, #-(CH₂)₅-piperazinylene-(CH₂)₂-, #-(CH₂)₅- piperazinylene-(CH₂)₃-, #-(CH₂)₅-piperazinylene-(CH₂)₄-, #-(CH₂)₅-piperazinylene-(CH₂)₅-, #-(CH₂)₅- piperazinylene-(CH₂)₆-, #-(CH₂)₅-piperazinylene-(CH₂)₇-, #-(CH₂)₅-piperazinylene-(CH₂)₈-, #-(CH₂)₆- piperazinylene-(CH₂)₁-, #-(CH₂)₆-piperazinylene-(CH₂)₂-, #-(CH₂)₆-piperazinylene-(CH₂)₃-, #-(CH₂)₆- piperazinylene-(CH₂)₄-, #-(CH₂)₆-piperazinylene-(CH₂)₅-, #-(CH₂)₆-piperazinylene-(CH₂)₆-, #-(CH₂)₆- piperazinylene-(CH₂)₇-, #-(CH₂)₆-piperazinylene-(CH₂)₈-, #-(CH₂)₇-piperazinylene-(CH₂)₁-, #-(CH₂)₇- piperazinylene-(CH₂)₂-, #-(CH₂)₇-piperazinylene-(CH₂)₃-, #-(CH₂)₇-piperazinylene-(CH₂)₄-, #-(CH₂)₇- piperazinylene-(CH₂)₈-, #-(CH₂)₈-piperazinylene-CH₂-, #-(CH₂)₈-piperazinylene-(CH₂)₂-, #-(CH₂)₈- piperazinylene-(CH₂)₃-, #-(CH₂)₈-piperazinylene-(CH₂)₄-, #-(CH₂)₈-piperazinylene-(CH₂)₅-, #-(CH₂)₈- piperazinylene-(CH₂)₆-, #-(CH₂)₈-piperazinylene-(CH₂)₇-, or #-(CH₂)₈-piperazinylene-(CH₂)₈-;
wherein the piperidinylene and the piperazinylene are each independently optionally substituted with a substituent selected from the group consisting of C₁₋₆ alkyl, halogenated C₁₋₆ alkyl, halogen, C₁₋₆ alkoxy, or any combination thereof;
each R_{d} independently represents H or C₁₋₆ alkyl;
symbol # indicates the point of attachment to R₁₀; and
n4, n5, n6, n7, m1, m2, and m3 are each independently selected from the group consisting of integers of 1, 2, 3, 4, 5, 6, 7, 8, 9, 10, 11, 12, 13, 14, or 15.

18. The compound of Formula (I) or salts, enantiomers, stereoisomers, solvates, isotopically enriched analogs, prodrugs, or polymorphs thereof as claimed in claim 1, which is also of the compounds of Formula (I-2-2), Formula (I-2-3), Formula (I-2-4), Formula (I-2-5), Formula (I-3-1), Formula (I-3-2), Formula (I-3-3), or Formula (I-3-4): 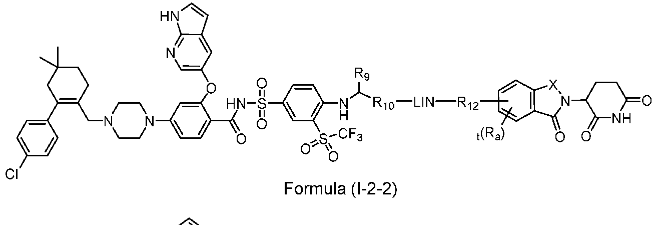 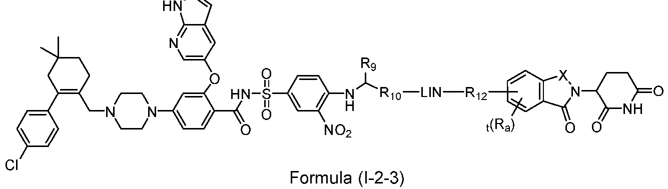 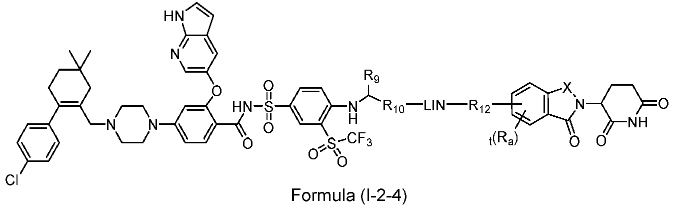 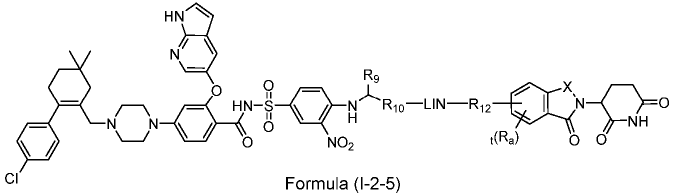 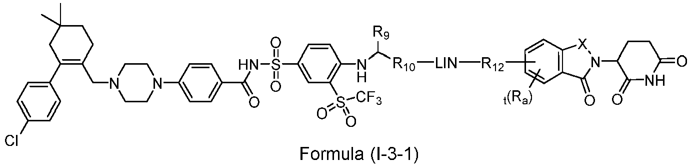 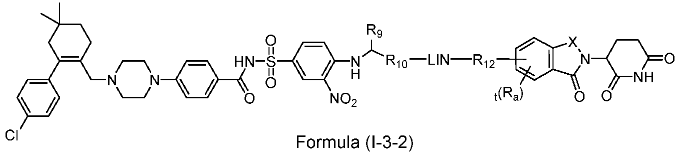 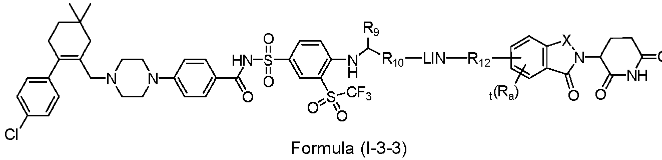 or 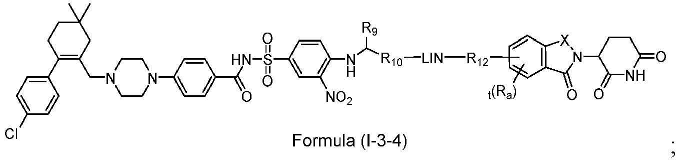 wherein R₉, R₁₀, LIN, R₁₂, (Rₐ)ₜ and X are as defined in claim 1.

19. The compound of Formula (I) or salts, enantiomers, stereoisomers, solvates, isotopically enriched analogs, prodrugs, or polymorphs thereof as claimed in claim 1, which is selected from the group consisting of:
2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((cis)-4-((3 S)-4-((2-(2,6-dioxopiperidin-3-yl)-1-oxoisoindolin-5-yl)methyl)-3-methylpiperazin-1-yl)cyclohexyl)methyl)amino)-3-nitrophenyl)sulfonyl)benzamide;
2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-((3S)-4-((2-(2,6-dioxopiperidin-3-yl)-1-oxoisoindolin-5-yl)methyl)-3-methylpiperazin-1-yl)cyclohexyl)methyl)amino)-3-nitrophenyl)sulfonyl)benzamide;
2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((cis)-4-((3R)-4-((2-(2,6-dioxopiperidin-3-yl)-1-oxoisoindolin-5-yl)methyl)-3-methylpiperazin-1-yl)cyclohexyl)methyl)amino)-3-nitrophenyl)sulfonyl)benzamide;
2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-((3R)-4-((2-(2,6-dioxopiperidin-3-yl)-1-oxoisoindolin-5-yl)methyl)-3-methylpiperazin-1-yl)cyclohexyl)methyl)amino)-3-nitrophenyl)sulfonyl)benzamide;
2-((1H-pyrrolo[2,3-b]pyridin-5-yl)oxy)-N-((4-((((cis)-4-((3 S)-4-((7-bromo-2-(2,6-dioxopiperidin-3-yl)-3-oxoisoindolin-5-yl)methyl)-3-methylpiperazin-1-yl)cyclohexyl)methyl)amino)-3-ni trophenyl)sulfonyl)-4-(4-((4'-chloro-5,5-dimethyl-3,4,5,6-tetrahydro-[1,1'-biphenyl]-2-yl)methyl)piperazin-1-yl)benzamide;
2-((1H-pyrrolo[2,3-b]pyridin-5-yl)oxy)-N-((4-((((trans)-4-((3S)-4-((7-bromo-2-(2,6-dioxopiperidin-3-yl)-3-oxoisoindolin-5-yl)methyl)-3-methylpiperazin-1-yl)cyclohexyl)methyl)amino)-3-ni trophenyl)sulfonyl)-4-(4-((4'-chloro-5,5-dimethyl-3,4,5,6-tetrahydro-[1,1'-biphenyl]-2-yl)methyl)piperazin-1-yl)benzamide;
2-((1H-pyrrolo[2,3-b]pyridin-5-yl)oxy)-N-((4-((((cis)-4-((3R)-4-((7-bromo-2-(2,6-dioxopiperidin-3-yl)-3-oxoisoindolin-5-yl)methyl)-3-methylpiperazin-1-yl)cyclohexyl)methyl)amino)-3-nitrophenyl)sulfonyl)-4-(4-((4'-chloro-5,5-dimethyl-3,4,5,6-tetrahydro-[1,1'-biphenyl]-2-yl)methyl)piperazin-1-yl)benzamide;
2-((1H-pyrrolo[2,3-b]pyridin-5-yl)oxy)-N-((4-((((trans)-4-((3R)-4-((7-bromo-2-(2,6-dioxopiperidin-3-yl)-3-oxoisoindolin-5-yl)methyl)-3-methylpiperazin-1-yl)cyclohexyl)methyl)amino)-3-nitrophenyl)sulfonyl)-4-(4-((4'-chloro-5,5-dimethyl-3,4,5,6-tetrahydro-[1,1'-biphenyl]-2-yl)methyl)piperazin-1-yl)benzamide;
2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((cis)-4-((3 S)-4-((2-(2,6-dioxopiperidin-3-yl)-1,3-dioxoisoindolin-5-yl)methyl)-3-methylpiperazin-1-yl)cyclohexyl)methyl)amino)-3-nitrophenyl)sulfonyl)benzamide;
2-((1H-pyrrolo[2,3-b]pyridin-5-yl)oxy)-N-((4-((((trans)-4-(4-((7-bromo-2-(2,6-dioxopiperidin-3-yl)-3-oxoisoindolin-5-yl)methyl)piperazin-1-yl)cyclohexyl)methyl)amino)-3-nitrophenyl)sulfonyl)-4-(4-((4'-chloro-5,5-dimethyl-3,4,5,6-tetrahydro-[1,1'-biphenyl]-2-yl)methyl)piperazin-1-yl)benzamide;
2-((1H-pyrrolo[2,3-b]pyridin-5-yl)oxy)-N-((4-((((cis)-4-(4-((7-bromo-2-(2,6-dioxopiperidin-3-yl)-3-oxoisoindolin-5-yl)methyl)piperazin-1-yl)cyclohexyl)methyl)amino)-3-nitrophenyl)sulfonyl)-4-(4-((4'-chloro-5,5-dimethyl-3,4,5,6-tetrahydro-[1,1'-biphenyl]-2-yl)methyl)piperazin-1-yl)benzamide;
2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-(4-((2-(2,6-dioxopiperidin-3-yl)-1-oxoisoindolin-5-yl)methyl)piperazin-1-yl)cyclohexyl)methyl)amino)-3-((trifluoromethyl)sulfonyl)phenyl)sulfonyl)benzamide;
2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-(4-((2-(2,6-dioxopiperidin-3-yl)-1,3-dioxoisoindolin-5-yl)methyl)piperazin-1-yl)cyclohexyl)methyl)amino)-3-((trifluoromethyl)sulfonyl)phenyl)sulfonyl)benzamide;
4-(4-((4'-chloro-4,4-dimethyl-3,4,5,6-tetrahydro-[1,1l'-biphenyl]-2-yl)methyl)piperazin-1-yl)-N-((4-((((trans)-4-(4-((2-(2, 6-dioxopip eri din-3 -yl)-1-oxoi soindolin-5 -yl)methyl)pip erazin-1-yl)cyclohexyl)methyl)amino)-3 -((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)plperazin-1-yl)-N-((4-((((trans)-4-(4-((2-(2, 6-dioxopip eri din-3 -yl)-1-oxoi soindolin-5 -yl)methyl)pip erazin-1-yl)cyclohexyl)methyl)amino)-3 -nitrophenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-((((trans)-4-(4-((2-(2,6-dioxopiperidin-3-yl)-1,3-dioxoisoindolin-5-yl)methyl)piperazin-1-yl)cyclohexyl)methyl)amino)-3 -((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-((((trans)-4-(4-((2-(2,6-dioxopiperidin-3-yl)-1,3-dioxoisoindolin-5-yl)methyl)piperazin-1-yl)cyclohexyl)methyl)amino)-3-nitrophenyl)sulfonyl)benzamide;
2-((1H-pyrrolo[2,3-b]pyridin-5-yl)oxy)-N-((4-((((trans)-4-(4-((7-bromo-2-(2,6-dioxopiperidin-3-yl)-3-oxoisoindolin-5-yl)methyl)piperazin-1-yl)cyclohexyl)methyl)amino)-3-((trifluoromethyl)sulfonyl)phenyl)sulfonyl)-4-(4-((4'-chloro-5,5-dimethyl-3,4,5,6-tetrahydro-[1,1'-biphenyl]-2-yl)methyl)piperazin-1-yl)benzamide;
N-((4-((((trans)-4-(4-((7-bromo-2-(2,6-dioxopiperidin-3-yl)-3-oxoisoindolin-5-yl)methyl)piperazin-1-yl)cyclohexyl)methyl)amino)-3-((trifluoromethyl)sulfonyl)phenyl)sulfonyl)-4-(4-((4'-chloro-4,4-dimethyl-3,4,5,6-tetrahydro-[1,1'-biphenyl]-2-yl)methyl)piperazin-1-yl)benzamide; and
N-((4-((((trans)-4-(4-((7-bromo-2-(2,6-dioxopiperidin-3-yl)-3-oxoisoindolin-5-yl)methyl)piperazin-1-yl)cyclohexyl)methyl)amino)-3-nitrophenyl)sulfonyl)-4-(4-((4'-chloro-4,4-dimethyl-3,4,5,6-tetrahydro-[1,1'-biphenyl]-2-yl)methyl)piperazin-1-yl)benzamide.

20. The compound of Formula (I) or salts, enantiomers, stereoisomers, solvates, or polymorphs thereof as claimed in any one of claims 1-19, which is hydrochlorides, sulfates, citrates, maleates, methanesulfonates, citrates, lactates, tartrates, fumarates, phosphates, dihydrophosphates, pyrophosphates, metaphosphates, oxalates, malonates, benzoates, mandelates, succinates, trifluoroacetates, hydroxy acetates, or p-toluenesulfonates of the compound of Formula (I).

21. A compound or salts, enantiomers, stereoisomers, solvates, prodrugs, or polymorphs thereof, which is selected from the group consisting of:
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-oxoisoindolin-5-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1,3-dioxoisoindolin-5-yl)methyl)piperazin-1-yl)-1-(phenylthio)butan-2-yl)amino)-3-((trifluoromethyl)sulfonyl)phenyl)sulfonyl)benzamide;
2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-(4-((2-(2,6-dioxopiperidin-3-yl)-1-oxoisoindolin-5-yl)methyl)piperazin-1-yl)cyclohexyl)methyl)amino)-3-nitrophenyl)sulfonyl)benzamide;
2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((cis)-4-(4-((2-(2,6-dioxopiperidin-3-yl)-1-oxoisoindolin-5-yl)methyl)piperazin-1-yl)cyclohexyl)methyl)amino)-3-nitrophenyl)sulfonyl)benzamide;
2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-((((trans)-4-(4-((2-(2,6-dioxopiperidin-3-yl)-1,3-dioxoisoindolin-5-yl)methyl)piperazin-1-yl)cyclohexyl)methyl)amino)-3-
nitrophenyl)sulfonyl)benzamide; and
N-((4-(((2R)-4-(4-((7-bromo-2-(2,6-dioxopiperidin-3-yl)-3-oxoisoindolin-5-yl)methyl)piperazin-1-yl)-1-(phenylthio)butan-2-yl)amino)-3 -((trifluoromethyl)sulfonyl)phenyl)sulfonyl)-4-(4-((4'-chloro-4,4-dimethyl-3,4,5,6-tetrahydro-[1,1'-biphenyl]-2-yl)methyl)piperazin-1-yl)benzamide.

22. The compound or salts, enantiomers, stereoisomers, solvates, or polymorphs thereof as claimed in claim 21, which is hydrochlorides, sulfates, citrates, maleates, sulfonates, citrates, lactates, tartrates, fumarates, phosphates, dihydrophosphates, pyrophosphates, metaphosphates, oxalates, malonates, benzoates, mandelates, succinates, trifluoroacetates, hydroxyacetates, or p-toluenesulfonates of the compound.

23. A pharmaceutical composition comprising as active ingredient the compound of Formula (I) or pharmaceutically acceptable salts, enantiomers, stereoisomers, solvates, isotopically enriched analogs, prodrugs, or polymorphs thereof as claimed in any one of claims 1-20, and at least one pharmaceutically acceptable carrier.

24. The pharmaceutical composition as claimed in claim 23, further comprising at least one additional therapeutic agent, e.g., an anticancer agent.

25. A pharmaceutical composition comprising as active ingredient the compound or pharmaceutically acceptable salts, enantiomers, stereoisomers, solvates, prodrugs, or polymorphs thereof as claimed in claim 21 or 22, and at least one pharmaceutically acceptable carrier.

26. The pharmaceutical composition as claimed in claim 25, further comprising at least one additional therapeutic agent, e.g., an anticancer agent.

27. A medicine kit or reagent kit comprising:
the compound of Formula (I) or a pharmaceutically acceptable salt thereof as claimed in any one of claims 1-20 or the pharmaceutical composition as claimed in claim 23 or 24; or
the compound or a pharmaceutically acceptable salt thereof as claimed in claim 21 or 22 or the pharmaceutical composition as claimed in claim 25 or 26.

28. Use of the compound of Formula (I) or salts, enantiomers, stereoisomers, solvates, isotopically enriched analogs, prodrugs, or polymorphs thereof as claimed in any one of claims 1-20 or the compound or salts, enantiomers, stereoisomers, solvates, prodrugs, or polymorphs thereof as claimed in claim 21 or 22 for the manufacture of a medicament for the prevention and/or treatment of a disease or disorder selected from the group consisting of neurodegenerative diseases, including Parkinson's disease and Alzheimer's disease; angiocardiopathy, including coronary heart disease, congestive heart failure, myocardial infarction, and atherosclerosis; autoimmune disease, including rheumatoid arthritis, autoimmune encephalomyelitis, ankylosing spondylitis, psoriasis, systemic lupus erythematosus, multiple sclerosis, recurrent oral ulcer, Kawasaki disease, polymyositis/dermatomyositis, Sjogren's syndrome, and atopic dermatitis; myelofibrosis; renal fibrosis; hepatic fibrosis; cirrhosis; tumors, including hematological malignancies, and solid tumors; multiple-organ dysfunction syndrome (MODS), including multiple-organ failure caused by cachexia and tangnsepsis shock; acute liver failure; graft-rejection, including organ (including kidney, heart, lung) or tissue transplant rejection; retinopathy, including diabetic macular edema (DME) and wet age-related macular degeneration (wAMD); and diabetes mellitus.

29. The use as claimed in claim 28, wherein the tumors include: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplant-related cancer; neutropenia; leukemia, including acute myeloid leukemia, chronic myelogenous leukemia, chronic lymphocytic leukemia, B-cell chronic lymphocytic leukemia, leukemia-related anemia, acute myeloid leukemia (AML); lymphomas, including diffuse large B-cell lymphoma, non-Hodgkin lymphoma, anaplastic lymphoma, anaplastic large cell lymphoma, CD20-positive lymphoma, mantle cell lymphoma, primary lymphoma, T-cell lymphoma (including relapsed or refractory peripheral T-cell lymphoma), small lymphocytic lymphoma, B-cell lymphoma, recurrent B-cell non-Hodgkin lymphoma, recurrent diffuse large B-cell lymphoma, recurrent mediastinal (thymic) large B-cell lymphoma, primary mediastinal (thymus) large B-cell lymphoma, recurrent transformed non-Hodgkin lymphoma, refractory B cellular non-Hodgkin's lymphoma, refractory diffuse large B cell lymphoma, refractory primary mediastinal (thymic) large B cell lymphoma, refractory transformed non-Hodgkin's lymphoma, lymphoplasmacytic lymphoma, Waldenstroem's macroglobulinemia; thyroid cancer; melanoma; lung cancer, including non-small cell lung cancer, small cell lung cancer, lung adenocarcinoma, and lung squamous cell carcinoma; inflammatory myofibroblastic tumor; colorectal cancer; glioma; astroblastoma; ovarian cancer; bronchial cancer; prostate cancer; breast cancer, including estrogen-dependent breast cancer, HER2-positive breast cancer, triplenegative breast cancer, incidental breast cancer, and Coden's disease; pancreatic cancer; neuroblastoma; extramedullary plasmacytoma; medulloblastoma; plaemocytoma; gastric cancer; gastrointestinal stromal tumor; esophagus cancer; colorectal cancer; esophageal squamous cell cancer; liver cancer; renal cell cancer; bladder cancer; endometrial cancer; brain cancer; oral cancer; sarcomas, including rhabdomyosarcoma, various adipose-derived tumors, ewing's sarcoma/primitive neuroectodermal tumors (Ewing/PNETs), and leiomyosarcoma; chondrosarcoma; urothelial carcinoma; basal cell carcinoma; oral squamous cell cancer; cholangiocarcinoma; bone cancer; cervical cancer; and skin cancer.

30. The use as claimed in claim 28 or 29, wherein the compound of Formula (I) or a pharmaceutically acceptable salt thereof as claimed in any one of claims 1-20, or the compound or a pharmaceutically acceptable salt thereof as claimed in claim 21 or 22, or the pharmaceutical composition is formulated as a medicament which is administered through at least one mode of administration selected from the group consisting of nasal administration, inhalation administration, topical administration, oral administration, oral mucosal administration, rectal administration, pleural cavity administration, peritoneal administration, vaginal administration, intramuscular administration, subcutaneous administration, transdermal administration, epidural administration, intrathecal administration, and intravenous administration.
